# EUROPEAN PATENT APPLICATION

(11) **EP 2 243 834 A1**
(43) Date of publication of application: **27.10.2010**
(21) Application number: 10007611.6
(22) Date of filing: 05.03.2008
(51) Int. Cl.: C12N 15/11, C07H 21/00, C12Q 1/68, C40B 30/00, G01N 33/53, G01N 33/68, C12N 15/12, C12P 19/34

(54) **Assessment of risk for colorectal cancer**

(30) Priority: 05.03.2007 AU 2007901147
(62) Divisional of application: 08714743.5
(71) Applicant: Cancer Care Ontario, Toronto ON M5G 2L7 (CA)
(72) Inventor: Cotterchio, Michelle, Toronto, Ontario M5N 1M3 (CA); Gallinger, Steven, Toronto, Ontario M6C 3B4 (CA); Greenwood, Celia, Toronto, Ontario M9A 2W6 (CA); Hudson, Thomas J., Toronto, Ontario M4S 1T4 (CA); Zanke, Brent W., Ottawa, Ontario K1M 0S9 (CA); Phillips, Michael, Hudson, Québec J0P 1H0 (CA); Sundararajan, Saravanan, Montréal, Québec H2K 4B7 (CA); Montpetit, Alexandre, Montréal, Québec H2P 2K2 (CA); Laflamme, Philippe, Montreal, Québec H2P 2N6 (CA); Ferretti, Vincent, Mont-Royal, Québec H3P 1Z7 (CA)
(74) Representative: Zwicker, Jörk

(57) **Abstract**

Disclosed is a method for identifying an individual who has an altered risk for developing colorectal cancer comprising detecting a single nucleotide polymorphism (SNP).

SEQUENCE LISTING

<110> Cancer Care Ontario and McGill University

<120> Assessment of Risk for Colorectal Cancer

<130> 614-9 T1

<150> EP
<151> 2008-03-05

<150> AU2007901147
<151> 2007-03-05

<160> 19

<210> 1
<211> 661
<212> DNA
<213> Homo sapiens

<220>
<221> misc_feature
<222> [331]..[331]
<223> Chromosome 11, SNP identification no. = rs11213801

<220>
<221> misc_feature
<222> [331]..[331]
<223> allele = c, in linkage disequilibrium
with rs3802842, respectively

<220>
<221> misc_feature
<222> [331]..[331]
<223> n is a, t, c or g

<400> 1
gatgattttg ctttttcttt ctttttcttt gtttgaacta tctctttatt cccttacgta 60
aagtcacaaa aacctgctta aaaacattaa ataatttaaa agcagtaaca ggtacctcag 120
agaatgtcat caaaaatagt aaaaataaga aggtgcttta agtgccataa gtgtatttga 180
tatttaataa ctaaaacagt ttaaaaatca cctgtatttt aaatgacaaa ttgatttcag 240
aatacgtgtt tgatttgata gagaaaacgt ttaattttga aaaacaatct taaggtaaca 300
tcaccattaa gtctactata gactaaattc ntcgtcatta aagtaaatga gaaggagatg 360
acatgactga gacaatttgt tgcatggaca caaacacagt cctacagtgc aaaaaaacat 420
ctttttccaa attctacagg atgagtcgat tcctattaaa tttgcttgtg ctttctttcc 480
ggaagcacac gtctttgttt tctcaacaac tgatctgaat gcaagtttgt atctagagta 540
tttgaataaa aactcttaag ctctcctggt gtatgcaggt gtggattctt taaatagaca 600
caatgcacag gggaaaaaaa tgttttggag taggaatcct ggtaaaattt ttaaactcta 660
a 661

<210> 2
<211> 661
<212> DNA
<213> Homo sapiens

<220>
<221> misc_feature
<222> [331]..[331]
<223> Chromosome 11, SNP identification no. = rs10891239

<220>
<221> misc_feature
<222> [331]..[331]
<223> allele = t, in linkage disequilibrium
with rs3802842, respectively

<220>
<221> misc_feature
<222> [331]..[331]
<223> n is a, t, c or g

<400> 2
accttcgttt tcctcaactt ctctaaggcg tttcatttat ttcataagca tttattgagc 60
aactactaaa taccagatac ttgacaacac tgattgttcc tttttctctc taaccctctt 120
tctttcattt tcctgatgct actactctgg tatattccta catttctggc ctctcctccg 180
tgcaccccta ccgcttgaag tccttctcca gcccctctct tcccctgcgc atcagaatgg 240
tagcatttcc caagacttca ggcttgaatt tctactcttc tcttgctaca aagtcttcct 300
tggagaactt tactttctta gcttcaatta ncataattct aaagatgctt cttagatgta 360
cagtttttac caggtccagg ttctcatatc tccagaatat ttttattttt tattttatca 420
tgtcaccaca acctcaaagc caacatatta aaaactgcat tcttcttccc catcaaaacc 480
gactctttcc ccgaccttct tgacttctct tattaatcat agtgctgttt tgggggggtt 540
ggggggtgtg tgtgtctttt cccttgaaag gtctcttaaa tatgtccttt ccttgtcatt 600
tccagtattt ctggctagcc tctgttttac catttcccat cctctcagtt tcctcatccc 660
c 661

<210> 3
<211> 661
<212> DNA
<213> Homo sapiens

<220>
<223> MGC50104

<220>
<221> misc_feature
<222> [331]..[331]
<223> Chromosome 11, SNP identification no. = rs2001754

<220>
<221> misc_feature
<222> [331]..[331]
<223> allele = g, in linkage disequilibrium
with rs3802842, respectively

<220>
<221> misc_feature
<222> [331]..[331]
<223> n is a, t, c or g

<400> 3
aaaacgtttc cccaaggttc taaaattgac agcattgctt ctagggatag tgagaacctt 60
attactgagg atggtgaaga aataataaga gaaccttttg tttggcctat tctagaaagc 120
atttatacac tgtattagtg gttccactag aggaattcta agatgttttc taactataat 180
tattataatt gttgttctgt agtaggttag acaacttcca tttaattaaa attaaataca 240
tgatagttga ggtgttgctg gcttaccagc aaattcagct ggtacagaat tcagcatcag 300
tgtgtatggc agaatataaa aacaaatgta ngtatgaggc acataaccaa aattattctc 360
taatctggtt gaatttgtag atagtccaga atatatttga tttgtgagtc aaattggatt 420
gtaaaaagaa tgaaaggaag gaagaaagga agaaattaaa gctacaacca gaggtagtat 480
ggtgcattgg agaaaagagg ttgctggtaa tcacagttac acattagtca ctggcttgga 540
acaataataa tctctcagtt ccattttcct catgtgtaaa atgggtataa tcacctcttc 600
ttagctgtct tcagaggatt agaaggcaaa aataggatat acatgtgaaa attccttgaa 660
a 661

<210> 4
<211> 661
<212> DNA
<213> Homo sapiens

<220>
<223> MGC50104

<220>
<221> misc_feature
<222> [331]..[331]

<223>  Chromosome 11, SNP identification no. = rs7116087

<220>
<221>  misc_feature
<222>  [331]..[331]
<223>  allele = c, in linkage disequilibrium
       with rs3802842, respectively

<220>
<221>  misc_feature
<222>  [331]..[331]
<223>  n is a, t, c or g

<400>  4
tgctctacat atagagacca aaagtgaact atttccacac agcgtgattc ttgcatgcca      60
atctcaagtg tttgcttttc aaatcagtat tacttacagt tattttcttt ctgtacatta     120
tcctaaattc ttacatttaa attatttcac tgctaaaatc tctacatcaa gggcatacag     180
gataaggcaa tattgtcagc tgggtttctt ggttaataat ctttcctaat tattttggag     240
ggttaatatt acaagcaacg gatagaagta tgacaatagt acgtatttga tgggaaattt     300
tgggggaaca gaaggtgaat aggcaccatt nggtgtgttt atgacataga gcaattgcca     360
taaagttggg cagactttaa ctgtagaaca gatgtgcatt gtctatctta gtccctaaaa     420
ccgcctgtcc ttccagcaag actgctagtt ggacgtttgt ttagttacac attcagctgc     480
tgctttgcta taccaattgt gcacaattca gttaccacaa gtgtaaacat catgacatct     540
agatctgtga tttgcatttg gatttcttgt gaagtattaa gaggaaaaaa tacatgcaat     600
tggaagctgc atattctatc atctcaaagt ttctttgatt tccatgaaac tttgggtaac     660
a                                                                     661

<210>  5
<211>  661
<212>  DNA
<213>  Homo sapiens

<220>
<223>  MGC50104

<220>
<221>  misc_feature
<222>  [331]..[331]
<223>  Chromosome 11, SNP identification no. = rs11213809

<220>
<221>  misc_feature
<222>  [331]..[331]
<223>  allele = a, in linkage disequilibrium
       with rs3802842, respectively

<220>
<221>  misc_feature
<222>  [331]..[331]
<223>  n is a, t, c or g

<400>  5
gacagagaaa gaaagaaaga agaagaagag aaagaaagaa agagaaagaa aggaaggagg      60
gagggaagag acaggaagga aggaaggaga tgaaggaagg aagggaggga agggaaagag     120
acaaagaaag ggaaagaaag aaagagagga aggaaggaag gaaaagaaaa gagacagaag     180
gaaggaagga aggaaaagaa aagagaagga aggaaggaaa agaaagagaa agaaagaaga     240
aagaaagagg gagggaggga gggagaggaa ggaaggaagg aagaaaggaa gggagggaag     300
gaaagagaaa aagacttctt ggaggaaagc ntgccacgag ctaagctttg aaggaagaag     360
atgacccagg tgagcagtga gcaggagtag aatccaagca gaacacacga aaggggcaaa     420
ggcacagggg aaaggaagca attccctgtg acgtgcagcg gatgaaccaa ttttgtctgc     480
agcatagaga agtgaaagag aaggttggga ggcctgcaca atccttcaaa attcagttct     540
catcttgcct gttaaggtct cccagacact cccctctgct cccaaagcct ccggaatatt     600
tttctgctaa aatattctta cattgcactg tcacttcttg ttttcttaaa tctgtccctc     660
a                                                                     661

<210>  6
<211>  661
<212>  DNA

<213>  Homo sapiens

<220>
<223>  MGC50104

<220>
<221>  misc_feature
<222>  [331]..[331]
<223>  Chromosome 11, SNP identification no. = rs4548657

<220>
<221>  misc_feature
<222>  [331]..[331]
<223>  allele = c, in linkage disequilibrium
       with rs3802842, respectively

<220>
<221>  misc_feature
<222>  [331]..[331]
<223>  n is a, t, c or g

<400>  6
aatatatctt atttatcctt gctacttttt catgaatgcc agcagaagaa tctatttctc      60
atttgaaatt ttgcaaggtt attgataaag ggaatcagta ttcaccctgg gaacatctcc     120
ctataccttt tgctccatct catagcacag tgctttgcat attgcttgtt aacacggcca     180
gctaaccccc tccacgagcc tacaaagaat ttaaaaagaa aaaaaaaatc ttccgaggag     240
gcagtgtcta gtaaactcaa aacaccaatg tttgaataca gaggtacaca ctatattctt     300
ggcattcatt tgaatgaatg ctttactaag nctttctaaa tggcctattg tctgacttta     360
attagcccac tgtcctgccc tgtttagaga tctgaaccac attcccagtg ctttctgtgt     420
tccccaccca accacctgct ccctcacaat cacagaaatt tttaaaataa taataaaggc     480
aaacctttttg caagcaactt attttttcag ctgctctatt ggttctgcag ggttttttta     540
atgtcataaa atttaaaata attcctggtg gtgaattctt tcatgcagct tgcaaaaata     600
atcagttttt gcagcggaac agccgtggca agtccatctg gcagggatgt agttctgcag     660
a                                                                     661

<210>  7
<211>  661
<212>  DNA
<213>  Homo sapiens

<220>
<223>  MGC50104

<220>
<221>  misc_feature
<222>  [331]..[331]
<223>  Chromosome 11, SNP identification no. = rs1987128

<220>
<221>  misc_feature
<222>  [331]..[331]
<223>  allele = c, in linkage disequilibrium
       with rs3802842, respectively

<220>
<221>  misc_feature
<222>  [331]..[331]
<223>  n is a, t, c or g

<400>  7
tagaaggcag gagatgccac caaatttcaa tggcactata tatgattggg aaacaacata      60
cagacattta aatattcggg atcctcgatt gctcttattc ctttccctta tccagctctg     120
aaggtgataa aactattagg gaatgtgccg cgtctgagtt gaactaaatg agattgtctt     180
tgcctgtgtg gtgtgtgttt atacaagaat gtccaacagt attattagga attccccttt     240
tacatcccca caggagtatt taatgagaca taggcattga aatgaattca aaatactgaa     300
ccaaatgcct gactccctct gctcgcagac ngctttcccc tttctgtttg aaagcagatg     360
cctcatctat cagatcatgg tgtcagaact ttagacaatt taaaactgta tcaagggcat     420
aatttatatt gcctttgccc caaaagcttg ggataaaagg gctgctttca agacaaaatg     480

aattttaaat tcagaaatta ctgcatgagt tctgtccttc gctgattttg tattttagac 540
aaaactattt taaaggagca gagttttgga agtgttgtaa ggtcgttatg aaagaatgga 600
agcccagatg cttgatttaa attcaaaagg gaataattgc ttattgaatt aaagtactca 660
c 661

<210> 8
<211> 661
<212> DNA
<213> Homo sapiens

<220>
<223> MGC50104

<220>
<221> misc_feature
<222> [331]..[331]
<223> Chromosome 11, SNP identification no. = rs7130173

<220>
<221> misc_feature
<222> [331]..[331]
<223> allele = a, in linkage disequilibrium
with rs3802842, respectively

<220>
<221> misc_feature
<222> [331]..[331]
<223> n is a, t, c or g

<400> 8
agtttctgtc cctccctctc tccaccaagg aaaacttttt actttttttt tttttttttt 60
tgagacggac tcttgctctg ggcgagatct cagctcactg caacctccac ctcccgggtt 120
caagtaattc tcctgcctca gcctcctgag taactgggac tacagcagct gtgattacag 180
gcatggtggc ccagctgatt tttgtattgt ttttagtaga gatgggtttt caccatgttg 240
gccaggatgg tctcaatctc ttgacctcgt gatccgccag cctgggcctc ccaaagtgct 300
gggattacag gtgtgagcca ccgagcctgc nccaccaagg gaaactttat gtagccggca 360
ctgtcttcct aaacgtgctg ggccctggtg agttgagtga aggagtccat gaggccacaa 420
gagggcagca aataacataa aactcatcaa aaccctaaaa ctacaggcct ctaaaattgg 480
cttctgtgtg gccatctaat cctaaagaaa gtcatttatt gatgtctgcc aggcgtgcga 540
cccagtgtaa ggtcaatgtg tgaagaatta gaaggcagag tacagcctgc ctctttgagg 600
tggggttata tgtgttacct tatccagaca tagcgatctc agataacagg ccaggagcac 660
c 661

<210> 9
<211> 661
<212> DNA
<213> Homo sapiens

<220>
<223> MGC50104

<220>
<221> misc_feature
<222> [331]..[331]
<223> Chromosome 11, SNP identification no. = rs3087967

<220>
<221> misc_feature
<222> [331]..[331]
<223> allele = t, in linkage disequilibrium
with rs3802842, respectively

<220>
<221> misc_feature
<222> [331]..[331]
<223> n is a, t, c or g

<400> 9
   aaaactaaga aacatctgac aggaaaatca gtatctttta tgatctgcat aaacttttta      60
   atgtaattta agaaaaatca gtgccaaaaa tgatacttca aagataacag aagattcaga     120
   acgtaatggg ttacaacagt tccaaaaaga ccacattgta aggttatctt actagaatac     180
   ggtctctgac acatttaatt aacattctgt tttatttcag tggggttaca gtggagagga     240
   ttacaaataa atgccctcgt ccactaacaa agagcagcat tgaggcttat tctgaaaatc     300
   cagtcctgaa tgggctgaca cacctaaaac natctttgga atacacctca agttaaagaa     360
   aaacccagaa gtcatttggc cctcccctca ataagctctg cggcattcat atgaccccca     420
   ggcaatactc ccatcttctt tcacccaagg ggaagggtca tggagggaac cggtgtctgc     480
   ttcctcatct ggggagagtg gccccacctt gggcggtagg tcatttgaca ccgtcatgaa     540
   aggggtgaag gggtaaggag gcggggtagg gtaccccgga gtgtggtgca gatcttccag     600
   agcatgcagc gagtatgact gagccccagc cagggatgac ccccagcttg agcccctgtg     660
   c                                                                     661

<210>  10
<211>  661
<212>  DNA
<213>  Homo sapiens

<220>
<221>  misc_feature
<222>  [331]..[331]
<223>  Chromosome 11, SNP identification no. = rs4477469

<220>
<221>  misc_feature
<222>  [331]..[331]
<223>  allele = c, in linkage disequilibrium
       with rs3802842, respectively

<220>
<221>  misc_feature
<222>  [331]..[331]
<223>  n is a, t, c or g

<400>  10
cccgtctcta ctaaaaaata caaaaaatta gctgggcatg gtggcaggcg cctgtagtcc      60
cagctacttg ggaggctgag gcaggagaat tgcttgaacc tgggaggcgg aggttgcagt     120
gaccgagatc gcgccactgc actccagcct agcgacagag cgaaactctg tctcaaaaaa     180
caaaaagaat tctgtttagt ttgacaatgt gtctctgttt tacatatatt ttaatttgat     240
tctgttttat tgttcaggga atactttgtt aaaaggtgaa atcaaactac aaaattatga     300
caggaaaatt aattctattt ccttatccaa ncccattttt ctttcaattc ctctctgttt     360
tctttccttt cttttttttt ttttttgaga cagtcaccca ggctggagga gggtggaggg     420
cagtgacaca atctcggctc actgcaacct ccgcctccca ggtccgagca attctccttc     480
ttcagccacc tgaatagctg ggattatagg catgcaccac cacacccagc taatttttgt     540
atttttagta gagatggggt ttcaccatgt tggccaggct ggtctggaac tcctggcctc     600
aagtgaccca cctatgttgg tctcccaaag tgctgggatt acaggcgtga gccaccgcgc     660
c                                                                     661

<210>  11
<211>  661
<212>  DNA
<213>  Homo sapiens

<220>
<221>  misc_feature
<222>  [331]..[331]
<223>  Chromosome 11, SNP identification no. = rs10789822

<220>
<221>  misc_feature
<222>  [331]..[331]
<223>  allele = a, in linkage disequilibrium
       with rs3802842, respectively

<220>
<221>  misc_feature
<222>  [331]..[331]
<223>  n is a, t, c or g

<400>  11
atgaaataaa attactgaac ctttgaaata gcacagaaaa atgttgatat attcatttag      60
tatgtacatg tactatgatc tgttgctaag aaatcttata aataaaagtt atttaatgtt     120
tgatcattat ctctggtgtg actaaaacaa gtatgataaa tcaggaacaa gaatccccaa     180
aggtcaagct aaataaacat taaaatattc tatagttatt tgcaaatgat ggcattatat     240
tatatttgat ttttgatatg gaagtatcct ctgaccaggt gtggcagttc acgccgcctg     300
taatcccagc actttgggag gccgaagcgg naggattgct caagcccagg aggtcaaggc     360
tgcagtgaac catcattgca ccactgcact ccagcctggg agacagaatg agaccctgtc     420
tcaaaaaaga aaaaaaaagt atcctctaat tataatctct gataaataac atacgtgcat     480
ttgtaatatg caatgtcatt gttagtacaa ttaaaaaaaa gaacagtaaa aaatgttaga     540
agttccaaga ggaatgaaat gtggaccaaa catacagtat ctaggaatta gctcttttgg     600
attaggaaaa ttgatagata tacctgctaa acttccccct aagaaaatat cttaacagga     660
a                                                                     661

<210>  12
<211>  661
<212>  DNA
<213>  Homo sapiens

<220>
<223>  FLJ45803

<220>
<221>  misc_feature
<222>  [331]..[331]
<223>  Chromosome 11, SNP identification no. = rs6589218

<220>
<221>  misc_feature
<222>  [331]..[331]
<223>  allele = a, in linkage disequilibrium
       with rs3802842, respectively

<220>
<221>  misc_feature
<222>  [331]..[331]
<223>  n is a, t, c or g

<400>  12
   tagaaaggag aggttaggaa ggagatgaag cctgaaagca ctgggaggac accccagcat      60
   aggggaacac agagacacgt gaagccaggg agaaaaccag tagtacagat aagaaatgga     120
   agaaagcaat gacaggaaga gagacaaaga aacacagaaa gaaataaaca aaaatgcaac     180
   aaagaaggtt tttaaaagca aatcaaaaaa taagagtata acaaaaagga ggagagcagc     240
   gaagaaaaga agtaatggaa gacgctatga ggacaagaga ccatccctag attccaagct     300
   gcttggttca ctgttccttc agagggggca nggcctaggc acagcgagga agaaggcatg     360
   cagggagggg aggtgccatc tccaccccca ttgtccttcc cctcctcagg tctggtccat     420
   cagggaaacc taggacacag gctcaggggc atttgtagaa agaagttcca gaagctgtca     480
   gatttcaata catccatcct gccctccact gccaggtgcc acctcaggtg ttcccaggcc     540
   tgctcctctc ccccagagat gtggcttcag ctgccaagca attcctatca gtcttgtcat     600
   tctcagttgc cctttcccta agctctgtgt gtttccctct gtatttgaca catttggggg     660
   c                                                                     661

<210>  13
<211>  661
<212>  DNA
<213>  Homo sapiens

<220>
<223>  LOC120376

<220>
<221>  misc_feature
<222>  [331]..[331]
<223>  Chromosome 11, SNP identification no. = rs3802840

<220>
<221>  misc_feature

<222>   [331]..[331]
<223>   allele = t, in linkage disequilibrium
        with rs3802842, respectively

<220>
<221>   misc_feature
<222>   [331]..[331]
<223>   n is a, t, c or g

<400>   13
acgggcacac caggcggcct ccgggggaac cgtaagcata agcccttccc tcattcccca      60
gcccatgcca ggcctctttc aaagctgtgt ctgcaaggtg tggaggccac tcaaggcccc     120
acgcactcac cattcccaga cctggccttc ctctgctgtt cctatgactt ctgtacccag     180
ctgcatccca atcgggaagc tacagaggac agctggggca tgttgtgggg ctcaggattt     240
gggggagccc atccatcccc catgggtgaa agtaaagtat aaaaagtgcg ttggtcctct     300
gcagggggag gagcggccag caggtaagta nacatcgttt tgttaggaag acagtggata     360
tatgtacagc ccttgcagac ccatagaaaa tctctcccag aaattcacct cattttaggg     420
gccccttcca ggatagcatc tgtgtggaac atcctccctt ccctcctcat tcttctggaa     480
ctcaccctct gagatcattt atctgtaaat catcagttac ctaaagtatc tagcagaaaa     540
gggccctgtc tctgcagaag agacagctgg agtacaggac cacttcccta aaatgtatca     600
acaccttaag cagtttcagg ttattacaaa attgaactga gcagtcataa gggtataccc     660
t                                                                     661

<210>   14
<211>   661
<212>   DNA
<213>   Homo sapiens

<220>
<223>   LOC120376

<220>
<221>   misc_feature
<222>   [331]..[331]
<223>   Chromosome 11, SNP identification no. = rs3802842

<220>
<221>   misc_feature
<222>   [331]..[331]
<223>   allele = a, c

<220>
<221>   misc_feature
<222>   [331]..[331]
<223>   n is a, t, c or g

<400>   14
catgccaggc ctctttcaaa gctgtgtctg caaggtgtgg aggccactca aggccccacg      60
cactcaccat tcccagacct ggccttcctc tgctgttcct atgacttctg tacccagctg     120
catcccaatc gggaagctac agaggacagc tggggcatgt tgtggggctc aggatttggg     180
ggagcccatc catcccccat gggtgaaagt aaagtataaa aagtgcgttg gtcctctgca     240
gggggaggag cggccagcag gtaagtatac atcgttttgt taggaagaca gtggatatat     300
gtacagccct tgcagaccca tagaaaatct ntcccagaaa ttcacctcat tttaggggcc     360
ccttccagga tagcatctgt gtggaacatc ctcccttccc tcctcattct tctggaactc     420
accctctgag atcatttatc tgtaaatcat cagttaccta aagtatctag cagaaaaggg     480
ccctgtctct gcagaagaga cagctggagt acaggaccac ttccctaaaa tgtatcaaca     540
ccttaagcag tttcaggtta ttacaaaatt gaactgagca gtcataaggg tataccctga     600
cactttatac accttcctac ggtaagtact gccaaaggca ctcagggcag caacagaacc     660
a                                                                     661

<210>   15
<211>   661
<212>   DNA
<213>   Homo sapiens

<220>
<223>   LOC120376

<220>
<221> misc_feature
<222> [331]..[331]
<223> Chromosome 11, SNP identification no. = rs4608113

<220>
<221> misc_feature
<222> [331]..[331]
<223> allele = t, in linkage disequilibrium
      with rs3802842, respectively

<220>
<221> misc_feature
<222> [331]..[331]
<223> n is a, t, c or g

<400> 15
cagaaaaggg ccctgtctct gcagaagaga cagctggagt acaggaccac ttccctaaaa      60
tgtatcaaca ccttaagcag tttcaggtta ttacaaaatt gaactgagca gtcataaggg     120
tataccctga cactttatac accttcctac ggtaagtact gccaaaggca ctcagggcag     180
caacagaacc aaataaacta ggtggggtta ttgcccaaga gagggcagct catccctaaa     240
aagcctcaat catacaattg cttgggggtt ggtttgggtg tgaacatagg atctgacagc     300
tggggctcat attgcctctg ttaggattgg nccttctcca gcccattggc atctgcccca     360
caacagtttt tgtgcctgat gctgttcatg tgcaatgtgt gacttcatga aataggcctt     420
tgacagcggg acttcttgaa atgggagggg gcaaatgatg taggggaggt aaacacaggt     480
tccagaacca gatggactgg gctcagatct cagttcctcc atggtgtgta tcctcaagca     540
aattacccaa ttcctcttgc ctctgtttat tcgtcttcaa attggggatg ataggatttt     600
gtgagggtta atcgagttat tgcatacaaa gccgttagaa gaatgctttc cacatagtga     660
g                                                                     661

<210> 16
<211> 661
<212> DNA
<213> Homo sapiens

<220>
<223> LOC120376

<220>
<221> misc_feature
<222> [331]..[331]
<223> Chromosome 11, SNP identification no. = rs6589220

<220>
<221> misc_feature
<222> [331]..[331]
<223> allele = c, in linkage disequilibrium
      with rs3802842, respectively

<220>
<221> misc_feature
<222> [331]..[331]
<223> n is a, t, c or g

<400> 16
ctctttatgc tctaaattca tcattctgca gatctgacga tactaagcag gagtctgata      60
gcctcaaccc cttcagttca gagatggtta tataattaac aaaaccaggt acaagggaac     120
tggaaaatgc ttctggagaa aaacatcatc aagactttca taataaaatg taacgtttcc     180
ttaaggagct ttggtacttg ctacagttct gctcagctgc acctggagag ccccatgtga     240
gtgtggcact tctgttgctg ctgggccctt acagatgggt gtggccactg ttcccttctt     300
agctcgctac aggagctaat gtgagggtaa nacacttcac ttgtgtatct gaccagtttc     360
tgttccccgg agtagtttga cttatttaaa tttttctgaa ttccctcctg agaaataaag     420
caggacttct gaaccagtgt cttataggaa caggcagagt aaagcctgct ctactttgct     480
tcccttaaga ctgcatcagc ctatgccctc gcaagcaagc aggtttaaca cgcatataat     540
ttaattacaa aagccagacg taaaatccct cactggaagc catcggatgg atctgggatt     600
ctaaagaagc tgtgagagcg tacttagtag gcaagggtag cagacaccag gggatgatgt     660
t                                                                     661

<210>  17
<211>  661
<212>  DNA
<213>  Homo sapiens

<220>
<221>  misc_feature
<222>  [331]..[331]
<223>  Chromosome 11, SNP identification no. = rs11213825

<220>
<221>  misc_feature
<222>  [331]..[331]
<223>  allele = a, in linkage disequilibrium
       with rs3802842, respectively

<220>
<221>  misc_feature
<222>  [331]..[331]
<223>  n is a, t, c or g

<400>  17
ttctcttatc taacattagt caatgaagtg ttaggacgac caaaaagctc atgttgcctg      60
cagctatgtt agcctgtaga tcattctttt tcctttccaa agcattgctt tggaaacttt     120
ctgagcaaat agttgatctt ttccttgaga gctgccatac agttaatgca gcccaaagtt     180
tagaagtcat tattttctat ttctgaattt ttggctttag gttcttaaat atacatcact     240
tttgtttaca cgttgattta tagtattgac tatgttgttt acacttgaat tttagagttt     300
gcttcctctg attaatctat ttgaagaagc ncagctgtct ttcaaggtaa agaggagtta     360
tttaatctac ctatgtggga ctagactgtt gaatcttgtt tgctgaaggc attaaggcac     420
aggacttaat ctctgaagtg aagtctgaag agcctttgcc acatctcaag tgccagtaca     480
atggcagcaa tactggtacc ttcagcattg gccacaggta ccattttctg agcacgtcct     540
atgtgccaga attatgtaag agtattctca ctcgttatct gatttaatct tcacaatact     600
gacagcaaaa tgaggagtct gaggctctgg gaagttagtt cacctgtcca gtgtcaccca     660
g                                                                     661

<210>  18
<211>  661
<212>  DNA
<213>  Homo sapiens

<220>
<221>  misc_feature
<222>  [331]..[331]
<223>  Chromosome 11, SNP identification no. = rs7933961

<220>
<221>  misc_feature
<222>  [331]..[331]
<223>  allele = a, in linkage disequilibrium
       with rs3802842, respectively

<220>
<221>  misc_feature
<222>  [331]..[331]
<223>  n is a, t, c or g

<400>  18
ttgtttacac gttgatttat agtattgact atgttgttta cacttgaatt ttagagtttg      60
cttcctctga ttaatctatt tgaagaagcc cagctgtctt tcaaggtaaa gaggagttat     120
ttaatctacc tatgtgggac tagactgttg aatcttgttt gctgaaggca ttaaggcaca     180
ggacttaatc tctgaagtga agtctgaaga gcctttgcca catctcaagt gccagtacaa     240
tggcagcaat actggtacct tcagcattgg ccacaggtac cattttctga gcacgtccta     300
tgtgccagaa ttatgtaaga gtattctcac ncgttatctg atttaatctt cacaatactg     360
acagcaaaat gaggagtctg aggctctggg aagttagttc acctgtccag tgtcacccag     420
cctgtaggca cccaagcttg ggtcagaaag atagcaaagc cctgttcctt cctctccagg     480
gctgggaagt ttgagccacc attcgtgaaa gaccattcag gaacccatgg catcattaaa     540
tgccaacttc ctcatataca caaaggtgtg tcccaaaaac aattcagtca gacctcgcca     600
aaagggccgg attgtccata ggacacagag atgccaggca caagagctac cagattatag     660

g                                                                 661

<210>  19
<211>  50172
<212>  DNA
<213>  Homo sapiens

<220>
<223>  LOC120376 gene - 110674775 - 110684946 of chr11; NCBI Entrez Gene
       ID: 120376; Build 35; sequence includes 20,000 bases upstream
       of initiation codon and 20,000 bases downstream of
       termination codon of the gene

<400>  19
agccaacttt gtaattggta aaagttccta aagaagaaat gggtttcctc agagggtgct      60
aagttctgtg tgcctggata ttagatgcac ctggtgttag taatatggag agaatgtaat     120
cattgaatga gactctataa aatttcttcc aactcttgag agtctataga atcattaaag     180
tcactgcaaa agagctcgga ttcaatgaac tggatgttga ggattcttaa cttccacaga     240
agctacaccc tctccactta ctttgctcca ggacatctcg gagccatgat ttgcgtattt     300
taagactcta cacgcctaga tactcttcct tcagaggtca gcttaggcac tgcatcctgc     360
aagatgtgtc tcccacccca ttcttgacac cagtttaggt gcccgcatca ccccatcttt     420
ccctgaccct gccacttctc atactacacc gaatattctg ttttgtcatc aatcacccct     480
gtattggtgt cttagggctg cagtaacaag ttaccacaaa tgggatgact tacagcaaca     540
gaaatgtatc atctcataga tagctctaga ggctagaagt ccaaaatcaa ggtgtcagca     600
gggccatgct ccctctggag cctctgggga gaatccttcc ttgcctcttc ctagctggca     660
gttgtcactg gtgatcctcg atgttccttg gcttgaggtt gtatcactca aactgctgcc     720
tttgtcttca catgaccttg tcccatctgc atctgtgtct ttgtgcgtcc ctctcctctt     780
cctattagga catcagtcac atgagattta ggacccaccc taatttaata tgacctcatc     840
ttaatttaag tagttacatc tttaacagcc ctattagcaa ataccgtcac attcacaggt     900
atcagggatt aaggtatcaa tataactttg ggtggcttat gcctgaaatt ccagcaattt     960
gggaggccga aacaggtaga tcacctgagg tcaggagttc gagaccagcc tggccaacac    1020
agtgaaaccc atctctacta aaaatgcaaa aagtagccag gtgtggtggt gcgtgcctgt    1080
aatcccagct actcaggaga ctgaggcagg agaatcactt gaatccggga ggtggaagtt    1140
gcagtgagcc gagatcgtgc cattgcacta tagcctgggt gagagagtga gactccatct    1200
caaaaaaaaa aaaaaaaaat atatatatat atatatatat atcttttgga gggggcacat    1260
aatgcaaccc accacattcc ccaacctctc cctgctctct agcctgtggg ttctttgaag    1320
attgtgtctt cattccacat atttttctta cgtctattat gaactacacc ctgttctagg    1380
cactgagggc acatcacagt caaggtccct acagttggtg gagcttcttc ctccacatgg    1440
attcagacag taaacattga agtggccctt cagtgctgaa gccaaacact aagtgcatgt    1500
atgcacatac acacatacag taggcgcccc ttagccatgg tttccctttc tgaggtttca    1560
gctactcatg gttaactgca gtctgaacat actaaatgcg aaattccaga aataaacaat    1620
ttctagatct taaatggcat gcctttctca gtggtgtgat gaaatttcat tcagtcccac    1680
tctgtgctgc ccaggacttg aatcttacct ttgtccagcc aatccacact gtctacacca    1740
cctgcccgtt agtgtctcag tagccgactc agtgattgga tcacctgtct ctgtatggca    1800
gtgcttgtgt tcaagtcacc cttattgtat ttcacaatgg cccgaaagca caagagtagt    1860
gacactggca atttagatat gccaaagaga agccggaatg tgcttccttt aaatgaaaaa    1920
gtaaacgttc tcaacttaat aaggaaagaa aaaaaatgta tgctgaggtc agacattggt    1980
atggtaagaa caaatctttt gtctgtgaaa ttgtgaagaa ggcgaaagaa attcatgcga    2040
gttttgctgt tgcacctcga actgcaaaac tgtggccaca atgcatgata aattcttagc    2100
taagatggaa aaggcattga gtttgtgggt ggaggacatg atggcaatca ggtgtggtgc    2160
tatcctcagt tttaggaacc cactggggag tcttggaaca tatccctcgt gggtaagggg    2220
aggctactgg atatgcgtat atatagtcac ttaatatata ttggatgagg gaatgttaac    2280
tggaacagta gaagatgaag gataaattac tacatttcat ccagccagtg ctttctggca    2340
tgtttatcaa aatgtaaaag tgtggttgct ctttgtccct tgtaaaactt tgttctatgg    2400
tacttgaatt cttattctca gttgacctga gggtcaaccc aactccccag ataatacttc    2460
tgaatatcat aaaaccaagc ttataaggtg tattcctaat ttttattaaa ttcaaactta    2520
caactaaaga aaacacttga attctgttta ccttccaggg cagcgtcagt tcctctcagt    2580
ctccatttgt gcagatgcca ggtgagtact ttaattcaat aagcaattat tcccttttga    2640
atttaaatca agcatctggg cttccattct ttcataacga ccttacaaca cttccaaaac    2700
tctgctcctt taaaatagtt ttgtctaaaa tacaaaatca gcgaaggaca gaactcatgc    2760
agtaatttct gaatttaaaa ttcattttgt cttgaaagca gcccttttat cccaagcttt    2820
tggggcaaag gcaatataaa ttatgccctt gatacagttt taaattgtct aaagttctga    2880
caccatgatc tgatagatga ggcatctgct ttcaaacaga aaggggaaag ccgtctgcga    2940
gcagagggag tcaggcattt ggttcagtat tttgaattca tttcaatgcc tatgtctcat    3000
taaatactcc tgtggggatg taaaagggga attcctaata atactgttgg acattcttgt    3060
ataaacacac accacacagg caaagacaat ctcatttagt tcaactcaga cgcggcacat    3120
tccctaatag ttttatcacc ttcagagctg gataagggaa aggaataaga gcaatcgagg    3180
atcccgaata tttaaatgtc tgtatgttgt ttcccaatca tatatagtgc cattgaaatt    3240

tggtggcatc tcctgccttc tacagaaaaa aaaaagaaaa atttccatag cttctcttta 3300
tctagaaatt atcctttctc tttgtacaga gaaaatccat gctaagagag cagaggcttt 3360
tttcttcgtg gttgttttga tttggaaatt gggagtatag atgagagcct ggaaaccttg 3420
ttttcatatt ctcaacctta accgctctca taaatataat ttcattgtat gaaacagaac 3480
cttatttatt tatattaagg ttcagctgca cagatttttt cccaaggaag tatgcttata 3540
atgaaaaccc taacttcagc tttcaaatgc atgagcaggt tagaagttat agaatgtaag 3600
atgaaagtga agtgagtgga gtcgtgccac aggtttgat ggggaatttt gatttcttct 3660
tctggatgaa taaattcaca tgccattctt gaacacaatg ttatactact aggtttaaag 3720
aaaatgtata aggcatgaga tctgccccct tgggctcgca ggttgactgg aaagatcatt 3780
gtgactcagg tgaaagagga atgagcataa tataaagtgc cacatcctgt ggaacgggct 3840
ggaattctgt gtctatagaa tcagaagctc agaagtggtc tgctgctgtg gttggggtaa 3900
ggtaaggaag acaggcactt atatcttctc cccagttcag cctttttgtg acctatgacc 3960
taagggtgtt ggtgttagca ttagatcaca cccaaggcca gctggaacaa ttaaaccatc 4020
agttttatag acctcaagtc tatgccctca aacatgttct gaatgatcag tgttttctgc 4080
tacaactgag ctacggggtc agaaagtgtt gagcaaacac accttttttcc ttggaaacca 4140
tttcactgct gggcttacct taaatgaagc tcttatcagt ttctgtccct ccctctctcc 4200
accaaggaaa actttttact tttttttttt tttttttgga gacggactct tgctctgggc 4260
gagatctcag ctcactgcaa cctccacctc ccgggttcaa gtaattctcc tgcctcagcc 4320
tcctgagtaa ctgggactac agcagctgtg attacaggca tggtggccca gctgattttt 4380
gtattgtttt tagtagagat gggtttcac catgttggcc aggatggtct caatctcttg 4440
acctcgtgat ccgccagcct gggcctccca aagtgctggg attacaggtg tgagccaccg 4500
agcctgcacc accaagggaa actttatgta gccggcactg tcttcctaaa cgtgctgggc 4560
cctggtgagt tgagtgaagg agtccatgag gccacaagag ggcagcaaat aacataaaac 4620
tcatcaaaac cctaaaacta caggcctcta aaattggctt ctgtgtggcc atctaatcct 4680
aaagaaagtc atttattgat gtctgccagg cgtgcgaccc agtgtaaggt caatgtgtga 4740
agaattagaa ggcagagtac agcctgcctc tttgaggtgg ggttatatgt gttaccttat 4800
ccagacatag cgatctcaga taacaggcca ggagcacctc ttgttaacag ttctatgtat 4860
atggagaggt agctccgatg ttgtgaaaaa gatgcttcct tggttaaaat atgaatgcca 4920
agagattatt tcatttatgt cttcctagca cagcgccggt caccgggaag gtactcagta 4980
aatgtgaatg aatgaacaga cacggtactg tctaaaatga actcacccct aaaggcgaca 5040
atgttttcaa acgttagtta tttgggctaa tgcaggaata tttcacgctt ttcaagacat 5100
ttctctattt ccatgttagc gttggagtca ggcacgcgtt agtaacatcg tttcccaggc 5160
agagactcag ctgcaaccga ggccttgacc gccctgggag gaattttctg cccatgtgtt 5220
tttcatttgg caacaggttc gccggtcacg tcaggttact acggtgtcag aagatctttc 5280
ttatctgact cagacttcca caacagtaaa cagttttcaa atgacgtcta cacctccagc 5340
gtggggaagc cgtttccctg tgagtcctcc gcagggcaga gccatgcggc tctcctggag 5400
ccctacttcc cccaggagcc ctacggagac taccggcctc cggcgctgac gcccaacgcg 5460
ggctctctgt tcagcgcctc gcccctaccg ccgctcctgc caccgccctt ccccggagac 5520
ccagctcact tcctatttgt gagtatgcag agacctcgtg tgagggagta aaagaggggc 5580
gaggctcgcc ctctggccag ccgctgtgct tggcatccgg gttgaagcca ggtctggctc 5640
accctgtaga ctccagaggc tgctttgcag ctgcctagag atgccaggac accctccgag 5700
ggagccttgt tcaaggatcc ctgttagcca tgacagccag gagcgtctac gcagggcttg 5760
gaaccgggag gaatgtgcag caggatttat atccattttg ttaatgctct actgatattg 5820
cctgatgttc ccccaccctg ttagtgccag tcgtcatcat tcctgcagcc cctaaggaag 5880
gaacactgaa gctgcatctc agacatgcta ggcagtaatg agatggatca taatctccca 5940
aggagggtac atagggctcc tctttctttc caagtcccct gcctcccctt ccccacattt 6000
ttagcctaaa aatccttgtt ctctctcaat ccttccaaac aggtgagttc ataaagatct 6060
gaagattgct aatgctcata gagaatagta aaggtttcta ccacaaagta atgaagctct 6120
aaagacagga gtttagacaa aagggagagg atgagaattc acattattaa gtatactcta 6180
tgccaggaaa tgtgatgtgt tcattcaata cttgcaacaa ctcaggatat atttatgaaa 6240
gtcccatttt acagatgagc aatgtcgggc tcagagtcag gaggagccac gaatgaatcc 6300
aaatctttct agctccaaac ttacgggatt cctacctctt tcctcctgct ttaatgagga 6360
cagagtgggc ttctcagtcc cctcaaaaat tcaccatgga gttgaaggcc tactccgagg 6420
agtgggccaa gcgctgacct tccccactgt gtaaatggca ttctaggacc cagactaatg 6480
ggagactcaa aaagaatcca agacagagtg cattgcacac agtaaaatgt tgcatacatg 6540
tgcattttat tagtctcaca tttcactcat ttaatgttgt cttctgggaa aaggaatctc 6600
aatatctata gcaaatctaa ttctagagta ggaagggagg aaaactttca ctgctggtgt 6660
caaaagccgg agagaagagc agcctgagag ccaggcttca ggcagcagag agaggggacg 6720
aaggctgccc tgaagccaca gtctggcagc acacaatgta tcatcagagt gtcacctagt 6780
gaataacctc cgtggccttt ccattctcct cagagggact catgggagca gacgttgcct 6840
gacggtctca gccagcctga ccctgtgtct gccgatgccc tgctgacctt gccacccagc 6900
acgagttgcc tctcccagct tgagtccggg agcatcgccc agcacagggg ctcaagctgg 6960
gggtcatccc tggctggggc tcagtcatac tcgctgcatg ctctggaaga tctgcaccac 7020
actccggggt accctacccc gcctccttac cccttcaccc ctttcatgac ggtgtcaaat 7080
gacctaccgc ccaaggtggg gccactctcc ccagatgagg aagcagacac cggttccctc 7140
catgaccctt ccccttgggt gaaagaagat gggagtattg cctgggggtc atatgaatgc 7200
cgcagagctt attgagggga gggccaaatg acttctgggt ttttctttaa cttgaggtgt 7260
attccaaaga ttgttttagg tgtgtcagcc cattcaggac tggattttca gaataagcct 7320

caatgctgct ctttgttagt ggacgagggc atttatttgt aatcctctcc actgtaaccc 7380
cactgaaata aaacagaatg ttaattaaat gtgtcagaga ccgtattcta gtaagataac 7440
cttacaatgt ggtcttttg gaactgttgt aacccattac gttctgaatc ttctgttatc 7500
tttgaagtat catttttggc actgattttt cttaaattac attaaaaagt ttatgcagat 7560
cataaaagat actgatttc ctgtcagatg tttcttagtt ttaaactatg tgtattctgc 7620
agtaaaagac actggatagg atataatata gatatgacca cataaaactc attttatagc 7680
atgtgacttg aaagagtgaa gtgtttcata tacatatatt tatggcttat tatagtgact 7740
tgtaaaaccc cagttaaaaa aaaggtacaa tctataaata tgttattgtc cataaaatag 7800
gatgagatca ggaaactatg gctttgtaca taacaaagtt attttttgac tctatcctaa 7860
agcccagaat gtccccaaat tagttcaatc caaaaaagtc caaaaatttt ctttgatcag 7920
tgatggcctt aattaaacca gacattgtct ctctgatgga ataaacctac agttatcagc 7980
atgaatgagt aaaataggca ttctgtaatc aaatggaaat tttttttttt tttgagacag 8040
ggtctcactc tatcacccag gctggagtat agtggtgtaa tcatggctca ctgcagcctc 8100
aatctcctgg gctcaaggaa tcctcctact tctgcctccc aagtagctgg gacccaggtg 8160
tatgccacca tgcccagcta gtgtttttat tttttacttt ttttttaaag tttttgtaga 8220
gacaatgtct tactatttgc ccaggctggt cttgaattat tgacctcaat ctatccatcc 8280
accatggcct tccaaagtgt taggattaca ggcgtgagcc accacaccca accaaattca 8340
taccctttaa agaaaataca gaaaatagga ctaagctgtt ggcaagaaac agaccacaaa 8400
acaccaagaa gcaaaaggac tttggagaag gacatattgc catgtgtgag ccgatggctt 8460
gacaaacagc acctcctatt tggtggctta ctagtcggaa aagtgcagct cttactatgt 8520
gtcaatgaag atattgacat ctgaagcatg aatctgtcca cattggattg gtgtgggttc 8580
ctttgctaat tgggttccca gcataattcc agcatgccta tctgcggcca agctcatcat 8640
acatgcctgg tatacactga gactttgcag atgcatgagt ggaggccccc gggttgtacc 8700
tgcacccttc ttcttagctt cctaccacct gtctttctgc acttcattct cacatctttt 8760
ccaccctctg gtccctcctg atccaatgcc atttctctcc atcttctgga ggccaaggcc 8820
ttcattttcc ctctagttgc ccaaagaccc tcaacacaat ctccatcaca tcctctgtga 8880
ctgtcccata ctctacctca gcaaggtaga agcccagaaa ttccaacaag ggattaatt 8940
cacacttaca agcaaataat tgatattcct tttttttcca ggccttcaag gtattttctg 9000
gttaagcaaa ctcacttcta gtttatggag tgccaccaga aatgcttggg tagtgagctt 9060
tcgttcacct catcctttcg gctccctcca ggcagagcat tcttcaggga gtagatcaag 9120
agtgaacgcg tggggctctc tgctcctggg cagcactgcc aggccccctg ccccacttcc 9180
tccccctcca ccagctcctt tgggcagcct gaactttgct ttgcttagtg cttgctcttc 9240
agtgaaaagt ggcaacattc acagtgcaag aaaggagctg ttctttccta aatccagcat 9300
gacttgcaac ctcagtcttg gttcccaata tggctggtag aaccatctgt cattcagcag 9360
ttactcatac ctgaagagca ggcctgtgca tcagaaagaa gagatctggc cctctctgca 9420
gatatagagg tcttcacaag ggtctcaggg tcattttcct agattggtgt tgtaaagttt 9480
aaaatgattt gtcctctact gcttaccatt ttactgggta tcagtaacac agtagtggct 9540
gtgtaaatca tgaatatgtg tccacataac tttaactttc atgtgattat gactctgaat 9600
ttttgtttgt ttgtttttg tttttttaat tattattata ctttaagttc tagggtacat 9660
gtgcacaacg tgcaggtttg ttacatatgt atacatgtgc catgttggtg tgctgtaccc 9720
attaactcat catttacatt aggtatatct cctaatgcta tccctccccg ccccacccca 9780
tgacaggccc cggtgtgtta tgtccccctt cctgtgtcca agtgttccca ttgttcaatt 9840
cccacctatg agtgagaaca tgcggtgttt ggtttttgt ctttgcaata gtttgctgag 9900
aatgatggtt tccagcttca tccatgtccc tacaaaggac atgaactcat ccttttgtat 9960
ggctgcatag tattccatgg tgtatatgtg ccacattttc ttaatccagt ctatcattgt 10020
tggacatttg ggtttgttcc aagtctttgc tattgtgagt agtgccacaa taaacatact 10080
tgtgcatgtg tctttatagc agcatgattt ataatccttt gggtgtatac ccagtaatgg 10140
gatggctggg tcaaatggta tttctagttc tagatccttg aggaatcgcc acactgtctt 10200
ccacaatggt tgaaccagtt tacagttcca ccaatagtgt aaaagtgttc ccatttctcc 10260
acatcctctc cagcacctgt tgtttcctga ctttttaatg attgccattc taactggtgt 10320
gagatggtat cccattgtgg ttttgatttg catttctctg atggccagtg atgatgagca 10380
tttttcatg tgtctgttgg ctgcataaat gtcttctttt gagaggtgac actgaatatt 10440
atgcatgttc acgtgttcct tcatctccta acaacttgaa gatgcactgg ctctcagaca 10500
aagagagctc aacaaccaga ccttggaaca gaggtaaata cagggagcac gacaatgttt 10560
acattcttct taaagcatcc tatccctttc atagcatttg actaaattaa cttataaaaa 10620
agagacacac tcaggcccac atatgcctgc tagtgtccac ttcagttatc tagtctaaaa 10680
ggggtttggg gagtagggct ggtgctaata attccaaata ataagtgatc ttcaagaatt 10740
ctgtttagtt tgggctgggc gcagtggctc acgcttgtaa tcccagcact ttgggaggcc 10800
gaggagggtg gatcacaagg tcaggagatc gagaccatcc tggctaacat ggtgaaaccc 10860
cgtctctact aaaaaataca aaaaattagc tgggcatggt ggcaggcgcc tgtagtccca 10920
gctacttggg aggctgaggc aggagaattg cttgaacctg ggaggcggag gttgcagtga 10980
ccgagatcgc gccactgcac tccagcctag cgacagagcg aaactctgtc tcaaaaaaca 11040
aaaagaattc tgtttagttt gacaatgtgt ctctgtttta catatatttt aatttgattc 11100
tgttttattg ttcagggaat actttgttaa aaggtgaaat caaactacaa aattatgaca 11160
ggaaaattaa ttctatttcc ttatccaacc ccatttttct ttcaattcct ctctgttttc 11220
tttcctttct tttttttttt ttttgagaca gtcacccagg ctggaggagg gtggagggca 11280
gtgacacaat ctcggctcac tgcaacctcc gcctcccagg tccgagcaat tctccttctt 11340
cagccacctg aatagctggg attataggca tgcaccacca cacccagcta atttttgtat 11400

ttttagtaga gatggggttt caccatgttg gccaggctgg tctggaactc ctggcctcaa 11460
gtgacccacc tatgttggtc tcccaaagtg ctgggattac aggcgtgagc caccgcgccc 11520
agccaatttt ctacaacacg tggggactaa tcgcaccttt tcataacaat gtaccacgcc 11580
aacatacaaa aataggtgca ttcttctctc cctcaccttc cttaaacctg ctattcactg 11640
acttacaact ttcctgagaa agtcagagaa aattaaagag ctctgtttca acactccatc 11700
tctttcaggt cttgtaagga cacaggatat atttaagact taaagtaatc agagatggta 11760
tgtcaagttc aagccactaa agaaagctga ctagatgata atgccaaaaa gaaattgagc 11820
acaatttgcc catgcaaaag ctgcaatata gacaatcatg ccatgaaaca catgcagtat 11880
gtagttgaag gcatttgcat tcctcttaca atattacaca aaccccaaaa aaaggagaga 11940
agaaaactac atctttacct catttattta ataaatgatt tcaaatgatc attatgactt 12000
gctatagctc agttaataat acaatttcat ggtactattg ggtaggagga gaagacagaa 12060
gcactctaaa agaaattgca taaagggaaa ataattacta atgccaatag taaaacatac 12120
attgacatac atacatacat atatacatac gtacacacat gtataaaagt attatagtat 12180
ctttgtgatc agtttttcga tatttaatgt aaatatctaa atagtgtagt cacaaggaat 12240
aaaacatcag ttatatttaa agtaccaaaa taatgattga tgtctaaggt ttaagtcaca 12300
ttacattata gtgattctac caaccacaca ccaaaattat actctttact taaggctcca 12360
tcactgggtt ctgtctacag tatcctctct atgcaaaaca atattacact gttcatctca 12420
cacaccatag actctagaga ggttaagtgc aaaatcacac actctagaca aagtggttct 12480
ccagctaaag agactcatgg ggaggactca tgagtggctc acctctgtgt cccagcacct 12540
ggctcgaggc ccagaacact gtagatgctc agtaaatttt tgttagattg aagtaaaatt 12600
ataccataaa gatgagtaac tttttagccc ttggttctat gggtgacagc tctctgaaac 12660
cagtatctga gaggattctc accatgttta gtatctaaca ttagccatac tctctccaaa 12720
ttcagacagt aggtggatat tgggctttag tggtaatata tttaagatga ctggtttagt 12780
ctttttagat acctttatga aataaaatta ctgaaccttt gaaatagcac agaaaaatgt 12840
tgatatattc atttagtatg tacatgtact atgatctgtt gctaagaaat cttataaata 12900
aaagttattt aatgtttgat cattatctct ggtgtgacta aaacaagtat gataaatcag 12960
gaacaagaat ccccaaaggt caagctaaat aaacattaaa atattctata gttatttgca 13020
aatgatggca ttatattata tttgattttt gatatggaag tatcctctga ccaggtgtgg 13080
cagttcacgc cgcctgtaat cccagcactt tgggaggccg aagcggaagg attgctcaag 13140
cccaggaggt caaggctgca gtgaaccatc attgcaccac tgcactccag cctgggagac 13200
agaatgagac cctgtctcaa aaaagaaaaa aaaagtatcc tctaattata atctctgata 13260
aataacatac gtgcatttgt aatatgcaat gtcattgtta gtacaattaa aaaaaagaac 13320
agtaaaaaat gttagaagtt ccaagaggaa tgaaatgtgg accaaacata cagtatctag 13380
gaattagctc ttttggatta ggaaaattga tagatatacc tgctaaactt cccccctaaga 13440
aaatatctta acaggaataa gcatgggcaa ggacttcatg tctaaaacac caaaagcaat 13500
ggcaacaaaa gccaaaattg acaaatggga tctaattaaa ctaaagagct tctacacagc 13560
aaaagaaact accatcagag tgaacaggta acctacagaa tgggagaaca tttttgcaat 13620
ctactcatct gacaaaggga taatatccag aatctacaaa gaactcaaac aaatttacaa 13680
gaaaaaaaaa caaaacaacc ccatcaacaa gtgggcaaag gatatgaaca gacacttctc 13740
gaaagaagac atttatgcag ccaacagaca catgaaaaaa tgctcatcat cactggccat 13800
cagagaaaag caaatcaaaa ccacaataag ataccatctc acaccagtta gaatggcaat 13860
cattaaaaag tcaggaaaca acaggtgctg gagaggatgt ggagaaataa gaacactttt 13920
acactgttag tgggactgta aactagttca accattgtgg aagacagtgt ggcgattcct 13980
cagggatcta gaactagaaa caccatttga cccagccatc ccattactgg gtatataccc 14040
aaggattata aatcatgctg ctataaagac acatgcacat atatgtttat tgaggcacta 14100
ttcacaatag caaagacttg gaaccaaccc aaatgtccaa taatgataga ctggattaag 14160
aaaatgtggc acatatacac catggaatac tatgcagcca taaaaaagga tgagttcatg 14220
tcctttgcag ggacatggat gaagctggac accatctttc tcagcaaact attgcaagga 14280
caaaaaacca aacaccgcat gttctcactc ataggtggga attgaacaat gagaacactt 14340
ggacacagag tggggaacat cacacaccgg gacctgtcat ggggtgggtg gaggggggag 14400
ggatagcatt aggatatata cctaatgtaa atgacgagtt aatgggtgca gcacaccaac 14460
atggtgcatg tatacatatg taacaaacct gcacattgtg cacatgtacc ctagaactta 14520
aagcataata ataaaaaaaa gaaaatatct taacaggaat aatccaaaag ttttaatggt 14580
tttaataagt taatttagtc ataaatgaga gatcactcaa ctgttttatt tcttaagctt 14640
ggtaatacag tcatagattt tcatcatggt attctttata tgtttttgta tgccttaaat 14700
gttgcacaat aaaaacgttt taagtaatag aagaggaact cttcaagcag tgtacggtgg 14760
tgtttttctt aaaggatgaa ttatcaaatg cttgctcccc atggaagcct cttaaggaaa 14820
agttaaaact ttgaatatgt cttatgggaa gagactgacg tagcacaaat cattaaaaaa 14880
ccttcaccta attgtcttcc tggtcatttt catatggacc aaatgttcac tctagggccc 14940
aaaaaacatt tgtttcctgc atgtattcaa tcttctggaa cttgatattt cccacgaggt 15000
ttggtaacag tgtcccaaag tcaaggtgac aaaagaactt tgcattaata aagacaattt 15060
cgagctgtag tcaaatttgg agggaataaa ctaccccttt tccttatact gaaagtcttt 15120
taatatgcct gtgccttagt tgaataaaat ctcctacgtg gggggagaa aacaatgctc 15180
tgcagtgtca tttgcataca agactgccaa aatttttcag tcaaaatatt cgtttaaaac 15240
tttaaaaaga aatctattcc atttccatca ctttgggtat ggactgggta taagaaggtt 15300
aaggtcaaat acaggatttc tgaggtcaat ggcaaggata gccagagatc tgaaaaacag 15360
tgatatatga agaactaaag aaactggatg aggagtctca aaaggagaag gcttggggtg 15420
aagatgggat gggcataact tcttcagagt tagtactgga cctggtcaga gtagctaatc 15480

agtatatgat tgttgaatga atgaataaac caattaatga actaaacaaa ctcacaatag 15540
aaaagcagta gtacagaagt aggagaagag agtaattgat agcagaaatc aaggtttact 15600
tatttttgta ctcctggtac ttgataccat gcctagtgca tagttggtgc ccaattaata 15660
catgattgga ttatagcata atcactgatc actacattta aagcagtcca atagtggaac 15720
aggctgcctc acaaatagtg agctctcgtt tcctctacac agattctact atgggccagg 15780
atctcagcca agcagtggag ccagaggctg gacagacaca agcagagatt ttggaaaaag 15840
actgagtccc tctcagcact tcaaagatcc cttccaacac ccaaacatca aaagccatgt 15900
tatattgcca agcaagggtg cctatgaacg cctgttgaaa ccccacaaca atttttttcag 15960
ccatttatta aaaccccaaa gagactatca ggacttcagg gcaaacatcc tagagatagc 16020
aatgccccca gcaagaagaa aagagttctc agtctgagag gcacaaactg taatcagtgt 16080
aattaacagc ttaccctcac ctctagccag gcgtttcttc aagattctag tgtttccagt 16140
ccttacagaa cattctcaag tcagcaggct acactgtctt agtgtccttg tacaaactga 16200
tggtaaaaca gatagcaccc caaaatattt ccacatgcac aaacaacctt gaaacgtgtg 16260
ttcgcatatc ttacaagcag gtatttttcc aatgccactc acagcctatt agtgggtcat 16320
taaattaact cagtgactcc caaccaacat atatttccca taaatagaat gaaatggaaa 16380
aggtggacta gaatagaaaa tatcagagtg cctgacatat gtaggtatat cttgtttcat 16440
gaaaagtttg ttttagaaat ataaatgttg ttatagatac atgtgaacat gtaaaatgga 16500
tttcttattg taggttaaag tccaaaaagt ttaaaagctt cttgtagaaa agatcaggaa 16560
gacaacatca gtagttaaga gcaaaggccg ggcatggtgg ctcatgccta taatcccagt 16620
actttgggag gccgaggcag gcagatcaca aggtcaagag attgagacca tcctggccaa 16680
cacggtgaaa cctcctctct actaaaaaca caaaaattag ctgggcgtgg tggtgcatgc 16740
ctgtagtccc agctactcag gaggctaagg caggggaatc acttgaaccc aggaggcaga 16800
gattgcagtg agctgagatc atgccactgc actccagccc ggtgacagag taagattcca 16860
tctcaaaaaa aaaaaaagcc agggctttgg agttaggcct aacaaagtct gaatcctaat 16920
tctgctactg atatactcag tgatcttggc atgccactta ttcctctgag cctgtttcct 16980
cacctgtgga atgtgaatgc tatacaatat agcacctgat gtccagtaaa caataatgag 17040
tctcctatga agtcttctct gagtctccta tgaagaacag ccaatgctgt gggtacctgg 17100
ggtctcaggg ttgctctggg cctgatcatc cactcagatc tgtaaggagg atttgcagga 17160
tccatttaga aagatcctcc cttacttcca caagcatggc ctttggctct taaatacctg 17220
tgctggggtt ttgtaattat agaaacaaca ggaaccaaaa ctcattaatg ttgagctaca 17280
aaccagaggg aagcttcttt ctcaaaacag ggctcaggcc tagaaaaatc tagttttctg 17340
aaatcgctag ccagcaacag cactgagatg gccatcccag aaacaaggcc aacacagaag 17400
cacccataaa ggctgctgga ggttgggaca aagagatcct tgctgtcctt acagaccccc 17460
tgacttccaa ggagctcccc tctcacccag cctggcctgc cttctccaca gggtagctga 17520
tcgtcagcat catcttcaat ggtgttgtca agagcactca gtgctcctgc catccctgtc 17580
catctccaca tgaaaggaga ggttagcact ccagcctggg cgactgagca agactccatc 17640
tcaaaaaaaa aaaaaaaaaa aatagaaagg agaggttagg aaggagatga agcctgaaag 17700
cactgggagg acaccccagc ataggggaac acagagacac gtgaagccag ggagaaaacc 17760
agtagtacag ataagaaatg gaagaaagca atgacaggaa gagagacaaa gaaacacaga 17820
aagaaataaa caaaaatgca acaaagaagg tttttaaaag caaatcaaaa aataagagta 17880
taacaaaaag gaggagagca gcgaagaaaa gaagtaatgg aagacgctat gaggacaaga 17940
gaccatccct agattccaag ctgcttggtt cactgttcct tcagaggggg caaggcctag 18000
gcacagcgag gaagaaggca tgcagggagg ggaggtgcca tctccacccc cattgtcctt 18060
cccctcctca ggtctggtcc atcagggaaa cctaggacac aggctcaggg gcatttgtag 18120
aaagaagttc cagaagctgt cagatttcaa tacatccatc ctgccctcca ctgccaggtg 18180
ccacctcagg tgttcccagg cctgctcctc tcccccagag atgtggcttc agctgccaag 18240
caattcctat cagtcttgtc attctcagtt gccctttccc taagctctgt gtgtttccct 18300
ctgtatttga cacatttggg ggcaccccaa tctttttctt gaggcctctg gggctaaact 18360
acactttttg catttctgag aactcagaga ataaacagca gaggcctaaa accatggact 18420
ggtcttgaat cctggaaata tcttggttat ttgcaaaagg aacctcacca tgcctctctc 18480
ttctcttgct gtccccttct gtggaggggg cgggtggcta tgagaggact tggcctggca 18540
ctctctccct ctgtggacca ttcctgtctc ctcaccttcc aaatcacatg gaagaggcac 18600
agtaagtact cacagaagat gacccagtag aatcccaagt cattagtgag gggcttgggc 18660
ctggaccaag gggaagcct gatcccaagt ccctccccct tctcacccca atgggagaag 18720
gcttgaacct aagtccacat cctttcagtt aaaatgagca ggagccaaac ttgtcattgt 18780
ttcctaacat gtgctcctac agcaaaaagg caggggtggc tctagaaggc gaaaaagcaa 18840
agagggcact tcgcagtgcg gtggtgaaac ccagagtcag aaagtctcca gaggatgagg 18900
ctcctggcat ccccagctct gtggggagga ggaaagggtc ttaatgggca gtcattctga 18960
gcagaaaccc cagtgtcggg cccaggaaga gcgggatgaa agggggaggg aagcccgctg 19020
gtagccaggc ggagccagaa aaataggagg tgcagagcga aggcctctgc taggccgctg 19080
gcctgccacc tgggcccaga gcgacttctt gctgtgccac acccactagc tgccatgttc 19140
attcctcaaa acctgagatt tcccctcaaa acacagatgg gggatgcggg aatcagtctg 19200
gccagccagt ctggctccgg tgttcatggc cagggcagcc taagtcactc atggcttctt 19260
agctgccctg aaggctgaaa tacagagcat ctttgaggac atctgctcta tctgtgggaa 19320
ggaggacaaa atgctcaaac tataataaaa gcaagggcca ggacccaaag ggtctcact 19380
ccctgtttct ctggactctc tgtggggtgc ctgtgtgcag gatttctgca gaagatagtc 19440
cattttgtta agcctttgaa tattcaaatt gaaagtatcg cgcaatcatt gattgtcaaa 19500
tcatgggttt ctagaggttt taaatacttg gcatgagggg gaaaaaactc aaacctatac 19560

ccgaaggtat aaaacattaa ttctgtttgt ctcctgttcc agggacctgg gtgcagccgg 19620
atgctttgtg aaattttaaa aatgttgcat ctctgcttct ctccctgccc caggagccct 19680
cccaggcgct gagtccctgc tgctgcagca tccagccacc gacccagctc ctgctgacca 19740
cgatgtcggg taactcggct gcttctccta gggtcgggtc aggcggccca ggggcatttc 19800
aatccgggtg tgtttccacg gcttttcctg catagaacag agaggcacaa agtcaaggca 19860
tgcgagtaag ggccggggat ccggagagga cgcgagccac gctgtggccg ctcccactca 19920
gcgcctgtcc cggagggcag aggcccagag gccgagttgg ccgctccgga cgtccgcgta 19980
ccccaggccc ccgcccgccc tcccacgtat ccactgttgt gtcctgagac acgcggtgcg 20040
gaccctgcgc gcccagaagc tgctacgggg ggagctagag gctcacagtg agcctgggac 20100
gggggcagag cgcaccgact gcactgagcc gagctgtggc ggtccctgcg cggaccccga 20160
ggggcgcccg ctgcccgcgg agtccggagc cgatcgggga acggggtggt ggggctgcgg 20220
taggctggag cgcggagagg gcgcggcgcc tgggccctgc gggcggggca cggattcccc 20280
tgggtccggg ctaggaagag cgcaatctcc tttcctgggg agaccctaac tcctggaacc 20340
cggcttgagg gggatccctg ctgtccagct gcggtccccg cagtgaccag gcgaggcgct 20400
ggcatcctgg cactatctcc aggcgtccag gctgaacgcg cggtcaccct cggccgccgc 20460
acccagcgca cttcccggcg cgattcctgg acgcacactg cagggtagtg gtcagggccg 20520
gagccgcccc ggactcctgc ggccacctcc accttgaaag ctcgcccggc tcggcttggc 20580
ctaacagcca gtccccaacc tactcactgc ggcttttcgg ggcccggggc tgaggctgcc 20640
cctgctccca gcgctcaggg gtccctccca gcgaacccct cttgagcgcg cccgccacct 20700
cccgcgagcc ggggcagtcc gaccgggccc cgccagcccc ggggcggccc ctaccgaagg 20760
gcggcctccc ggcggagtgc agggtcatcg ggagccccgg acggctttcc ccagctcgcg 20820
ctgcgcggac tcgccccgga gcctcagtgc gcctcggaga aacgcctgcc ccagaaaggg 20880
agggggtagg gagagagggg aagaagggga gagtaggagc gggggcgaag gagggaggag 20940
ggcaagatgg agcgcggaaa aggcggagaa aagggacgag ggagagcggg cagaaggcaa 21000
agacagaagg gagcgaggga gggagttcct cgggcctggc ccctttacta ggtcagtctg 21060
gcaggtacct cgccggccca ggacggggct ggccaaacct caccgcttgc tcccgggctg 21120
gcttccagac caagggcacg cagaggtcgg agcctgccca gaagccacac ctggccaggc 21180
ggagaaggaa gggactcggg acttccatgc tagtgggaac atcacagtgg ggtgggctac 21240
gtcagccgcc ataatcagag aaagccccct gccccaggcc tggcccctcc ccaggtccat 21300
aataccctct ctctcggctg tctgggtctg aaagccaagg ttcccccgac ctaatcagag 21360
tggtgggctc cgctgcactg gtctccatat tccagagcag aaaaacccgt ttgctattgg 21420
cctaggactg aggcccagcc aggggtggat ggccccacaa atttccacgg attcttttcc 21480
tactggggct ctgaaacctg ctcagctact cttcattctt tcaagtttga aatgtttggg 21540
tttttaccat cctttgacat tttttgtgtg tgttctccga acttagaaac ttggctttgg 21600
gtgccatagc ctataaaagg ggcccccact ctgcaccctg gcacccagac cttggactcg 21660
accactgact cagttgcctt tctccagaaa aaccgaaggt gtatcaaggt gtccgagtga 21720
agatcacagt gaaggagctg ctgcagcaaa gacgggcaca ccaggcggcc tccgggggaa 21780
ccgtaagcat aagcccttcc ctcattcccc agcccatgcc aggcctcttt caaagctgtg 21840
tctgcaaggt gtggaggcca ctcaaggccc cacgcactca ccattcccag acctggcctt 21900
cctctgctgt tcctatgact tctgtaccca gctgcatccc aatcgggaag ctacagagga 21960
cagctggggc atgttgtggg gctcaggatt tgggggagcc catccatccc ccatgggtga 22020
aagtaaagta taaaaagtgc gttggtcctc tgcaggggga ggagcggcca gcaggtaagt 22080
atacatcgtt ttgttaggaa gacagtggat atatgtacag cccttgcaga cccatagaaa 22140
atctctccca gaaattcacc tcattttagg ggccccttcc aggatagcat ctgtgtggaa 22200
catcctccct tccctcctca ttcttctgga actcaccctc tgagatcatt tatctgtaaa 22260
tcatcagtta cctaaagtat ctagcagaaa agggccctgt ctctgcagaa gagacagctg 22320
gagtacagga ccacttccct aaaatgtatc aacaccttaa gcagtttcag gttattacaa 22380
aattgaactg agcagtcata agggtatacc ctgacacttt atacaccttc ctacggtaag 22440
tactgccaaa ggcactcagg gcagcaacag aaccaaataa actaggtggg gttattgccc 22500
aagagagggc agctcatccc taaaaagcct caatcataca attgcttggg ggttggtttg 22560
ggtgtgaaca taggatctga cagctggggc tcatattgcc tctgttagga ttggtccttc 22620
tccagcccat tggcatctgc cccacaacag tttttgtgcc tgatgctgtt catgtgcaat 22680
gtgtgacttc atgaaatagg cctttgacag cgggacttct tgaaatggga gggggcaaat 22740
gatgtagggg aggtaaacac aggttccaga accagatgga ctgggctcag atctcagttc 22800
ctccatggtg tgtatcctca agcaaattac ccaattcctc ttgcctctgt ttattcgtct 22860
tcaaattggg gatgatagga ttttgtgagg gttaatcgag ttattgcata caaagccgtt 22920
agaagaatgc tttccacata gtgagtgctc aataaatgtt agttgtcatt atttgagaaa 22980
taataatatt atctggggaa agcaactaat attccaacca aggattgaaa gccagctatt 23040
tggtaatgtt tgttcacacc ctttgctcag agtttagagt tcagatgcga gccagctgca 23100
atttggcata tacagcacat gcagctggaa aagtaaggac ctccatgtgc agaatttcag 23160
gtaacgcatt aacactccct tacatttcta gaggacttca cagtggttct tcttttaaag 23220
cacttttgca cagccactat ttcatttagt ttttataaca accagcggag gtaagtagat 23280
cagagatctg tacctcgatt acaggcaaaa ttcaaaacct tgaagttgct catggcaacg 23340
ctaaaagtag gactaaggtc tttttcttag tgtaatataa aaacccttct gttggctctt 23400
tatgctctaa attcatcatt ctgcagatct gacgatacta agcaggagtc tgatagcctc 23460
aaccccttca gttcagagat ggttatataa ttaacaaaac caggtacaag ggaactggaa 23520
aatgcttctg gagaaaaaca tcatcaagac tttcataata aaatgtaacg tttccttaag 23580
gagctttggt acttgctaca gttctgctca gctgcacctg gagagcccca tgtgagtgtg 23640

gcacttctgt tgctgctggg cccttacaga tgggtgtggc cactgttccc ttcttagctc 23700
gctacaggag ctaatgtgag ggtaacacac ttcacttgtg tatctgacca gtttctgttc 23760
cccggagtag tttgacttat ttaaattttt ctgaattccc tcctgagaaa taaagcagga 23820
cttctgaacc agtgtcttat aggaacaggc agagtaaagc ctgctctact ttgcttccct 23880
taagactgca tcagcctatg ccctcgcaag caagcaggtt taacacgcat ataatttaat 23940
tacaaaagcc agacgtaaaa tccctcactg gaagccatcg gatggatctg ggattctaaa 24000
gaagctgtga gagcgtactt agtaggcaag ggtagcagac accaggggat gatgttgcct 24060
gcttcttagt gtagaccttt ctggtataac agagatgatc cccagtttgt tcatttgttg 24120
aaggatgcct ctgggaaact ttaagccaag gagagggtta attatatcaa atactacaaa 24180
gtggcaagaa atctatctta atttgttata aagtcagtgt agtatccaca atttaaaatt 24240
ttaagaaggc aatcacgaat ggaagttata ggttgagaca gacattcagt aaagaattca 24300
aatattcttc aagcagtgcc ggaagaaact gtgtgtatgc cggattggaa cactcgattt 24360
taaaaaactt atttcagcca gatcaatttg caaacatgcg catgcacgca cacacacaga 24420
cacacacaca cttgcctttg gggtaaaagg aaacttgagt tagtcgttat caaaagatat 24480
catttacctt tcttttttta aatcattaat tttttaaaca gccttgatgt gtttttataa 24540
attgtagact gtgaattttt gtgctgtttg aaatctaaga gagagttgcc tttctttgta 24600
ttcttggagc aaagtagaaa gtgaacccat gactagaaag ataatatcaa gtaggatatt 24660
gtaaacacac ctcagggaga taacatgggg tggaggcaga caaacaggcc aatccggggg 24720
aggaatggag ttaaggaaag ctctacaatg aggcagattt tttaataccc actccttcca 24780
agtccttcag ctcaagctgc agccaaatat ttgcataaag agctggtaaa ggattccagc 24840
ttcctggttc atgacattac agcatggatc gttttcaaaa ttaatactgc ctcctgtttc 24900
agcagtggct gcctgcacag ctctccctgt ggctcagagt atgggagtga gtatgccctg 24960
taacccaaaa gaggtgtggt gagctcggct tcccagcaca gaatcacaga gcaatacggg 25020
tacctttaaa ctttaaagcc acacctgaaa tttaaaaaaa aaaaaaaaat cctaaattac 25080
tcagtataat tattaattca actgaaaaat gcaggctgct ttactttgaa tagttaaaaa 25140
gagcagtctc ataaactttt aagtatttaa atacagtagt ttaaataaaa gcaactttta 25200
tagatagtag tacaaaacat acaaagtact ggaataattc ccgaccatta actagaatga 25260
aagtaggttc caactcacca accacagctt taagaaaata ttttctattt taatatttca 25320
tgtatactaa gaacatgtga atatttttag ctactcagaa ttaacaaaat taagaattgt 25380
gttctttggg gaaacgactc cctaaggcta agtagccaga tggtatatga aatttttgag 25440
aaaaataatt gactaagcca tagatagttg agattaaata aatgttagtc cttaataatt 25500
ttaaatcgaa tagtttaata ggaagccttt tgctgcattt tctcaaactg ttacattttt 25560
accacataaa attatttaca ttaatttttt actaaaaatt aatcactttt aaaatttaag 25620
tagaagaatc tgatattagg aaaaaaactc cccaagcaag caaacataaa tattggatct 25680
agataaaaca taatattggt tgtatttaag aagtctgttt tatgattcga tttcaggttt 25740
tcattgttcc aatgcaattt tccagattga atcttaaaag gattataaac gtcaaaaggc 25800
atttaaaatt ctacacggac ttaatacacc taccgcaatt ctatgcacct gtctttcaaa 25860
ttctataaaa tatttgttga cactttccct ttcacttgtt ttttacaata tattttgtac 25920
aaaaatttgt caaacattga aaaaaaaaaa gaaagcaact ttttctgaga aggaagaatc 25980
tggcttacct tatagcagaa agaaacatag tcctcatcaa aatgtcttag ctttcccttc 26040
agagcatttc tttcagcctg cttcacggtg atattcttgc tttgacagcg gtccggaggc 26100
agcagtgtcc acctttcaga cccagttgca ccatcttctg caggtcagta cagccagcat 26160
taacagttcc ctcaaacact ctttcaaaag tccatctcaa aagtctttca gtcttgattc 26220
aggggttaag ataggcacca ccatttaatt ggaatccaac caatcagaag gtgaaaaagg 26280
catagtcact ccacatgcaa gcccagcaag aaaacaagca taaatgttgt cttcatttct 26340
gaattctcta ctctctgcta tattgtctca agagtttgtt cttcacattt attccagttc 26400
ctcatcgcca agtaactaaa ctgaactaca aaatcagaat tgttctcagg aacttatctt 26460
tttttagtgc actgttagat ggaatacaga acatcccaag acactcaaat aatgcctttg 26520
ctctgttcct ttagtttcca gccgacaggg gcttccctcc ccttctctgt tcccagaaag 26580
cagttcaaac aagtgtaggt ttctcagccc caataagaat aactcgatta cataaacact 26640
cctacctctg ctactcaaca gctaagagtt taaggaggtc gtatataagg aaacctctgc 26700
ctatggcctc tccccactaa atgtgtcacc gtagcttcac cttaaaggaa cttgagacct 26760
caagaagaac acagtgtcct ttagtccctt tgatgagcac actctaatcc tctcttcttc 26820
cagcatttac agataattat cacaaattgg gcctattaat aataaaatgg aaaaggagta 26880
atgtttactg attatctttg tttgatgttt aacaacttac tttgatcaca tcatgatgtt 26940
aacatgttca ctgagaaaac atttttgctt tttgtacttc cgagccaata gtgttaattg 27000
gtactctatg ttcctggttt ccatgattat catttgcatt ctcatttaaa tcacttctca 27060
aaattctctc ctccaaactt aagttactga gtaagagtga aaagttcaaa aaagcaatca 27120
ccataagtca aaagaattat ttcccgtagc atcagaagtt ttctcaaagc ctcaacaact 27180
ttgtcagtta cttctgagag gtttacgtga actgccggta ggggctggat gtccatggtt 27240
ttacagccca agaaagggga agtttgagcc attatttgac tgtgacactt ggacacaaat 27300
tgggtcacca tcctttttatt tttgccctaa ccagttttac agccagtttc cacattatta 27360
accatgttca tttccctgga gcttgaatta gctaggacaa ctaaagagtc aaaattgcat 27420
tcccgtttac ttttctggat ttgttaagcc ccctttcccc ctagacatag taaatattca 27480
gagtatagat ctccattcaa atctgtgttt tgcaattttt atgcaaaaac ctgccttttt 27540
cctttctcct ctaggactgt attttgagcc tgaaccaatt tcttccacgc ccaattattt 27600
gcaacgggga gaattttcca gttgtgtttc atgtgaagaa aactcaagct gcctcgacca 27660
gatctttgat tcctaccttc agacagagat gcacccggag cctttgctca attccacaca 27720

aagtgctcca caccatttcc cagacagctt ccaggccacc cctttctgct ttaaccagag 27780
cctggtaatt tttctcagtt ctttatatac ctgattgtgt ctaactatgg ggtaatagtg 27840
ctttgtgagt ttgcaccatc ccgtgggaaa tcatacaaaa actatcagca aggaaatcat 27900
ccaaggaaac aagcagagaa gaatttaaaa gtcaatgtat agaagtagcc acacactgca 27960
gggcataaac ctttggatac acacgtaatt tagatttccc aagatttaga tttttcggcc 28020
accaagtaag ttggtgggta gcaataaaaa gaggttcctg gcaatattca tttaatgttt 28080
tttaaactgt aaagtcaaga gacctactaa ttggaagtca gtggtttaaa caatggctct 28140
tggatttttt gatggaattt tgagttcttg gaaatggaat taaggctgtg tatatatggt 28200
aacaaacttc atttcattag gggaggtttt tgctttgttt tgtcattgaa ctggctctac 28260
taactgatag tttgagtctg ctataagcac aataactcat ttgggtctga gatggttagt 28320
aaaaatgaag ccgtaatctt tgaaaaaaat taaattggtt gttctcggtg gatctcaaat 28380
gtttgaaaat gtgttatcct tcaaaatgaa gtaaggtttc tattagtgga aactagttat 28440
ttcagttgtc ttttagagac tcacaggttt gttttgtaaa ccttaaattg taaagttttc 28500
tttttttaagg aatgtaaata ctttttgcct ttggattgtc aaaagtctgt gtgggcatca 28560
gactaaattt tagagcggat gggaaattaa ctctgcagaa tatgcacatt gtcacgggga 28620
aaatatgggg ccccagtgac ccagttcctt cccagcctcc aagttaggtt ttctcagtaa 28680
caattgtaga cttgcttaag agaaaacaga cagtagctct cactccaggg aacaagactt 28740
tagtggcgag acaggtagtg acacatcttg gcatatgcca gggatactcc aagatccttg 28800
aagatccaag ttatgccttt gcatgatcac ctgattcttc atggtgaggg tgtggagaaa 28860
catccagggt gccacctaca gcttagattg tggcatacag caggatttga tatgcaggtg 28920
acttgaagca caatgggatt ttgtggtttc agaacgagca ccctctaaag taggattata 28980
acaaaggaag ccagtgaagc aaggcaattt attgtttgga aagaaatagg taactataga 29040
ttgtggtgtc atgaaatcat gtagtcagca aataaaatta cttctgcaga cttgggttgg 29100
gtgttgtttc gtttgatttg gtttttttacc cctcatttc tcactctgca ttggtaaacc 29160
cacactgttc agttctttga tcctgtctaa gttattaagc tctttgtttc ttggttgtca 29220
tttctctaga tcccaggatc accttcaaat tcctccattc tctctggctc cttagactac 29280
agttactcgc cagtgcagct gccttcatat gctccagaga attacaattc ccctgcttct 29340
ctggacacca gaacctgtgg ctacccccca gaagaccatt cctaccaaca cttgtcctca 29400
cacgcccagt acagctgctt ctcctcggcc accacctcca tctgctactg cgcatcgtgt 29460
gaggcagagg acttggatgc tctccaggca gcagagtact tctacccgag cacagactgt 29520
gtggactttg ccccctcagc agccgccacc agtgatttct ataagaggga aacaaactgt 29580
gacatctgct atagttaata gaaattacag taattcagaa catggcatgg gtatatctat 29640
ttttctacca cgtctagatg acactgcaaa atatgcaact tggtaacaca atatcccaag 29700
cacagtttac atgtcactat ttccaatttt ctgatgctaa gcattcatat gaagtcctca 29760
gacccggtca cagcgccact cctactttgt atgctcatag tttaaatttt tgtaggaaac 29820
tttcaattgt tttacttttt gtataacgaa caaatgctgt ctcctttttt actaataaat 29880
aattttgtat tactaactgt tggatttttt ttaatttcca gtagtcattt ttaattgatt 29940
tgaacattat ccattaattc tggaaattgc taattactct caagtggttt aatcttatgg 30000
attcatgaat ttaaaattat ttttatttgg gccaggcgaa gtggctcaca cctgtaatcc 30060
caacactttg agaggctaaa gcgggagggt cacctgagat gaggagtttg agaccagcct 30120
agcaacatgg agaaatcgtg tctctactaa aaatacaaaa aaaaaaaaaa aaaaaaaaaa 30180
aaaaatagcc aggtgtggtg gtgcattccc gtaatcccag ctactcagga ggctgaaaca 30240
ggagaatcac ttgaacccag gaggcggagg ttgcagtgag ccgagatcgc accacttcac 30300
tccagactgg gcgacaaagc aagactccat atatatatat atatattcat ttaaaatgca 30360
gtgagacacc ttggcttcca tgggatggag ggtaggcaca gctaggatgt tggaaaaggg 30420
aaaccctgcc tcacctccag atgagggggt acatcttaga gcaacatcac gaaaatgcat 30480
ttatgaacca ttagtttctc ttatctaaca ttagtcaatg aagtgttagg acgaccaaaa 30540
agctcatgtt gcctgcagct atgttagcct gtagatcatt ctttttcctt tccaaagcat 30600
tgctttggaa actttctgag caaatagttg atcttttcct tgagagctgc catacagtta 30660
atgcagccca aagtttagaa gtcattattt tctatttctg aatttttggc tttaggttct 30720
taaatataca tcacttttgt ttacacgttg atttatagta ttgactatgt tgtttacact 30780
tgaattttag agtttgcttc ctctgattaa tctatttgaa gaagcccagc tgtctttcaa 30840
ggtaaagagg agttatttaa tctacctatg tgggactaga ctgttgaatc ttgtttgctg 30900
aaggcattaa ggcacaggac ttaatctctg aagtgaagtc tgaagagcct ttgccacatc 30960
tcaagtgcca gtacaatggc agcaatactg gtaccttcag cattggccac aggtaccatt 31020
ttctgagcac gtcctatgtg ccagaattat gtaagagtat tctcactcgt tatctgattt 31080
aatcttcaca atactgacag caaaatgagg agtctgaggc tctgggaagt tagttcacct 31140
gtccagtgtc acccagcctg taggcaccca agcttgggtc agaaagatag caaagccctg 31200
ttccttcctc tccagggctg ggaagtttga gccaccattc gtgaaagacc attcaggaac 31260
ccatggcatc attaaatgcc aacttcctca tatacacaaa ggtgtgtccc aaaaacaatt 31320
cagtcagacc tcgccaaaag ggccggattg tccataggac acagagatgc caggcacaag 31380
agctaccaga ttataggtcc tatactctgg ggactcagga agaaactgac tcaccaccac 31440
ttcagtgtgt ttcttcctac caggaaacaa atatgctttg gaaatgtcac catctgacca 31500
ccctccctgg ggactcaact aaggcttggg caaatagaac aagttgcagc ctgccccatg 31560
ggataatcgt gccagggaaa cagaacaatt gctgcctttc tgagatgcca gtaaaactag 31620
atttttaatg gcttgaaact agcctcttgt cagcaagtat ctattcacac agaccaaaga 31680
cccttcatac tgttcaccca ccacctcacc cccagcaagt gttcccaaga tatagagagc 31740
agagaatcca aagctgtcta gtggttggca ccaatatacc ccttttctag atgccattct 31800

tttttttattc cccatgtcag tcctgtggca cctgaatcaa ttctcttgca gcttaaacag 31860
agagcatttt tctacctcct taggacttac tctttcctcg cacgctccta gtttttccct 31920
aaatctagtg cagaaaagga ttcatagagg aatgtgagac aagggtgagc acagctcctg 31980
tgcatcacga tgtgtaactc caccttaaaa ccgtcattaa gaacttgcta gaaatttggg 32040
ccaatgggta acctggtgca ttcctaagtg ggtggacact gcccactcag tctctcaggt 32100
gctgcagctc acacagaaca cctcaggagc cctgtagcct caggcagcac atccatcagc 32160
agcacgtctc atcaatatat actgtggcat ctggtaaaac ccgggtgctt acacctctgt 32220
gtgagaaaga gcaattcaat aaatccaggg tggggcccag gcatctgcat gttctaaatg 32280
tacctaagtt gatactttct gtttttgtt tatcgtgtgt aaatgtatgt gtgtgtgcgt 32340
gtgtgtgtgt gtgtgtgtgt atgatggagt ctcactcagt cacccaggct ggagtgcagt 32400
ggcccagtct cagctcattg caacctccac ctcctgggtt gaagcaattc tcctgcctca 32460
gcctccccag taactgggat tacaggtgca tgccaccacg cctggctaat gtttgtattt 32520
ttagtagagg cggggtttca ccatgttggc caggctggtc tcgaactccc gacctcagat 32580
gatccacctg cctcggcctc ccaaagtgct gggattacag gcatgagcca ccacgcccgg 32640
cctaaatata cctaagttga ttctgataga gatgatcttc tgtttacact tgaaaaacac 32700
tgacatagtc aatgtccaga ttgtttactt gaagaaccag cagcacaata ataatagctt 32760
tacatggatt aagctttttc tatggtccgg tgctgtgcag attatctcac ttaatcctca 32820
aaaaaaaatc acacaaaata tgtgttattg tcgtcctcat ttttatagat gatgaagctg 32880
aagtttggag agatatgata acttgctcaa tatcacagca ctgatatacc aggaaaagag 32940
ttctctaaca caggtgatcc gatgttactg cctgaatttc caatggcctc agtatacttt 33000
ccatgcatta tacacggaag aataatcttc tctggaattt tatcttttgc cttaaatgtc 33060
acaggtatat ctacaaccag ttcctcatac cacaaatgca tttgataaca ttataaatgg 33120
aagtttacat gaaggaatat attttgttat tcagaaccat tatggaaatc aataatccac 33180
aaaatgtgaa tttagtgctt gccatatgca agactgatta catgttacat gccttcaaaa 33240
tgcaaaggca aaggttaaaa ggggatgtga ttttaaaaat ttaaaattat gaggatttca 33300
ggcaggatgg gcatggacag attttaaaat tcttagaaaa gtttagttag gagtcaactc 33360
ttgaagctcg agagagagga gacaaagctg aaggtagtaa acatttggaa actattatct 33420
caagaaattt ccagagcatg acatcttggc agctggccta aaccagcagg tgaacacttg 33480
ggagcaaggt atagacagat aaggataact gaaatgatgg agtcagggag gggaggctac 33540
ctatggataa aaaatgcttt taaaaatcac tctttactct agaaagatga agactgaaaa 33600
gggttaggat caaagctagt gaaattgtga ataattaggt gaaagtaaac atgaacttac 33660
cattctttcc aaatcctcgc aacctacgta actaacccca tagcaactca ctggtgaggc 33720
ttttctgaga agcccccctc atggctccct cctcctccta caagtgacag tatggagatg 33780
ccctgctaat agcattattt ggtcaatggg gtatctcaga gccctggggg ctagatttcg 33840
gggtggccag agggtggcca tccagggcca ggtggctctg tgctttaagg cagagtaatt 33900
gacctcattt tttatgcttc tataggcctc tgggaaagat gtagaggctg gaagatgaac 33960
aaacctgaga caacacagct ttgcaggtaa taaagcacca aactcagtga atacaggaac 34020
ctgtgtccat taggtcatac ttcagaaaag tttcttttgc ttaatatgga tagagaacat 34080
acagttgtta tttgagaaat atggaagcat gtgtgtgtgt atgtgtgcgt gtgtgatctt 34140
tttcattctt tttcagacaa acaaatgctg agagaattca ccactaccaa gccagcacta 34200
caagaactgc caaaaggagt tctaaatctt gaaacaaatc ctggaaacac accaaaacag 34260
aacatcttta aagcataaat ctcacaggac ctataaaaca aaaatacaat taaaaaaatc 34320
aaggtataca ggcaacaaaa tcacaataaa tggaatggta cttcacatct caatactaac 34380
attgaatgta aatggcttaa atgctccact taaaagatac agaatcacag aatggataag 34440
aattcaccaa ccaactatct gctgccttca agagactcac ctaacacata agaactcaca 34500
taaacttaag gaaaaggggc ggaaaaaaca ttccatgcaa atggacacca aaagtgagca 34560
gcagtagcta ttcttaaaac agacaaaaca aattttaaag caacagtagt taaaaaaaaa 34620
gaccaagagg gacattatat aacgaaaaca gatcttgtcc aacaggaaaa tatcacaatc 34680
ctaaatatat atacacctaa tactggagct cccaaatgta taaaacaatt actaatagac 34740
ctaagaaatg agatagacag caacacaata atagtggcac ttcaatactc cacttacagc 34800
actaggcagg tcattaagac agaaagtcaa caaggaaaaa atggatttaa actataccct 34860
gaaaaaatgg acttaacaga catttacaga acattctacc caacaactgc ataatataca 34920
ttctattcaa cagtgagtag aactttctcc aagatagacc atatgatagg ccacaaaaca 34980
agcctcaata aatttttttc tttttaatt tttttattat actttaagtt ctagggtaca 35040
tgtgcacaac gtgcaggttt gttacatgtg tatacatgtg ccatgttggt gtgctgcacc 35100
cattaacttg tcatttacat taggtatatc tcctaatgct atccttcccc cctcccccca 35160
ccccacaaca ggccctggtg tgtgatgttc cccttcctgt gtccaagtgt tctcattgtt 35220
caattcccac ctataagtga gaacatgcgg tgtttggttt tttgtccttg cgatagtttg 35280
ctgagaataa tggttttcag cttcatccat gtccctacaa aggacatgaa ctcatccttt 35340
tttatggctg catagtattc catggtgtat atgtgccaca ttttcttaat ccagtccatc 35400
attgttggac atttgggttg gttccaagtc cttgctattg tgagtagtgc cgcaataaat 35460
ttaagaaagt ttaaattata tcaaggattc tctcagacca cagtggaata aaactggaaa 35520
gcaacttcaa aaagaacctt caaaaccatg caaatgcatg gaaattaaac aacctgatcc 35580
tgaatgatta ttaggtcaaa aatgaaatcg agatggaaat ttaaaaattc tttgaactaa 35640
acaacaatag tgacaaaacc tatcaaaacc tctgggatac agcaaaggca gtgctaagag 35700
gaaagttcat aaccctaaac acctaaatca aaaagtctga gagagcacaa aaaatctaag 35760
gtcacacctc aaggaactag agaaacaaga acaaatcaaa cccaaaccca gcagaagaaa 35820
gaagataacc aagatcagag cagaactaaa cgaaattgaa acaaaaaaaa atacaaaaga 35880

taaatgaaac aaaaagctgg ttctttgaaa agacaaaatt aatagatcat tagcaagatt 35940
aaccaagaaa agaagagaga aaatccaaat aagttcaatt agaaacgaag caggaaatat 36000
tacaaccgac accacagaaa tacaaaacat cattcaaggc tactatgaac accttcatgc 36060
acataaacta gaaaacctag aggagatgga taaattcctg gaaatataca accttcctag 36120
cttaaatcag gaagaataag ataccttgag cagaccaata gcaagcagcg agactgaaac 36180
ggaaatttaa aaattaccaa caaaaaaatg tccaggacca gaaggattca cagcagaatt 36240
ctaccagaca ttcaaagaag aattgggagc aatcctattg acactattcc acaagataga 36300
gaaagaggga accttcccta aatcattctg tgaaaccagt attgccctaa taccaaaacc 36360
agcaaaggac ataaccataa aagaaaacta cagaccaata tccctgatga acacagatgc 36420
taaaatcttt aactaaatac tagctaaccg aatctaacta cgtattgaaa agataatcca 36480
ccatgatcaa gtgggtttca taccagggat gcagggatgg tttcaacata tgcaagtcaa 36540
aaaatgtgat acaccatata aacagaatca aaaacaaaat cacacgatta tctcaataga 36600
tgcagaaaaa gcatttgaca aaatccacca ttgttttatg actaaaactc tcagcaaaat 36660
cggcatacaa gggatatacc tcaatgtaat aaaagccatc tatgacaaac ccacagccaa 36720
cataatactg catggggaaa acttgaaagc attccctctg agaactggaa caagacaagg 36780
atgcccactc tcatcactcc ttttcaacat agtgctggaa gtcctggcca gagcaatcag 36840
acaagagaaa gaaataaatg gcatccaaat cagtgaagag gaagtcaaac tgttgccgtt 36900
tgttggtgat atgattgttt acttagaaaa ccctaaagac tcctccagaa agctcctaga 36960
actgataaaa gaattcagca aagtttctga atacaaaatt aatgtataca aatcagtagc 37020
tcttctatac accaacagcg accaagctga gaaacaaatc aagaactcaa taccttttac 37080
aatagctgca aaaataaaat aaaatactta ggaatatacc taaccaagga ggtgaaagac 37140
ctctgcaagg aaaactacaa aactctatgg aaataaatca tagatgacac aaacaaatgg 37200
aaacatctca tgctcatgga tgggtagaat caatattgtg aaaatgatca tactgcaaaa 37260
gcaatctaca aattcaatgc aattcccatg aaaataacat caccattctt cacagactta 37320
gaaaaaacaa ccccaaaatt cctatggaac caaaaaaaga gcccacatag ccaaagtaag 37380
actaagcaaa aagaacaaat ctggaatcat cacattaact gatttcaaac tatactatga 37440
ggccatagtc accaaaacag catggtactg gtataaaaat aggcacatag accaatgaaa 37500
cagaatagag aacccagaaa taaacccaaa tactgccaac tgatcttcaa cgaggcaaac 37560
aaaaacataa agtggagaaa ggacatccta ttcaacaaat tgttctggga taattggcaa 37620
gccacatgta ggtgactaaa actggttcct catctctcac cttttacaaa agtcaactct 37680
agattgatca aggacttaaa tccaagacct gaaatgataa aaattccaga agataacatt 37740
ggaaagcccc ttctagacat tggcttgaga atagacaatt atcaaaagaa gatatacaaa 37800
tggccaacaa acataagaaa atatgctcaa catcactaat gatcagggaa atgcaaatca 37860
aaaccacaat gtgataccac cttactcctg caagaatggt cataatcaaa aaatcaaaaa 37920
aaatagatgt tggcacggat gtggtgaaca aggaacactt acatgctgct gatgggaatg 37980
taaactagta taaccactat ggaaaacagt gtggagattc cttaaagaac taaaagtaga 38040
actatcattt tatccagcaa tcccacctct gggtatctac caagagaaaa agaactcatt 38100
atacaaaaaa gatacttgca tacaaaatgt ttacagcagc acaaatctca attgcaaaaa 38160
tgtggaacca acccaaatgc ccatcaatca acaagtggat aaagaaactg tggtatatat 38220
atatatatat atatatatat atatatatat atatatacca catcatatat atatatgatg 38280
aaataatact cagccataaa aagtaatgaa ctaatggcat tctcagcgac ctggatgaga 38340
ttggaggcta ctattctaag tgaagtaact caggaatgga aaaccaaaca gagtatgttc 38400
tcactcataa gtgggagcta agctatgagg atgcaaagtc ataagagtaa cacaatggac 38460
tttgaggact cggggaaagg atgggaagag ggtgaggtat aaaagactac aaattgggtg 38520
cagtgtatac tgctcgggtg ataggtacgc caaaatctca caaatcaccc ctgaagaact 38580
tatacatgta accaaacacc acctgttccc caataaccta tggaaacaaa aaaaaatttt 38640
taaaagaaaa taaaagttta ttttgcttaa tatggataga gaacatacaa ttgttatttg 38700
agaaatatga aacagagaga gagagtgtgt gtttgtgtgt gtgtgtgtgt gtgttccact 38760
agttaccacc tggataatca ggaagaaaga ggagacatcg gccccctaac ctgtcacaat 38820
tggacctgga atgctaaagc ggacctggaa tgctaaagct tacctgggac agtcctagtg 38880
aagccaccac tttccctcct ccattgtgta attcttggtt gattatctca gattttgaga 38940
gtatttcctc tgtgggttac tgaagttcta ggattatcct gatgaaagaa aaggggtgaa 39000
tcctggaaag ggatcaattt ggggatagac atcactttcc ctaaaggggg atggggtaaa 39060
gaatttcact attgctgtac aaagaaagga gttctctgaa aggaggactc atcagaggac 39120
atggaactga atgaggaatt ggagtgcatc ctcaaatggg ctccctgtga ttctggtata 39180
ccataaggct ttcagtgaaa gtatagagaa taaagtgaag aaactgcaaa gggcagcatg 39240
gcctgcccag ttcctccctc tctgctgaga atcatgtgga gctggtagca tcttattgca 39300
gggagttgca atcaagggat cataggaagt gattgtcagc cagtgagggg tgggtggctc 39360
atggagaagg tcagagtcat ggcaaagggc atccgttgag aagatgcagc tccaggacac 39420
aggtagagga caacaacgct tgggagtgaa agaggctaga gcttgggtca ctcaaagaag 39480
gagtaacttt aggacaaatt caagaaagtt tttatacagc aggtaataaa cttaacatct 39540
taccccctgca cactgaacat gtaaatagtt tcaagaaggg tttagataaa tttaagtgat 39600
aatattttag ggctggaaag ggatctggag atcacctact tcagtagttc tcaaccatgg 39660
ctgcacattg caatcacctg aggaggtttt aaaaaacatt gacgtccaag tcccagacta 39720
atgaatcaca acctttgggg tggaacttgg gcattaggat tttttttaac tttttatttt 39780
aaaattgtag gaagttgcaa aaatcataca tacagtctca tgtacccttc acctaacttc 39840
ccccagtggt gattatcatc ttaaatataa ataagacgat atcaaattca ggaagttgac 39900
attggtacaa tgctattaac tagactacag attattcgtc tttcaccatt ttttacatgc 39960

atgcatttat gtgttgtaag ggtatgtgtc tctatgcaat aaaaattagt atttttcaca 40020
aactctccag aagatcctaa ttcacagtaa aggttgagaa ctagggatct agtccagcca 40080
atcctcagtt cacaggtgag gaaactgagt ccagaatggc aaagtgacct gcccaagcac 40140
acaatcatct tttggaggga aggcagtgga ctttcttagt ccagtgttct ttgctatgca 40200
ttaagttacc tctgctcctt tatgggttac aggtcactaa tgtgctactt aagaagagct 40260
gatttcttgg gggcagggtc aggaggtaga agggcagtgt gccaattttt taggttgcct 40320
taattaaaag acaaacacaa aaacacaact tttggtagtt cttctaacgt tataaggtta 40380
ttgaatgaca tttctaaaat aaattcataa aaaacatggg ccctggtcaa tagtaagcac 40440
cctccttccc aactcccaca acaatattca ttagtattat tagagaattc tggcctagag 40500
ggatcacggg ttgggcatcc tgtcgaaatt attttattct ttggctttct cccactgtgg 40560
ctccttttat ttgctccacg ttgcaaaggg acttctagca gctgaggtca gcctcctgat 40620
cgtcactcca cagaatgagg agccacggtc aagcttgtta atatgacctc ctgccatgct 40680
ggtcctggga atattcctcc ctctccactg gcagcatgca gccaggcaag tgcgagaact 40740
gacttactct ctgtgggact taatgtcttc atctgatatg ctttcctggc ctgaacaaga 40800
cacagaaatg tttcttcctt ctacaacagc cctctgttga aagcagcaca tccagggaag 40860
tatgtcaaag gactcaatgg ctccagccca agctcttctc agcctaaata cttcacctgt 40920
ctctagaaat ccttctgtca gaggtgtttg aaccagagca actccatctt gaataggagc 40980
taggtaaaat aaggctgaga cctactgggc tgcattccca gccggtcagg cattctaagt 41040
cacaggatga gataggaggt cagcacaaga tacaggtcat aaagaccttg ctgataaaac 41100
agcttgcagt aaagaagcca gccaactaac atggtgaaac cccatgtcta ctaaaaacac 41160
acacacaaaa aaaaaatttt gccggacgtg gtggcaggca cttgtggtcc catctacttg 41220
ggaggctgag gcaggagaat gccgtgaacc cgtgagccga gatcccgcca ctgcactcca 41280
gcctgggcaa gagagcgaag actccatctc caagaaaaaa aaaaaggaaa gaaaccggcc 41340
aaaatccact aaaaccaaga gaatgacctc tggtcatcct cactgctaca ctcccaccag 41400
tgccatgaca gtttacaaat gccatgacaa cgtcaggaag ttaccctata tggtctaaaa 41460
aggggaggca tgaataatcc accccttgtt tagcatatca tcaagaaata accatataaa 41520
tgggcaacca atagccctcg ggctgctct gtctatggag tagccattct tttattcgaa 41580
tactttactc tcctaacaaa cttgctttca ttttactcta cagactcacc ctgaattctt 41640
tcttgcactt gctctaagaa ccctctcttg gggtctggat ccggaccgct ttctgctaac 41700
acttctacat accacgttta tttgagacag aaaaatacag caaggacatc catctaaact 41760
ttgtctggag tctgggccca ggggagctgc agtacatgga gggtctctgg aagtgggtat 41820
ggcagattag cgcctaagct caactggctt tcctcctctg cctctgttcc tgggacttgc 41880
tcacggctca caaggtagct tcccactgtc agtccatcag caacagccag ttagagtcga 41940
ttgggtgtga gaagcgagac tattcataag cagcctgtcc cctgtgccac tggcttatta 42000
agcagagcta ggcatagaca gctgggaact ctgggcagct cctttaaatt tctctttgaa 42060
gaatgttaag tattcaccag cagtgaaggt tacaaaggct gaagttttct ctccaccccc 42120
agcatttccc aggggagtaa aaggaaggat caaagctaca atgggcttgg ctgcccttct 42180
ccaggaatgg ccagaggaca acttctgctt ggtgagcagc catttatgcc accccggtgc 42240
tggcaggtca cctctcctgg gctggggagc aggagttcaa tgattaacta gaggaggcca 42300
tctctggagg acagaaagag tcttctgatc tggttagcca tgcagacctg agcctggatt 42360
ttccttccca ctccacttca agagaggggt gggttcattt ccacagttct ccttcctgca 42420
gcaggaaggg agagtagaaa cccaacttcc cagacaccaa ctccatttcc acacaaagca 42480
gtagagccca gacctaaggg ttctggagcc acacagacct ggtttgagtc ctggttccat 42540
cacttatctg ttctgggaca ttgggcaagt gccttaacct tcccgggcta caacttagtg 42600
gcggaaatca gtggagtaaa acctcattca gaaaattttt tcaaaactga tgtcatcctg 42660
ggaacaactt gcagaatgct atttttcttg cactgagcaa aaagaggtta gttacactag 42720
gtgtcatccc ggggaatcag gtctcatcac agggcccaca ccatctgaga ccttttcaga 42780
tcctctaaga tgctgagaca agaagccatc tggtcttgga gggaatggag atggtaagag 42840
cattgtcaat gacatggaag tctgagaagt gtcctattca tgcctttgga aaagacttct 42900
ctgcctgtct ggtttttatg ctctccccac actttaggag aaaataatgg ttttgctgac 42960
ttcatctggt cacctctgca aatgccccca accccctgtta ttttgtgtct gctgtgtacc 43020
agattctctg ctagactgcc tacatgcact atctcatttc atctccataa ccaaaccaat 43080
ggagtaggta ttattatccc attttataca tgagaaaact aagattcaga tagattgagt 43140
caactgccca aggcagtaag ttagagcacc ataattcaaa cctaactccg tgtaattcca 43200
cagcctattc catagcctgt gttttcttcc cctaggaaag ttcctggata cttctggaac 43260
agaattctcc tggggtgagt ggttcacaca actgacccac ttcatcccca ctcagcaaaa 43320
tgagagcgcc gttgactgac aacaaaatgc tgaactggaa acctccgcag taaaacgtga 43380
tgcagaacag agaaagaact aagatgttct gcacagacgg tatcatgccc tctatgagga 43440
aactactcga gcatatctgt gtttcaaatg atgtctttga actatttatg ttaaatgccc 43500
ccccaaaaaa atggaaaggc ttcttcgtag aggaaagagc ttgaagttgc taggctgttc 43560
tgccttcaac aggcatttat tgtgcagcta caaaatgctg caccaagcac tgggcagtgg 43620
aaaggtgatt cagacacagt tcctttgaca aaacctcaaa atcccatgtg aagttctctt 43680
ttataaagta aatcagaaaa gattcaaaag ttgaggtacc aaaaaaaaat aatataaatg 43740
caagtagtaa agcgctattg actgtaaata gaagcactta ataagtggtt gttgtttctg 43800
ttcttctctc taataagtgt atacaggtgg ctaaatgctt ttgttgagct atgagtacct 43860
cattctgtgg agtgactcag aaagtctcta ggtgttagac tggaaacagg tgatgttcag 43920
tgcacgcaat gcttgaagtg ttcccaggta tttagtttca ggtaaatttt actgtttaaa 43980
aaaattgttt ttacttgcta ttaagtgtgt tgaccttgga taatttactt aacctctcta 44040

tgccttgctt cctttttctgc aaaatgagag taagaaaagt gcttacctca ttggcttttt 44100
gtgaaattca atgagttaac acgtgggaga aaagatgttt ttaaataatc aattcttagc 44160
agttggtcat tgttatgagt tacatcatgc gtagttaaat attttttaaa aaattaaaat 44220
ttattttcct ataggaaaaa ggagggactg aattgtgaat ccattttctc tgctttcagt 44280
tatttctact cctgagaaaa accctaagca cttagggtct agaaccacat cccgagacgg 44340
tgagctcttc ccacattttc acatgtgcct gaatcatttg taggtctcgt tcaagcagag 44400
aatctccggg attctcactc gggaggtctg gggtcgggcc tgagtccttg catttctaac 44460
aaactcccag ttgatgctgg tgccactggt tctgaattaa gatgcttgtt tacctagtga 44520
cagcaacaaa aattgtagca atggtatcca gaaataaaca gcatactttt atagggctag 44580
tcaaatgaac ccaaacatac aaaataaagg caaacctcac aatcttcttg caatgattta 44640
ttactctgag agctcatttt aggtgagatt gtctccctcc ggagcctgat tcctaaaagc 44700
tttctatgac tttgaaatta tcgttttcct tttggaggta gcacaatata agagctaaga 44760
agagaatgag aactggagtc cctcccaggt ttgcccctct aggtatgtaa gctaaggtaa 44820
actacttagc ctttcttaag cctcagtttt ctcattacaa atgggcatat acggaacatt 44880
ggcttgcctc ctacagttgt ttgaggactc aatgaaaatg tacgtgaagc tggttgcggt 44940
gagctgagat tgtgccactg cactccagcc ttgtgacaca gcgagactcc gtctcaaaaa 45000
aataaataaa taaataaata aaaataaaag actggttgcg gttgctcatg cctgtaattc 45060
cagcactttg ggaggctgag gtgggcggat cacaaggtca agaaatcaag accatcttgg 45120
ctaacatggt gaaaccacat ctctactaaa actacaaaaa ttagctgggc atggtagcac 45180
atgcctgtag tcccagctac tcgggagggc tgaggcagga gaatcgcttg aaccagggag 45240
gcagagggtg cagtgagccg aaatcatgcc actgcactcc agccttgtga cagagtgaga 45300
ctccatctca aaaaaaaaaa aaaaaaaaag aaaaagaaaa aagaaaatgt acgtgaagca 45360
gttatcctgg tgccggtgcc tggtacgtat caagggcacc agaacattag ctatttaacc 45420
ttaaggtttc aaatccaccc ttctctggag acttcttgtt ccctactctc atgcatctct 45480
gcatcatggt cataacccat agcaacgaaa agccaaggtt tcactctttg aaatctggat 45540
aatggtattt gtctgcaata atatcctagg agtagagcta gagaatttgt tttgggtttt 45600
gtttgttttt gttcattttt cagacacggt gagtcagaaa cgtaaggctc agagatattt 45660
ttagtttttc cagccactaa gaattcttgt tgcctgcagg gaaataaaga aatgcagaga 45720
agggggagtg ctagacttgc ctgcttgcta tccaaaacca cttctttcct ttgaaaaagg 45780
gtctgtgcag ccaaaacaag cagcgctaac gctgggctga gagtaggcac cagccatcca 45840
ggaaagagac aaggcaacca tcagctacaa aagctgcttc aacagcagac agctcaaaga 45900
atcaaaaaat gcatcccagg agggctgggt gaggaaagac agtgatcttg ccttggtatt 45960
tgcagcaaag tcacagacca ggaaggtcaa atcctcgctg agaaccatgt ggacaaatgt 46020
ccaggacatg gtcgctggcc tcagggagcc cacaatttta ttgaagaagc aagattgtca 46080
gaagtaagga ccaaaactcg tgtatacaga gaaaggagaa agattgagag gaaagaagag 46140
gagaatcaag gaggcagaga ttaaaagaag gaagggatag aaaagaaaag aaaaagaaag 46200
agggaaggag agaaggggaa aagtacggac tccgtttcgt gactacatca caagcattga 46260
tgaaaagaag agaaatctgt gaaaacgaga aagcaggcac tgtatgttgc atcctgacac 46320
acagtgagtt gttacacttt aaaagccaca aagaagagac gctcacccag agacagactt 46380
gatggatttg gcagcttatc atactgggaa gggcagaatt gggttatgct atgttttcaa 46440
tgggtcccct ccaaaagtca gccaatgtga tagtattaag agatgaggcc tttaagaggt 46500
gattaggcca tgagagctcc ttcctcgggc ttggaattaa gtgtccttat acagaggttt 46560
ggcacaagga gttggccccc acttgccctt ccgtgttcag ccatgaggac acagtgttcc 46620
ttctctctgg aggatgcagc gtgagggcgc catcttggaa gcacagagca gccatcacca 46680
gacaccaaac ctgctggtgc cttgatcttc ccagcctcca gaactgtgag aaatttctgt 46740
tctttacaaa ttgcctagtc tcgggaattc tgttatagca gaacaaaagg actaagataa 46800
gttacctttt cttaaaggaa aactttgata aatattcttt gtcctctatt cctttcacct 46860
ataccccatg aagctgggag gataagggga gtaagaggag gagctgaaac taagaggtca 46920
gcagacccta agccctagtc ggaagcctcc aaaactgtca atcaaaccac tgtgtctcca 46980
gccctgggcc tgactcagga aaccatcacc tgcctcttct ccagcctctt ctctccatcc 47040
ttgcttgtga agtagtcatg gaatactctc catactcttt ttcccccctt tccttccctc 47100
tttttctttt gtttttctc ccttccttcc tttatcctcc tctgcctcct tgattttcct 47160
cttcatcttt cttctcaacc tttctccttt ctttttatac cctagtttta ttccttacct 47220
ctgacaatct tgcttctttg acaaaacatt aaactgagat ctcagactgg catgcaacct 47280
gattagtaga aacttgctct tcctggccta gtgcagttca tcatgcagcc tctcttcctc 47340
ttcccctggc acctcacagc agtcagcatc cacgtgctcc tgctaacaag tgcaaggcca 47400
aatgaagaaa acactaagaa tgcattcaca aaagtgacag catctcctct agccactttg 47460
aattttcccc tcatttactg aggaaaataa aattcctatt tgatctacag gtctgccacc 47520
tctaaggagt cgttccattt tctgaaaccc agaattaacc agaactacaa tccccactgt 47580
cacccaattt tgtgttgtgt tcagagatgt tggctggtca gccaggtttt tgagaataaa 47640
gctgacttca ggttgaaata gtcaaaatca aaaccttggg ccctttggac aattccctag 47700
ggactcttca taaagcccaa taaaggtctc catagaaaca ccttgcagcc catttatgag 47760
ctttttttagc aatagctgtc cagaaaactt ttacaccccc ctgaaatagt caatctaccc 47820
catctgctcc atgacaacag aaaaatggtg cctcatatca ctagccagca tctcgtaaca 47880
catcagggtt ttggcctgag attgttctct gagatacact atgcaaatat ccagctggta 47940
tcaccagaca acaggaattt agttgggaaa ggcacaagga ttgggaagtc tgaggacggg 48000
ctcaaggtct ccaactggac agccagctca ggactccttt gtcagggtcc ctggggacct 48060
ggctgaggtt ttcaacaggt agggcttgat cctagtgcaa caccttgttc ttaccctggg 48120

tctcttagat gagtgatctt ggccaaatca cttaagtgat ctcagattgt ttgctcatca 48180
tgataataat cgtaatattg ccaacctcac acggctgttg cgagtgccca gagaaagaaa 48240
aggtaaagga atgtggccct cctcacatga gaaaggagac taacagaaat taaggagctt 48300
gcccaagttc acacaggagg caagctgtgg tttaaatcta agtgtatctg acacatattg 48360
tgttctgttt ctactaagct ctagatataa gataaaaaat attatgctta tttgcatatt 48420
gttatgcata atgtccaaat gtatgcttca tttgcacata tgattgctac tgtctctcat 48480
ctgggatgta gttcccagct gggtaataca tttgagctaa actctcctca agcccagctt 48540
cagcccagca gagaattttt aggggcgaag tctcatcaca acacatagcc cttctgagca 48600
atcaggtgaa gcatagagtt ccaaaaagcc caaacttcac tcaaatttcc tctcgtagct 48660
caggatggga agctggggct cttccaaggg taagcaagcc aggtatcatg gcagcttcac 48720
accacatttt tgtattcatt cttgaccact ccccaccatc cccaaccctg ctgcctgtgc 48780
ccttttctaa agctcaagac tctcaatatt ccagaaacag ctaataccat taaaaatagt 48840
aattaatttt gtggaacacc taccaaggta agtcagcctc tttatgatat ttaatattca 48900
acatgaaact aaaagtaggg gtttttaac cattcccatt ttatggtcaa ggaaatggag 48960
gcctggagag atgaggtggc ccacccaggg tcatacaact aagaaaggta gaacaggatt 49020
caaatctagg tcctcttaca ccagacccag tgcctgtccc agggtgccgt cagtcccatt 49080
gctgctggag ggcagcatga gtagcacacc atcctgccag ccgccctcta gccagggcag 49140
actctcaaaa tgcaaaactt gccagacagg caggaatgca aatgcacaca cagcctgaag 49200
tagatctgcc ctgacagaag tcacccaggt ggccacttgg ccatggaatg tgcccagctt 49260
gataagaagg caaatgcctg gcgtcttgca gggccagggt gcatatcagg tttcatttct 49320
gggctgtggc aagacagaag ccgtatgtgg tctttgcctg ccaggactgg ctggccctga 49380
tgcccacggc ctgccaggca tgacaagccc agcagtcttc cctaacgggg ccttctcttg 49440
ccccaagcaa cagtcctaat gtcttccaag ttgggcacag ttccacagcc ccataaagct 49500
gcaagccaag gaatgcagct cccttttact tctgcctttc tgaactctac ccttctttca 49560
aggtcagctc aagcctgccc cccacattac aggttaggtt caaatgccaa ttctagctgt 49620
atggccttgg atgactcatt taacatctct tagcgccctt ttgtgaataa aatgggcgta 49680
aaaatgtcca ctccccaggg ctatggtaag gattgagagg cccagtgaaa cttcgcacat 49740
agtatatgca cagtaaatgg taggcagccc ccacttctca tagtgacctc tcttccctgc 49800
atttcagttt ctcttatcac ccgcctaatt caacttagca ttttattaaa cactgactca 49860
catctgttta gctcatcatt ttccattcat gtgtaagtct cgtctttctc tgatcttctt 49920
agaaccaaga accaggctgt tctttctctc atttcgccat ggaccactaa gcacagtttg 49980
gaatcgtcac atgacttatc agttgctgct ggtgggttgg agctcctaag tatattgata 50040
atacagattg aatatcctta atccaaaaat ccaaaatcca aagttctcca aaatctgaaa 50100
cgtttttaag caccaacatg ataatcaaag aaaatgctca ttatcacatt tcaaatttca 50160
gatttttgga tt 50172

## Description

### FIELD OF THE INVENTION

This invention relates to prediction of the susceptibility of an individual to colorectal cancer. Basis for the prediction lies in relating an individual's genetic makeup, as through molecular analysis, to the genetic makeup of a population of individuals.

### BACKGROUND

During the course of evolution, spontaneous mutations arise in the genomes of organisms. Variations in genomic DNA sequences are created continuously at a rate of about 100 new base changes per individual (Kondrashov, 1995; Crow, 1995). These germ-line changes may produce an evolutionary advantage and be retained in the population, or they may be deleterious and ultimately eliminated. In many cases, equilibrium between multiple germline forms of a sequence is established within a population if reproductive ability of individuals containing either polymorphism is not affected. Over time, significant numbers of mutations have accumulated within the human population that may be observed to varying extents in geographically separated groups based upon the presence of common ancestors.

Colorectal cancer is the third most common cancer and the third most common cause of death from cancer for both men and women. Colorectal cancer is responsible for more deaths that are not due primarily to tobacco use than any other type of cancer and inflicts a huge financial burden. Early detection of some human tumors such as uterine cervical cancer has dramatically reduced mortality from this condition (Herzog, 2003). Early detection of colorectal cancer can reasonably be expected to prevent death from this condition by identifying patients at risk for the disease, or those with the disease in an early stage and allow life saving intervention. A validated genetic test for colorectal cancer predisposition will have clinical utility, allowing prevention of cancer mortality through targeted screening programs. There are good reasons to expect that at least some of the genetic risks of common disease is due to common variants - for example, based on evolutionary arguments, and the fact that most human genetic variation is common. Although approximately 20% of colorectal cancers have a familial component with relatives exhibiting a doubling of risk (Carstensen *et al.,* 1996), less than 5% of colorectal cancer is explained by rare, highly penetrant genetic syndromes such as APC and HNPCC (de Leon *et al.,* 1999). Familial colorectal cancer occurring in patterns inconsistent with classical inherited syndromes suggests that variation in genome sequence plays a major role in determining individual risk to colorectal cancer. These genetic causes appear complex due to a variety of reasons such as genetic heterogeneity, incomplete penetrance, phenocopies and variation in exposures to environmental co-factors etc. There is little insight into the genetic or environmental determinants of almost 90% of cases of human colorectal carcinoma (Lynch and de La, 2003).

Although common human genetic variation is limited compared to other species, it remains impractical to discover and test every one of the estimated 10,000,000 common genotype variants (Sachidanandam *et al.,* 2001) as predictors of disease risk. Genotypic complexity is reduced through linkage disequilibrium that exists across long segments of the human genome with restriction in the diversity of haplotypes observed (Daly *et al.,* 2001; Rioux *et al.,* 2001; Liu *et al.,* 2004). That is, single nucleotide polymorphisms found at specific locations within the human genome are inherited in conjunction with nucleotides that can be polymorphic that are physically located near by. In European genomes, allelic association between pairs of markers typically extends over 10-50k, although there is tremendous variability in the magnitude of association observed at any given distance *(Clark et al.,* 1998; Kikuchi *et al.,* 2003; Dunning *et al.,* 2000; Abecasis *et al.,* 2001). Genome-wide data (Gabriel *et al.,* 2002; Reich *et al.,* 2001; Dawson *et al.,* 2002) supports the generality of this description as well as its application across populations. This confirms that measurement of single nucleotide polymorphisms at sites in tight linkage disequilibrium with adjacent genomic regions can provide information about the presence of diversity not just at sites actually measured, but also about large areas of the adjacent genome.

Numerous types of polymorphisms exist and are created when DNA sequences are either inserted or deleted from the genome. Another source of sequence variation results from the presence of repeated sequences in the genome variously termed short tandem repeats (STR), variable number of tandem repeats (VNTR), short sequence repeats (SSR) or microsatellites. These repeats commonly are comprised of 1 to 5 base pairs. Polymorphism occurs due to variation in the number of repeated sequences found at a particular locus.

The most common form of genomic variability are single nucleotide polymorphisms or SNPs. SNPs account for as much as 90% of human DNA polymorphism (Collins *et al.,* 1998). SNPs are single base pair positions in genomic DNA at which different sequence alternatives (genotypes) exist in a population. By common definition, the least frequent allele occurs at least 1% of the time. These nucleotide substitutions may be a transition, which is the substitution of one purine by another purine or the substitution of one pyrimidine by another, or they may be transversions in which a purine is replaced by a pyrimidine or vice versa.

Typically SNPs are observed in about 1 in 1000 base pairs (Wang *et al.,* 1998; Taillon-Miller *et al.,* 1999). The frequency of SNPs varies with the type and location of the change. Specifically, two-thirds of the substitutions involve the C ↔ T (G ↔ A) type, which may occur due to 5-methylcytosine deamination reactions that occur commonly. SNPs occur at a much higher frequency in non-coding regions than they do in coding regions.

### SUMMARY OF THE INVENTION

It has been discovered that polymorphic variations in a number of loci in human genomic DNA are associated with susceptibility to colorectal cancer. This invention thus includes methods for identifying a subject at risk of colorectal and/or determining risk of colorectal cancer in a subject, which comprise detecting the presence or absence of one or more polymorphic variations associated with colorectal cancer in a nucleic acid sample from the subject. In a specific embodiment, this invention relates to identifying an individual who is at altered risk for developing colorectal cancer based on the presence of specific genotypes defined by 230 single nucleotide polymorphism (SNPs), observed alone or in combination.

Through large scale genotyping studies on 2,198 blood samples from patients with colorectal cancer and 2,124 control samples from unaffected individuals we have identified 230 polymorphic markers found in 85 genes which are found more frequently in patients with colorectal cancer than in those without this disease. These markers, or those in close linkage disequilibrium, may change the composition, function or abundance of the elements of cellular constituents resulting in a predisposition to colorectal cancer. Measuring these markers in individuals who do not ostensibly have colorectal cancer will identify those at heightened risk for the subsequent development of colorectal cancer, providing benefit for, but not limited to, individuals, insurers, care givers and employers. Genes containing colorectal cancer-associated polymorphic markers that we have identified and genes found in linkage disequilibrium with these that we have identified are valuable targets for the development of therapeutics that inhibit or augment the activity of the gene products of these genes for therapeutic use in, but not restricted to, colorectal cancer. Information obtained from the detection of SNPs associated with colorectal cancer is of great value in the treatment and prevention of this condition.

Accordingly, one aspect of the present invention provides a method for diagnosing a genetic predisposition to colorectal cancer in a subject, comprising obtaining a sample containing at least one polynucleotide from the subject and analyzing the polynucleotide to detect the genetic polymorphism wherein the presence or absence of the polymorphism is associated with an altered susceptibility to developing colorectal cancer. In one embodiment, one or more of the 230 polymorphisms found distributed among 85 genes that we have identified may be used.

Another aspect of the present invention provides an isolated nucleic acid sequence comprising at least 16 contiguous nucleotides or their complements found in the genomic sequences of the 85 genes adjacent to and including the 230 polymorphic sites the inventors have identified to be associated with colorectal cancer.

Yet another aspect of the invention provides a method for treating colorectal cancer comprising obtaining a sample of biological material containing at least one polynucleotide from the subject, analyzing the polynucleotides to detect the presence of at least one polymorphism associated with colorectal cancer and treating the subject in such a way as to counteract the effect of any such polymorphism detected.

Still another aspect of the invention provides a method for the prophylactic treatment of a subject identified with a genetic predisposition to colorectal cancer identified through the measurement of all or some of the 230 polymorphic SNP markers described in Tables 1 to 230.

Further scope of the applicability of the present invention will become apparent from the detailed description provided below. It should be understood however, that the following detailed description and examples, while indicating preferred embodiments of the invention, are given by way of illustration only, since various changes and modification within the spirit and scope of the invention will become apparent to those skilled in the art from the following detailed description.

Tables 1 to 230 report the result of a genotyping analysis of 4,322 samples by measuring 385,562 single nucleotide polymorphisms in peripheral blood DNA from 2,128 subjects (1,059 cases with colorectal cancer and 1,069 age matched individuals undiseased at the time of testing), and validating the identified CRC-associated alleles by using peripheral blood DNA from a second and third, different, group of 2,194 subjects (687 and 452 cases, respectively, with colorectal cancer and 688 and 367 age matched individuals undiseased, respectively, at the time of testing).

### DETAILED DESCRIPTION OF THE INVENTION

It has been discovered that polymorphic variants in a number of sequences, SEQ ID NOs:1 to 18 are associated with an altered risk of developing colorectal cancer in subjects. The present invention thus provides SNPs associated with colorectal cancer, nucleic acid molecules containing SNPs, methods and reagents for the detection of the SNPs disclosed herein, uses of these SNPs for the development of detection reagents, and assays or kits that utilize such reagents. The colorectal cancer-associated SNPs disclosed herein are useful for diagnosing, screening for, and evaluating predisposition to colorectal cancer and related pathologies in humans. Furthermore, such SNPs and their encoded products are useful targets for the development of therapeutic agents.

A large number of colorectal cancer-associated SNPs have been identified by genotyping DNA from 4,322 individuals, 2,198 of these individuals having been previously diagnosed with colorectal cancer and 2,124 being "control" or individuals thought to be free of colorectal cancer.

The present invention thus provides individual SNPs associated with colorectal cancer, genomic sequences (SEQ ID NO:19) containing SNPs, and transcript sequences amino acid sequences. The invention includes methods of detecting these polymorphisms in a test sample, methods of determining the risk of an individual of having or developing colorectal cancer, methods of screening for compounds useful for treating disorders associated with a variant gene/protein such as colorectal cancer, compounds identified by these screening methods, methods of using the disclosed SNPs to select a treatment strategy, methods of treating a disorder associated with a variant gene/protein (i.e., therapeutic methods), and methods of using the SNPs of the present invention for human identification.

When the presence in the genome of an individual of a particular base, e.g., adenine, at a particular location in the genome correlates with an increased probability of that individual contracting colorectal cancer vis-à-vis a population not having that base at that location in the genome, that individual is said to be at "increased risk" of contracting colorectal cancer, i.e., to have an increased susceptibility. In certain cases, this effect can be a "dominant" effect in which case such increased probability exists when the base is present in one or the other or both alleles of the individual. In certain cases, the effect can be said to be "recessive", in which case such increased probability exists only when the base is present in both alleles of the individual.

When the presence in the genome of an individual of a particular base, e.g., adenine, at a particular location in the genome decreases the probability of that individual contracting colorectal cancer vis-à-vis a population not having that base at that location in the genome, that individual is said to be at "decreased risk" of contracting colorectal cancer, i.e., to have a decreased susceptibility. Such an allele is sometimes referred to in the art as being "protective". As with increased risk, it is also possible for a decreased risk to be characterized as dominant or recessive.

An "altered risk" means either an increased or a decreased risk.

The genetic analysis detailed below linked colorectal cancer with SNPs in the human genome. A SNP is a particular type of polymorphic site, a polymorphic site being a region in a nucleic acid sequence at which two or more alternative nucleotides are observed in a significant number of individuals from a population. A polymorphic site may be a nucleotide sequence of two or more nucleotides, an inserted nucleotide or nucleotide sequence, a deleted nucleotide or nucleotide sequence, or a microsatellite, for example. A polymorphic site that is two or more nucleotides in length may be 3, 4, 5, 6, 7, 8, 9, 10, 11, 12, 13, 14, 15 or more, 20 or more, 30 or more, 50 or more, 75 or more, 100 or more, 500 or more, or about 1000 nucleotides in length, where all or some of the nucleotide sequences differ within the region. Each of the specific polymorphic sites found in SEQ ID NO:19 is a "single nucleotide polymorphism" or a "SNP."

Where there are two, three, or four alternative nucleotide sequences at a polymorphic site, each nucleotide sequence is referred to as a "polymorphic variant" or "nucleic acid variant." Where two polymorphic variants exist, for example, the polymorphic variant represented in a majority of samples from a population is sometimes referred to as a "prevalent allele" and the polymorphic variant that is less prevalently represented is sometimes referred to as an "uncommon allele." An individual who possesses two prevalent alleles or two uncommon alleles is "homozygous" with respect to the polymorphism, and an individual who possesses one prevalent allele and one uncommon allele is "heterozygous" with respect to the polymorphism. Individuals who are homozygous with respect to one allele are sometimes predisposed to a different phenotype as compared to individuals who are heterozygous or homozygous with respect to another allele.

A genotype or polymorphic variant may also be expressed in terms of a "haplotype," which refers to the identity of two or more polymorphic variants occurring within genomic DNA on the same strand of DNA. For example, two SNPs may exist within a gene where each SNP position may include a cytosine variation or an adenine variation. Certain individuals in a population may carry an allele (heterozygous) or two alleles (homozygous) having the gene with a cytosine at each SNP position. As the two cytosines corresponding to each SNP in the gene travel together on one or both alleles in these individuals, the individuals can be characterized as having a cytosine/cytosine haplotype with respect to the two SNPs in the gene.

A "phenotype" is a trait which can be compared between individuals, such as presence or absence of a condition, for example, occurrence of colorectal cancer.

Polymorphic variants are often reported without any determination of whether the variant is represented in a significant fraction of a population. Some reported variants are sequencing errors and/or not biologically relevant. Thus, it is often not known whether a reported polymorphic variant is statistically significant or biologically relevant until the presence of the variant is detected in a population of individuals and the frequency of the variant is determined.

A polymorphic variant may be detected on either or both strands of a double-stranded nucleic acid. Also, a polymorphic variant may be located within an intron or exon of a gene or within a portion of a regulatory region such as a promoter, a 5' untranslated region (UTR), a 3' UTR, and in DNA (e.g., genomic DNA (gDNA) and complementary DNA (cDNA)), RNA (e.g., mRNA, tRNA, and rRNA), or a polypeptide. Polymorphic variations may or may not result in detectable differences in gene expression, polypeptide structure, or polypeptide function.

In our genetic analysis associating colorectal cancer with the polymorphic variants set forth in the tables, samples from individuals having been diagnosed with colorectal cancer and individuals not having cancer were allelotyped and genotyped. The allele frequency for each polymorphic variant among cases and controls was determined. These allele frequencies were compared in cases and controls, or combinations. Particular SNPs were thus found to be associated with colorectal cancer when genotype and haplotype frequency differences calculated between case and control pools were established to be statistically significant.

As mentioned above, polymorphic variants can travel together. Such variants are said to be in "linkage disequilibrium" so that heritable elements e.g., alleles that have a tendency to be inherited together instead of being inherited independently by random assortment are in linkage disequilibrium. Alleles are randomly assorted or inherited independently of each other if the frequency of the two alleles together is the product of the frequencies of the two alleles individually. For example, if two alleles at different polymorphic sites are present in 50% of the chromosomes in a population, then they would be said to assort randomly if the two alleles are present together on 25% of the chromosomes in the population. A higher percentage would mean that the two alleles are linked. For example, a first polymorphic site P1 having two alleles, e.g. A and C--each appearing in 50% of the individuals in a given population, is said to be in linkage disequilibrium with a second polymorphic site P2 having two alleles e.g. G and T--each appearing in 50% of the individuals in a given population, if particular combinations of alleles are observed in individuals at a frequency greater than 25% (if the polymorphic sites are not linked, then one would expect a 50% chance of an individual having A at P1 and a 50% chance of having G at P2 thus leading to a 25% chance of having the combination of A at P1 and G at P2 together). Heritable elements that are in linkage disequilibrium are said to be "linked" or "genetically linked" to each other.

One can see that in the case of a group of SNPs that are in linkage disequilibrium with each other, knowledge of the existence of all such SNPs in a particular individual generally provides redundant information. Thus, when identifying an individual who has an altered risk for developing colorectal cancer according to this invention, it is necessary to detect only one SNP of such a group of SNPs associated with an altered risk of developing colorectal cancer.

It has been shown that each SNP in the genomic sequences identified as SEQ ID NO:19 is associated with the occurrence of colorectal cancer. Thus, featured herein are methods for identifying a risk of colorectal cancer in a subject, which includes detecting the presence or absence of one or more of the SNPs described herein in a human nucleic acid sample.

Three different analyses were performed for each marker: (a) a test of trend across the 3 genotypes (Sasieni *et al.* 1997); (b) a dominant model where the homozygous genotype for allele "B" is combined with the prevalent heterozygote genotype; and (c) a recessive model where the homozygous genotype for allele "A" is combined with the heterozygous genotype. Using permutation analysis, the empirical p-value for the maximum of these three test statistics was calculated. Odds ratios measuring the strength of the association are also reported for the model corresponding to the largest of the three test statistics.

Pertinent results for each SNP are summarized in the tables: Chromosomal number and position- using the International Human Genome Sequencing Consortium build 35 (http://www.ncbi.nlm.nih.gov/genome/seq/) as made available by the National Center for Biotechnology Information (NCBI), National Library of Medicine, Building 38A, Bethesda, Maryland 20894 U.S.A., gene marker name-using the nomenclature of the NCBI dbSNP (http://www.ncbi.nlm.nih.gov/SNP/) and gene name-using the unigene naming convention. Under the "Case Flag" the number 1 designates Cases and the number 0 designates Controls. The identity of the base designated "A" in the analysis is indicated where 1 = A (adenine), 2 = C (cytosine), 3 = G (guanine) and 4 = T (thymidine). "B" indicates the polymorphic allele. AA, AB, BB are the counts of the number of individuals with the given genotype, by cases/controls. For dominant models, an odds ratio measuring the increase in risk associated with one or two copies of allele B is calculated. For recessive models, an odds ratio associated with exactly two copies of allele B is calculated. For the trend models, the Mantel-Haenszel odds ratio showing the increase in risk with each additional copy of allele B is calculated.

It has been discovered that each polymorphic variation in the genomic sequences identified as SEQ ID NO:19 is associated with the occurrence of colorectal cancer. Thus, featured herein are methods for identifying a risk of colorectal cancer in a subject, which comprises detecting the presence or absence of one or more of the polymorphic variations described herein in a human nucleic acid sample. The polymorphic variation, SNP, are detailed in the tables.

Methods for determining whether a subject is susceptible to, i.e., at risk of colorectal cancer are provided herein. These methods include detecting the presence or absence of one or more polymorphic variations, i.e., SNPs, associated with colorectal cancer in a sample from a subject.

SNPs can be associated with a disease state in humans or in animals. The association can be direct, as in conditions where the substitution of a base results in alteration of the protein coding sequence of a gene which contributes directly to the pathophysiology of the condition. Common examples of this include diseases such as sickle cell anemia and cystic fibrosis. The association can be indirect when the SNP plays no role in the disease, but is located close to the defective gene such that there is a strong association between the presence of the SNP and the disease state. Because of the high frequency of SNPs within the genome, there is a greater probability that a SNP will be linked to a genetic locus of interest than other types of genetic markers.

Disease-associated SNPs can occur in coding and non-coding regions of the genome. When located in the coding region altered function of the ensuing protein sequence may occur. If it occurs in the regulatory region of a gene it may affect expression of the protein. If the protein is involved in protecting the body against pathological conditions this can result in disease susceptibility.

Numerous methods exist for the measurement of specific SNP genotypes. Individuals carrying mutations in one or more SNPs of the present invention may be detected at the DNA level by a variety of techniques. Nucleic acids for diagnosis may be obtained from a patient's cells, such as from blood, urine, saliva, tissue biopsy and autopsy material.

The genomic DNA may be used directly for detection or may be amplified enzymatically by using PCR prior to analysis (Saiki *et al.,* 1986). RNA or cDNA may also be used in the same ways. As an example, PCR primers complementary to the nucleic acid of one or more SNPs of the present invention can be used to identify and analyze the presence or absence of the SNP. For example, deletions and insertions can be detected by a change in size of the amplified product in comparison to the normal genotype. Point mutations can be identified by hybridizing amplified DNA to radiolabeled SNP RNA of the present invention or alternatively, radiolabeled SNP antisense DNA sequences of the present invention. Perfectly matched sequences can be distinguished from mismatched duplexes by RNase A digestion or by differences in melting temperatures.

Sequence differences between a reference gene and genes having mutations also may be revealed by direct DNA sequencing. In addition, cloned DNA segments may be employed as probes to detect specific DNA segments. The sensitivity of such methods can be greatly enhanced by appropriate use of PCR or another amplification method. For example, a sequencing primer is used with double-stranded PCR product or a single-stranded template molecule generated by a modified PCR. The sequence determination is performed by conventional procedures with radiolabeled nucleotide or by automatic sequencing procedures with fluorescent-tags.

Genetic testing based on DNA sequence differences may be achieved by detection of alteration in electrophoretic mobility of DNA fragments in gels, with or without denaturing agents. Small sequence deletions and insertions can be visualized by high resolution gel electrophoresis. DNA fragments of different sequences may be distinguished on denaturing formamide gradient gels in which the mobilities of different DNA fragments are retarded in the gel at different positions according to their specific melting or partial melting temperatures (Myers *et al.,* 1985).

Sequence changes at specific locations also may be revealed by nuclease protection assays, such as RNase and S1 protection or the chemical cleavage method (Cotton *et al.,* 1988).

Thus, the detection of a specific DNA sequence may be achieved by methods which include, but are not limited to, hybridization, RNase protection, chemical cleavage, direct DNA sequencing or the use of restriction enzymes, (e.g., restriction fragment length polymorphisms ("RFLP") and Southern blotting of genomic DNA).

In addition to more conventional gel-electrophoresis and DNA sequencing, mutations also can be detected by *in situ* analysis.

Genetic mutations can be identified by hybridizing a sample and control nucleic acids, e.g., DNA or RNA, to high density arrays containing hundreds or thousands of oligonucleotide probes (Cronin *et al.,* 1996; Kozal *et al.,* 1996). For example, genetic mutations can be identified in two-dimensional arrays containing light-generated DNA probes as described in Cronin et al., supra. Briefly, a first hybridization array of probes can be used to scan through long stretches of DNA in a sample and control to identify base changes between the sequences by making linear arrays of sequential overlapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene. Specific mutations can also be determined through direct sequencing of one or both strands of DNA using dideoxy nucleotide chain termination chemistry, electrophoresis through a semi-solid matrix and fluorescent or radioactive chain length detection techniques.

Further mutation detection techniques may involve differential susceptibility of the polymorphic double strand to restriction endonuclease digestion, or altered electrophoretic gel mobility of single or double stranded gene fragments containing one polymorphic form. Other techniques to detect specific DNA polymorphisms or mutation may involve evaluation of the structural characteristics at the site of polymorphism using nuclear magnetic resonance or x-ray diffraction techniques.

These genetic tests are useful for prognosing and/or diagnosing colorectal cancer and often are useful for determining whether an individual is at an increased or decreased risk of developing or having colorectal cancer.

Thus, the invention includes a method for identifying a subject at risk of colorectal cancer, which includes detecting in a nucleic acid sample from the subject the presence or absence of a SNP associated with colorectal cancer at a polymorphic site in a nucleotide sequence identified as SEQ ID NOs:1 to 18.

Results from prognostic tests may be combined with other test results to diagnose colorectal cancer. For example, prognostic results may be gathered, a patient sample may be ordered based on a determined predisposition to colorectal cancer, the patient sample analyzed, and the results of the analysis may be utilized to diagnose colorectal cancer. Also colorectal cancer diagnostic methods can be developed from studies used to generate prognostic/diagnostic methods in which populations are stratified into subpopulations having different progressions of colorectal cancer. In another embodiment, prognostic results may be gathered; a patient's risk factors for developing colorectal cancer analyzed (e.g., age, family history); and a patient sample may be ordered based on a determined predisposition to colorectal cancer. In an alternative embodiment, the results from predisposition analyses may be combined with other test results indicative of colorectal cancer, which were previously, concurrently, or subsequently gathered with respect to the predisposition testing. In these embodiments, the combination of the prognostic test results with other test results can be probative of colorectal cancer, and the combination can be utilized as a colorectal cancer diagnostic.

Risk of colorectal cancer sometimes is expressed as a probability, such as an odds ratio, percentage, or risk factor. The risk is based upon the presence or absence of one or more of the SNP variants described herein, and also may be based in part upon phenotypic traits of the individual being tested. Methods for calculating risk based upon patient data are well known (Agresti, 2001). Allelotyping and genotyping analyses may be carried out in populations other than those exemplified herein to enhance the predictive power of the prognostic method. These further analyses are executed in view of the exemplified procedures described herein, and may be based upon the same polymorphic variations or additional polymorphic variations. Risk determinations for colorectal cancer are useful in a variety of applications. In one embodiment, colorectal cancer risk determinations are used by clinicians to direct appropriate detection, preventative and treatment procedures to subjects who most require these. In another embodiment, colorectal cancer risk determinations are used by health insurers for preparing actuarial tables and for calculating insurance premiums.

The nucleic acid sample typically is isolated from a biological sample obtained from a subject. For example, nucleic acid can be isolated from blood, saliva, sputum, urine, cell scrapings, and biopsy tissue. The nucleic acid sample can be isolated from a biological sample using standard techniques. The nucleic acid sample may be isolated from the subject and then directly utilized in a method for determining the presence of a polymorphic variant, or alternatively, the sample may be isolated and then stored (e.g., frozen) for a period of time before being subjected to analysis.

The presence or absence of a polymorphic variant is determined using one or both chromosomal complements represented in the nucleic acid sample. Determining the presence or absence of a polymorphic variant in both chromosomal complements represented in a nucleic acid sample is useful for determining the zygosity of an individual for the polymorphic variant (i.e., whether the individual is homozygous or heterozygous for the polymorphic variant). Any oligonucleotide-based diagnostic may be utilized to determine whether a sample includes the presence or absence of a polymorphic variant in a sample. For example, primer extension methods, ligase sequence determination methods (e.g., U.S. Pat. Nos. 5,679,524 and 5,952,174, and WO 01/27326), mismatch sequence determination methods (e.g., U.S. Pat. Nos. 5,851,770; 5,958,692; 6,110,684; and 6,183,958), microarray sequence determination methods, restriction fragment length polymorphism (RFLP), single strand conformation polymorphism detection (SSCP) (e.g., U.S. Pat. Nos. 5,891,625 and 6,013,499), PCR-based assays (e.g., TAQMAN™ PCR System (Applied Biosystems)), and nucleotide sequencing methods may be used.

Oligonucleotide extension methods typically involve providing a pair of oligonucleotide primers in a polymerase chain reaction (PCR) or in other nucleic acid amplification methods for the purpose of amplifying a region from the nucleic acid sample that comprises the polymorphic variation. One oligonucleotide primer is complementary to a region 3' of the polymorphism and the other is complementary to a region 5' of the polymorphism. A PCR primer pair may be used in methods disclosed in U.S. Pat. Nos. 4,683,195; 4,683,202, 4,965,188; 5,656,493; 5,998,143; 6,140,054; WO 01/27327; and WO 01/27329 for example. PCR primer pairs may also be used in any commercially available machines that perform PCR, such as any of the GENEAMP™, systems available from Applied Biosystems. Also, those of ordinary skill in the art will be able to design oligonucleotide primers based upon the nucleotide sequences set forth in SEQ ID NOs:1 to 18.

Also provided is an extension oligonucleotide that hybridizes to the amplified fragment adjacent to the polymorphic variation. An adjacent fragment refers to the 3' end of the extension oligonucleotide being often 1 nucleotide from the 5' end of the polymorphic site, and sometimes 2, 3, 4, 5, 6, 7, 8, 9, or 10 nucleotides from the 5' end of the polymorphic site, in the nucleic acid when the extension oligonucleotide is hybridized to the nucleic acid. The extension oligonucleotide then is extended by one or more nucleotides, and the number and/or type of nucleotides that are added to the extension oligonucleotide determine whether the polymorphic variant is present. Oligonucleotide extension methods are disclosed, for example, in U.S. Pat. Nos. 4,656,127; 4,851,331; 5,679,524; 5,834,189; 5,876,934; 5,908,755; 5,912,118; 5,976,802; 5,981,186; 6,004,744; 6,013,431; 6,017,702; 6,046,005; 6,087,095; 6,210,891; and WO 01/20039. Oligonucleotide extension methods using mass spectrometry are described, for example, in U.S. Pat. Nos. 5,547,835; 5,605,798; 5,691,141; 5,849,542; 5,869,242; 5,928,906; 6,043,031; and 6,194,144. Multiple extension oligonucleotides may be utilized in one reaction, which is referred to as multiplexing.

A microarray can be utilized for determining whether a SNP is present or absent in a nucleic acid sample. A microarray may include any oligonucleotides described herein, and methods for making and using oligonucleotide microarrays suitable for diagnostic use are disclosed in U.S. Pat. Nos. 5,492,806; 5,525,464; 5,589,330; 5,695,940; 5,849,483; 6,018,041; 6,045,996; 6,136,541; 6,142,681; 6,156,501; 6,197,506; 6,223,127; 6,225,625; 6,229,911; 6,239,273; WO 00/52625; WO 01/25485; and WO 01/29259. The microarray typically comprises a solid support and the oligonucleotides may be linked to this solid support by covalent bonds or by non-covalent interactions. The oligonucleotides may also be linked to the solid support directly or by a spacer molecule. A microarray may comprise one or more oligonucleotides complementary to a SNP set forth in the tables.

A kit also may be utilized for determining whether a polymorphic variant is present or absent in a nucleic acid sample. A kit can include one or more pairs of oligonucleotide primers useful for amplifying a fragment of a nucleotide sequence of interest, where the fragment includes a polymorphic site. The kit sometimes comprises a polymerizing agent, for example, a thermo-stable nucleic acid polymerase such as one disclosed in U.S. Pat. Nos. 4,889,818 or 6,077,664. Also, the kit often comprises an elongation oligonucleotide that hybridizes to the nucleotide sequence in a nucleic acid sample adjacent to the polymorphic site. Where the kit includes an elongation oligonucleotide, it can also include chain elongating nucleotides, such as dATP, dTTP, dGTP, dCTP, and dITP, including analogs of dATP, dTTP, dGTP, dCTP and dITP, provided that such analogs are substrates for a thermo-stable nucleic acid polymerase and can be incorporated into a nucleic acid chain elongated from the extension oligonucleotide. Along with chain elongating nucleotides would be one or more chain terminating nucleotides such as ddATP, ddTTP, ddGTP, ddCTP. The kit can include one or more oligonucleotide primer pairs, a polymerizing agent, chain elongating nucleotides, at least one elongation oligonucleotide, and one or more chain terminating nucleotides. Kits optionally include buffers, vials, microtiter plates, and instructions for use.

An individual identified as being susceptible to colorectal cancer may be heterozygous or homozygous with respect to the allele associated with an increased risk of colorectal cancer, as indicated in the tables. A subject homozygous for an allele associated with an increased risk of colorectal cancer is at a comparatively high risk of colorectal cancer as far as that SNP is concerned whether or not the allelic effect has been determined to be dominant or recessive. A subject who is heterozygous for an allele associated with an increased risk of colorectal cancer, in which the allelic effect is recessive would likely be at a comparatively reduced risk of colorectal cancer predicted by that SNP.

Individuals carrying mutations in one or more SNP of the present invention may be detected at the protein level by a variety of techniques. Cells suitable for diagnosis may be obtained from a patient's blood, urine, saliva, tissue biopsy and autopsy material.

Also featured are methods for determining risk of colorectal cancer and/or identifying a subject at risk of colorectal cancer by contacting a polypeptide or protein encoded by a nucleotide sequence from a subject with an antibody that specifically binds to an epitope associated with an altered, usually increased risk of colorectal cancer in the polypeptide.

### Isolated Nucleic Acids

Oligonucleotides can be linked to a second moiety, which can be another nucleic acid molecule to provide, for example, a tail sequence (e.g., a polyadenosine tail), an adapter sequence (e.g., phage M13 universal tail sequence), etc. Alternatively, the moiety might be one that facilitates linkage to a solid support or a detectable label, e.g., a radioactive label, a fluorescent label, a chemiluminescent label, a paramagnetic label, etc.

Nucleic acid sequences shown in the tables can be used for diagnostic purposes for detection and control of polypeptide expression. Also, oligonucleotide sequences such as antisense RNA, small-interfering RNA (siRNA) and DNA molecules and ribozymes that function to inhibit translation of a polypeptide are part of this invention.

Antisense RNA and DNA molecules, siRNA and ribozymes can be prepared by known methods. These include techniques for chemically synthesizing oligodeoxyribonucleotides such as solid phase phosphoramidite chemical synthesis. Alternatively, RNA molecules may be generated by *in vitro* and *in vivo* transcription of DNA sequences encoding the antisense RNA molecule. Such DNA sequences can be incorporated into vectors which incorporate suitable RNA polymerase promoters such as the T7 or SP6 polymerase promoters, or antisense cDNA constructs that synthesize antisense RNA constitutively or inducibly, depending on the promoter used, can be introduced stably into cell lines.

DNA encoding a polypeptide can also be used in the diagnosis of colorectal cancer, resulting from aberrant expression of a target gene. For example, the nucleic acid sequence can be used in hybridization assays of biopsies or autopsies to diagnose abnormalities of expression or function (e.g., Southern or Northern blot analysis, *in situ* hybridization assays).

Expression of a polypeptide during embryonic development can also be determined using nucleic acid encoding the polypeptide, particularly production of a functionally impaired polypeptide that is the cause of colorectal cancer. *In situ* hybridizations using a polypeptide as a probe can be employed to predict problems related to colorectal cancer. Administration of human active polypeptide, recombinantly produced can be used to treat disease states related to functionally impaired polypeptide. Alternatively, gene therapy approaches may be employed to remedy deficiencies of functional polypeptide or to replace or compete with a dysfunctional polypeptide.

Included as part of this invention are nucleic acid vectors, often expression vectors, which contain a nucleotide sequence set forth in the tables. A vector is a nucleic acid molecule capable of transporting another nucleic acid to which it has been linked and can include a plasmid, cosmid, or viral vector. The vector can be capable of autonomous replication or it can integrate into a host DNA. Viral vectors may include replication defective retroviruses, adenoviruses and adeno-associated viruses for example.

A vector can include a nucleotide sequence from the tables in a form suitable for expression of an encoded protein or nucleic acid in a host cell. The recombinant expression vector generally includes one or more regulatory sequences operatively linked to the nucleic acid sequence to be expressed. A regulatory sequence includes promoters, enhancers and other expression control elements (e.g., polyadenylation signals). Regulatory sequences include those that direct constitutive expression of a nucleotide sequence, as well as tissue-specific regulatory and/or inducible sequences. The design of the expression vector can depend on such factors as the choice of the host cell to be transformed, the level of expression of polypeptide desired, etc. Expression vectors can be introduced into host cells to produce the desired polypeptides, including fusion polypeptides.

Recombinant expression vectors can be designed for expression of polypeptides in prokaryotic or eukaryotic cells. For example, the polypeptides can be expressed in *E. coli,* insect cells (e.g., using baculovirus expression vectors), yeast cells, or mammalian cells. Suitable host cells are discussed further by Goeddel (Goeddel, 1990). A recombinant expression vector can also be transcribed and translated *in vitro,* for example using T7 promoter regulatory sequences and T7 polymerase.

Expression of polypeptides in prokaryotes can be carried out in *E. coli* with vectors containing constitutive or inducible promoters directing the expression of either fusion or non-fusion polypeptides. Fusion vectors add a number of amino acids to a polypeptide. Such fusion vectors typically serve to increase expression of recombinant polypeptide, to increase the solubility of the recombinant polypeptide and/or to aid in the purification of the recombinant polypeptide by acting as a ligand during purification. Often, a proteolytic cleavage site is introduced at the junction of the fusion moiety and the recombinant polypeptide to enable separation of the recombinant polypeptide from the fusion moiety after purification of the fusion polypeptide. Such enzymes, and their cognate recognition sequences, include Factor Xa, thrombin and enterokinase. Typical fusion expression vectors include pGEX (Pharmacia Biotech Inc; (Smith & Johnson, 1988)), pMAL (New England Biolabs, Beverly, Mass.) and pRIT5 (Pharmacia, Piscataway, N.J.) which fuse glutathione S-transferase (GST), maltose E binding polypeptide, or polypeptide A, respectively, to the target recombinant polypeptide.

Purified fusion polypeptides can be used in screening assays and to generate antibodies specific for polypeptides. In a therapeutic embodiment, fusion polypeptide expressed in a retroviral expression vector can be used to infect bone marrow cells that are subsequently transplanted into irradiated recipients. The pathology of the subject recipient is then examined after sufficient time has passed.

Expressing a polypeptide in host bacteria with an impaired capacity to proteolytically cleave the recombinant polypeptide can be used to maximize recombinant polypeptide expression (Gottesman, 1990). The nucleotide sequence of the nucleic acid to be inserted into an expression vector can be changed so that the individual codons for each amino acid are those preferentially utilized in *E. coli* (Wada *et al.,* 1992).

When used in mammalian cells, the expression vector's control functions are often provided by viral regulatory elements. For example, commonly used promoters are derived from polyoma, Adenovirus 2, cytomegalovirus and Simian Virus 40. Recombinant mammalian expression vectors can be capable of directing expression of the nucleic acid in a particular cell type (e.g., tissue-specific regulatory elements are used to express the nucleic acid). Examples of suitable tissue-specific promoters include an albumin promoter (Pinkert *et al.,* 1987), lymphoid-specific promoters (Calame and Eaton, 1988) , promoters of immunoglobulins (Banerji *et al.,* 1983; Queen and Baltimore, 1983), neuron-specific promoters (Byrne and Ruddle, 1989), pancreas-specific promoters *(*Edlund *et al.,* 1985), and mammary gland-specific promoters (e.g., milk whey promoter; U.S. Pat. No. 4,873,316 and European Application Publication No. 264,166). Developmentally-regulated promoters are sometimes utilized, for example, the murine hox promoters (Kessel and Gruss, 1990) and the .alpha.-fetopolypeptide promoter (Camper and Tilghman, 1989).

A nucleic acid from one of the tables might be cloned into an expression vector in an antisense orientation. Regulatory sequences (e.g., viral promoters and/or enhancers) operatively linked to a nucleic acid cloned in the antisense orientation can be chosen for directing constitutive, tissue specific or cell type specific expression of antisense RNA in a variety of cell types. Antisense expression vectors can be in the form of a recombinant plasmid, phagemid or attenuated virus.

The invention includes host cells having a nucleotide sequence from the tables within a recombinant expression vector or a fragment of such a sequence, which facilitate homologous recombination into a specific site of the host cell genome. Terms such as host cell and recombinant host cell refer not only to the particular subject cell but also to the progeny of a cell. Because certain modifications may occur in succeeding generations due to either mutation or environmental influences, such progeny may not, in fact, be identical to the parent cell. A host cell can be any prokaryotic or eukaryotic cell. For example, a polypeptide can be expressed in bacterial cells such as *E*. *coli,* insect cells, yeast or mammalian cells (such as Chinese hamster ovary cells (CHO) or COS cells).

Vectors can be introduced into host cells via conventional transformation or transfection techniques. The terms transformation and transfection refer to a variety of techniques known for introducing foreign nucleic acid (e.g., DNA) into a host cell, including calcium phosphate or calcium chloride co-precipitation, transduction/infection, DEAE-dextran-mediated transfection, lipofection, or electroporation.

A host cell can be used to produce a polypeptide. Accordingly, methods for producing a polypeptide using the host cells are included as part of this invention. Such a method can include culturing host cells into which a recombinant expression vector encoding a polypeptide has been introduced in a suitable medium such that the polypeptide is produced. The method can further include isolating the polypeptide from the medium or the host cell.

The invention also includes cells or purified preparations of cells which include a transgene from the tables, or which otherwise mis-express a polypeptide. Cell preparations can consist of human or non-human cells, e.g., rodent cells, e.g., mouse or rat cells, rabbit cells, or pig cells. The transgene can be mis-expressed, e.g., over-expressed or under-expressed. In other embodiments, the cell or cells include a gene which misexpresses an endogenous polypeptide (e.g., expression of a gene is disrupted, also known as a knockout). Such cells can serve as a model for studying disorders which are related to mutated or mis-expressed alleles or for use in drug screening. Also provided are human cells (e.g., hematopoietic stem cells) transformed with a nucleic acid from the tables.

The invention includes cells or a purified preparation thereof (e.g., human cells) in which an endogenous nucleic acid from the tables is under the control of a regulatory sequence that does not normally control the expression of the endogenous gene corresponding to the sequence. The expression characteristics of an endogenous gene within a cell (e.g., a cell line or microorganism) can be modified by inserting a heterologous DNA regulatory element into the genome of the cell such that the inserted regulatory element is operably linked to the corresponding endogenous gene. For example, an endogenous corresponding gene (e.g., a gene which is transcriptionally silent, not normally expressed, or expressed only at very low levels) may be activated by inserting a regulatory element which is capable of promoting the expression of a normally expressed gene product in that cell. Techniques such as targeted homologous recombinations, can be used to insert the heterologous DNA as described in, e.g., Chappel, U.S. Pat. No. 5,272,071; WO 91/06667, published on May 16, 1991.

Non-human transgenic animals that express a heterologous polypeptide (e.g., expressed from a nucleic acid from the tables) can be generated. Such animals are useful for studying the function and/or activity of a polypeptide and for identifying and/or evaluating modulators of the activity of the nucleic acids and encoded polypeptides. A transgenic animal is a non-human animal such as a mammal (e.g., a non-human primate such as chimpanzee, baboon, or macaque; an ungulate such as an equine, bovine, or caprine; or a rodent such as a rat, a mouse, or an Israeli sand rat), a bird (e.g., a chicken or a turkey), an amphibian (e.g., a frog, salamander, or newt), or an insect (e.g., *Drosophila melanogaster*), in which one or more of the cells of the animal includes a transgene. A transgene is exogenous DNA or a rearrangement (e.g., a deletion of endogenous chromosomal DNA) that is often integrated into or occurs in the genome of cells in a transgenic animal. A transgene can direct expression of an encoded gene product in one or more cell types or tissues of the transgenic animal. Thus, a transgenic animal can be one in which an endogenous nucleic acid homologous to a nucleic acid from the tables has been altered by homologous recombination between the endogenous gene and an exogenous DNA molecule introduced into a cell of the animal (e.g., an embryonic cell of the animal) prior to development of the animal.

Intronic sequences and polyadenylation signals can also be included in the transgene to increase expression efficiency of the transgene. One or more tissue-specific regulatory sequences can be operably linked to a nucleotide sequence from the tables to direct expression of an encoded polypeptide to particular cells. A transgenic founder animal can be identified based upon the presence of the nucleotide sequence in its genome and/or expression of encoded mRNA in tissues or cells of the animals. A transgenic founder animal can then be used to breed additional animals carrying the transgene. Moreover, transgenic animals carrying a nucleotide sequence can further be bred to other transgenic animals carrying other transgenes.

Polypeptides can be expressed in transgenic animals or plants by introducing a nucleic acid encoding the polypeptide into the genome of an animal. In certain embodiments the nucleic acid is placed under the control of a tissue specific promoter, e.g., a milk or egg specific promoter, and recovered from the milk or eggs produced by the animal. Also included is a population of cells from a transgenic animal.

Isolated polypeptides encoded by a nucleotide sequence from the tables can be synthesized. Isolated polypeptides include both the full-length polypeptide and the mature polypeptide (i.e., the polypeptide minus the signal sequence or propeptide domain). An isolated, or purified, polypeptide or protein is substantially free of cellular material or other contaminating proteins from the cell or tissue source from which the protein is derived, or is substantially free from chemical precursors or other chemicals when chemically synthesized. Substantially free means a preparation of a polypeptide having less than about 5% (by dry weight) of contaminating protein, or of chemical precursors or non-target chemicals. When the desired polypeptide is recombinantly produced, it is typically substantially free of culture medium, specifically, where culture medium represents less than about 10% of the polypeptide preparation.

Also, polypeptides may exist as chimeric or fusion polypeptides. As used herein, a "target chimeric polypeptide" or "target fusion polypeptide" includes a target polypeptide linked to a different polypeptide. The target polypeptide in the fusion polypeptide can correspond to an entire or nearly entire polypeptide as it exists in nature or a fragment thereof. The other polypeptide can be fused to the N-terminus or C-terminus of the target polypeptide.

Fusion polypeptides can include a moiety having high affinity for a ligand. For example, the fusion polypeptide can be a GST-target fusion polypeptide in which the target sequences are fused to the C-terminus of the GST sequences, or a polyhistidine-target fusion polypeptide in which the target polypeptide is fused at the N- or C-terminus to a string of histidine residues. Such fusion polypeptides can facilitate purification of recombinant target polypeptide. Expression vectors are commercially available that already encode a fusion moiety (e.g., a GST polypeptide), and a nucleotide sequence from the tables, or a substantially identical nucleotide sequence thereof, can be cloned into an expression vector such that the fusion moiety is linked in-frame to the target polypeptide. Further, the fusion polypeptide can be a target polypeptide containing a heterologous signal sequence at its N-terminus. In certain host cells (e.g., mammalian host cells), expression, secretion, cellular internalization, and cellular localization of a target polypeptide can be increased through use of a heterologous signal sequence. Fusion polypeptides can also include all or a part of a serum polypeptide (e.g., an IgG constant region or human serum albumin).

Target polypeptides can be incorporated into pharmaceutical compositions and administered to a subject *in vivo.* Administration of these polypeptides can be used to affect the bioavailability of a substrate of the polypeptide and may effectively increase polypeptide biological activity in a cell. Target fusion polypeptides may be useful therapeutically for the treatment of disorders caused by, for example, (i) aberrant modification or mutation of a gene encoding a polypeptide; (ii) mis-regulation of the gene encoding the polypeptide; and (iii) aberrant post-translational modification of a polypeptide. Also, target polypeptides can be used as immunogens to produce anti-target antibodies in a subject, to purify the polypeptide ligands or binding partners, and in screening assays to identify molecules which inhibit or enhance the interaction of a polypeptide with a substrate.

Polypeptides can be differentially modified during or after translation, e.g., by glycosylation, acetylation, phosphorylation, amidation, derivatization by known protecting/blocking groups, proteolytic cleavage, linkage to an antibody molecule or other cellular ligand, etc. Any known modification including specific chemical cleavage by cyanogen bromide, trypsin, chymotrypsin, papain, V8 protease, NaBH₄; acetylation, formylation, oxidation, reduction; metabolic synthesis in the presence of tunicamycin; etc. may be used. Additional post-translational modifications include, for example, N-linked or O-linked carbohydrate chains, processing of N-terminal or C-terminal ends), attachment of chemical moieties to the amino acid backbone, chemical modifications of N-linked or O-linked carbohydrate chains, and addition or deletion of an N-terminal methionine residue as a result of prokaryotic host cell expression. The polypeptide fragments may also be modified with a detectable label, such as an enzymatic, fluorescent, isotopic or affinity label to allow for detection and isolation of the polypeptide.

Chemically modified derivatives of polypeptides that can provide additional advantages such as increased solubility, stability and circulating time of the polypeptide, or decreased immunogenicity (see e.g., U.S. Pat. No. 4,179,337) are also part of this invention. The chemical moieties for derivitization may be selected from water soluble polymers such as polyethylene glycol, ethylene glycol/propylene glycol copolymers, carboxymethylcellulose, dextran, polyvinyl alcohol and the like. The polypeptides may be modified at random positions within the molecule, or at predetermined positions within the molecule and may include one, two, three or more attached chemical moieties.

The polymer may be of any molecular weight, and may be branched or unbranched. For polyethylene glycol, the molecular weight often is between about 1 kDa and about 100 kDa for ease in handling and manufacturing. Other sizes may be used, depending on the desired therapeutic profile (e.g., the duration of sustained release desired, the effects, if any on biological activity, the ease in handling, the degree or lack of antigenicity and other known effects of the polyethylene glycol to a therapeutic protein or analog).

The polymers can be attached to the polypeptide with consideration of effects on functional or antigenic domains of the polypeptide. There are a number of attachment methods available to those skilled in the art (e.g., EP 0 401 384 (coupling PEG to G-CSF) and Malik *et al.* (Malik *et al.,* 1992) For example, polyethylene glycol may be covalently bound through amino acid residues via a reactive group, such as a free amino or carboxyl group. Reactive groups are those to which an activated polyethylene glycol molecule may be bound. The amino acid residues having a free amino group may include lysine residues and the N-terminal amino acid residues; those having a free carboxyl group may include aspartic acid residues, glutamic acid residues and the C-terminal amino acid residue. Sulfhydryl groups may also be used as a reactive group for attaching the polyethylene glycol molecules. For therapeutic purposes, the attachment sometimes is at an amino group, such as attachment at the N-terminus or lysine group.

Proteins can be chemically modified at the N-terminus. Using polyethylene glycol, for example, one may select from a variety of polyethylene glycol molecules (by molecular weight, branching, and the like), the proportion of polyethylene glycol molecules to protein (polypeptide) molecules in the reaction mix, the type of pegylation reaction to be performed, and the method of obtaining the selected N-terminally pegylated protein. The method of obtaining the N-terminally pegylated preparation (i.e., separating this moiety from other monopegylated moieties if necessary) may be by purification of the N-terminally pegylated material from a population of pegylated protein molecules. Selective proteins chemically modified at the N-terminus may be accomplished by reductive alkylation, which exploits differential reactivity of different types of primary amino groups (lysine versus the N-terminal) available for derivatization in a particular protein. Under the appropriate reaction conditions, substantially selective derivatization of the protein at the N-terminus with a carbonyl group containing polymer is achievable.

### Applications of Prognostic and Diagnostic Results to Pharmacogenomic Methods

Pharmacogenomics is a discipline that involves tailoring a treatment for a subject according to the subject's genotype. For example, based upon the outcome of a prognostic test, a clinician or physician may target pertinent information and preventative or therapeutic treatments to a subject who would be benefited by the information or treatment and avoid directing such information and treatments to a subject who would not be benefited (e.g., the treatment has no therapeutic effect and/or the subject experiences adverse side effects). As therapeutic approaches for colorectal cancer continue to evolve and improve, the goal of treatments for colorectal cancer related disorders is to intervene even before clinical signs manifest themselves. Thus, genetic markers associated with susceptibility to colorectal cancer prove useful for early diagnosis, prevention and treatment of colorectal cancer.

The following is an example of a pharmacogenomic embodiment. A particular treatment regimen can exert a differential effect depending upon the subject's genotype. Where a candidate therapeutic exhibits a significant beneficial interaction with a prevalent allele and a comparatively weak interaction with an uncommon allele (e.g., an order of magnitude or greater difference in the interaction), such a therapeutic typically would not be administered to a subject genotyped as being homozygous for the uncommon allele, and sometimes not administered to a subject genotyped as being heterozygous for the uncommon allele. In another example, where a candidate therapeutic is not significantly toxic when administered to subjects who are homozygous for a prevalent allele but is comparatively toxic when administered to subjects heterozygous or homozygous for an uncommon allele, the candidate therapeutic is not typically administered to subjects who are genotyped as being heterozygous or homozygous with respect to the uncommon allele.

Methods of the invention are applicable to pharmacogenomic methods for detecting, preventing, alleviating and/or treating colorectal cancer. For example, a nucleic acid sample from an individual may be subjected to a genetic test. Where one or more SNPs associated with increased risk of colorectal cancer are identified in a subject, information for detecting, preventing or treating colorectal cancer and/or one or more colorectal cancer detection, prevention and/or treatment regimens then may be directed to and/or prescribed to that subject.

In certain embodiments, a detection, preventative and/or treatment regimen is specifically prescribed and/or administered to individuals who will most benefit from it based upon their risk of developing colorectal cancer assessed by the methods described herein. Methods are thus provided for identifying a subject at risk of colorectal cancer and then prescribing a detection, therapeutic or preventative regimen to individuals identified as being at increased risk of colorectal cancer. Thus, certain embodiments are directed to methods for treating colorectal cancer in a subject, reducing risk of colorectal cancer in a subject, or early detection of colorectal cancer in a subject, which comprise: detecting the presence or absence of a SNP associated with colorectal cancer in a nucleotide sequence set forth in SEQ ID NOs:1 to 19, and prescribing or administering a colorectal cancer treatment regimen, preventative regimen and/or detection regimen to a subject from whom the sample originated where the presence of one or more SNPs associated with colorectal cancer are detected in the nucleotide sequence. In these methods, genetic results may be utilized in combination with other test results to diagnose colorectal cancer as described above.

The use of certain colorectal cancer treatments are known in the art, and include surgery, chemotherapy and/or radiation therapy. Any of the treatments may be used in combination to treat or prevent colorectal cancer (e.g., surgery followed by radiation therapy or chemotherapy).

Pharmacogenomic methods also may be used to analyze and predict a response to a colorectal cancer treatment or a drug. For example, if pharmacogenomic analysis indicates a likelihood that an individual will respond positively to a colorectal cancer treatment with a particular drug, the drug may be administered to the individual. Conversely, if the analysis indicates that an individual is likely to respond negatively to treatment with a particular drug, an alternative course of treatment may be prescribed. A negative response may be defined as either the absence of an efficacious response or the presence of toxic side effects. The response to a therapeutic treatment can be predicted in a background study in which subjects in any of the following populations are genotyped: a population that responds favorably to a treatment regimen, a population that does not respond significantly to a treatment regimen, and a population that responds adversely to a treatment regiment (e.g., exhibits one or more side effects). These populations are provided as examples and other populations and subpopulations may be analyzed. Based upon the results of these analyses, a subject is genotyped to predict whether he or she will respond favorably to a treatment regimen, not respond significantly to a treatment regimen, or respond adversely to a treatment regimen.

The methods described herein also are applicable to clinical drug trials. One or more SNPs indicative of response to an agent for treating colorectal cancer or to side effects to an agent for treating colorectal cancer may be identified. Thereafter, potential participants in clinical trials of such an agent may be screened to identify those individuals most likely to respond favorably to the drug and exclude those likely to experience side effects. In that way, the effectiveness of drug treatment may be measured in individuals who respond positively to the drug, without lowering the measurement as a result of the inclusion of individuals who are unlikely to respond positively in the study and without risking undesirable safety problems.

Thus, another embodiment is a method of selecting an individual for inclusion in a clinical trial of a treatment or drug comprising the steps of: (a) obtaining a nucleic acid sample from an individual; (b) determining the identity of a polymorphic variant, e.g., SNP which is associated with a positive response to the treatment or the drug, or at least one SNP which is associated with a negative response to the treatment or the drug in the nucleic acid sample, and (c) including the individual in the clinical trial if the nucleic acid sample contains the SNP associated with a positive response to the treatment or the drug or if the nucleic acid sample lacks said SNP associated with a negative response to the treatment or the drug. The SNP may be in a sequence selected individually or in any combination from those disclosed in the tables. Step (c) can also include administering the drug or the treatment to the individual if the nucleic acid sample contains the SNP associated with a positive response to the treatment or the drug and the nucleic acid sample lacks the SNP associated with a negative response to the treatment or the drug.

### Compositions Comprising Colorectal Cancer-Directed Molecules

The invention includes a composition made up of a colorectal cancer cell and one or more molecules specifically directed and targeted to a nucleic acid comprising a nucleotide sequence shown in the tables, or a polypeptide encoded thereby. Such directed molecules include, but are not limited to, a compound that binds to a nucleic acid or a polypeptide; a RNAi or siRNA molecule having a strand complementary to a nucleotide sequence; an antisense nucleic acid complementary to an RNA encoded by a DNA sequence; a ribozyme that hybridizes to a nucleotide sequence; a nucleic acid aptamer that specifically binds a polypeptide; and an antibody that specifically binds to a polypeptide or binds to a nucleic acid. In specific embodiments, the colorectal cancer directed molecule interacts with a nucleic acid or polypeptide variant associated with colorectal cancer.

### Compounds

Compounds can be obtained using any of numerous approaches in combinatorial library methods known in the art, including: biological libraries; peptoid libraries (libraries of molecules having the functionalities of peptides, but with a novel, non-peptide backbone which are resistant to enzymatic degradation but which nevertheless remain bioactive (Zuckermann *et al.,* 1994). Biological library and peptoid library approaches are typically limited to peptide libraries, while the other approaches are applicable to peptide, non-peptide oligomer or small molecule libraries of compounds (Lam, 1997). Examples of methods for synthesizing molecular libraries are described, for example, in DeWitt *et al.* (DeWitt *et al.,* 1993), Erb *et al.* (Erb *et al.,* 1994), Zuckermann *et al.* (Zuckermann *et al.,* 1994), Cho *et al.* (Cho *et al.,* 1993) and Gallop *et al.* (Gallop *et al.,* 1994).

Libraries of compounds may be presented in solution (Houghten *et al.,* 1992), or on beads (Lam *et al.,* 1991), chips (Fodor *et al.,* 1993), bacteria or spores (Ladner, U.S. Pat. No. 5,223,409), plasmids (Cull *et al.,* 1992) or on phage (Scott and Smith, 1990; Devlin *et al.,* 1990; Cwirla *et al.,* 1990; Felici *et al.,* 1991).

A compound sometimes alters expression and sometimes alters activity of a target polypeptide and may be a small molecule. Small molecules include peptides, peptidomimetics (e.g., peptoids), amino acids, amino acid analogs, polynucleotides, polynucleotide analogs, nucleotides, nucleotide analogs, organic or inorganic compounds (i.e., including heteroorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, and salts, esters, and other pharmaceutically acceptable forms of such compounds.

An antisense nucleic acid refers to a nucleotide sequence complementary to a sense nucleic acid encoding a polypeptide, e.g., complementary to the, coding strand of a double-stranded cDNA molecule or complementary to an mRNA sequence. The antisense nucleic acid can be complementary to an entire coding strand in a nucleic acid molecule having a sequence of SEQ ID NO:19, or to a portion thereof. In another embodiment, the antisense nucleic acid molecule is antisense to a non-coding region of the coding strand of a nucleotide sequence, e.g., 5' and 3' untranslated regions.

An antisense nucleic acid can be designed such that it is complementary to the entire coding region of an mRNA encoded by a nucleotide sequence of interest, and often the antisense nucleic acid is an oligonucleotide antisense to only a portion of a coding or non-coding region of the mRNA. For example, the antisense oligonucleotide can be complementary to the region surrounding the translation start site of the mRNA, e.g., between the -10 and +10 regions of the target gene nucleotide (SNP) sequence of interest. An antisense oligonucleotide can be, for example, about 7, 10, 15, 20, 25, 30, 35, 40, 45, 50, 55, 60, 65, 70, 75, 80, or more nucleotides in length. The antisense nucleic acids, which include the ribozymes described below, can be designed to target a nucleotide sequence in SEQ ID NO:19. Uncommon alleles and prevalent alleles can be targeted, and those associated with an increased risk of colorectal cancer are often designed, tested, and administered to subjects.

An antisense nucleic acid can be constructed using chemical synthesis and enzymatic ligation reactions using standard procedures. For example, an antisense nucleic acid molecule can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. Antisense nucleic acid also can be produced biologically using an expression vector into which a nucleic acid has been subcloned in an antisense orientation (i.e., RNA transcribed from the inserted nucleic acid will be of an antisense orientation to a target nucleic acid of interest.

When utilized as therapeutics, antisense nucleic acids typically are administered to a subject (e.g., by direct injection at a tissue site) or generated *in situ* such that they hybridize with or bind to cellular mRNA and/or genomic DNA encoding a polypeptide and thereby inhibit expression of the polypeptide, for example, by inhibiting transcription and/or translation. Alternatively, antisense nucleic acid molecules can be modified to target selected cells and then are administered systemically. For systemic administration, antisense molecules can be modified such that they specifically bind to receptors or antigens expressed on a selected cell surface, for example, by linking antisense nucleic acid molecules to peptides or antibodies which bind to cell surface receptors or antigens. Antisense nucleic acid molecules can also be delivered to cells using vectors. Sufficient intracellular concentrations of antisense molecules are achieved by incorporating a strong promoter, such as a pol II or pol III promoter, in the vector construct.

Antisense nucleic acid molecules sometimes are anomeric nucleic acid molecules (Gautier *et al*., 1987). Antisense nucleic acid molecules can also comprise a 2'-o-methylribonucleotide (Inoue *et al.,* 1987a) or a chimeric RNA-DNA analogue (Inoue *et al.,* 1987b). Antisense nucleic acids sometimes are composed of DNA or peptide nucleic acid (PNA).

In another embodiment, an antisense nucleic acid is a ribozyme. A ribozyme having specificity for a target nucleotide sequence can include one or more sequences complementary to such a nucleotide sequence, and a sequence having a known catalytic region responsible for mRNA cleavage (see e.g., U.S. Pat. No. 5,093,246 or Haselhoff and Gerlach (Haseloff and Gerlach, 1988). For example, a derivative of a Tetrahymena L-19 IVS RNA is sometimes utilized in which the nucleotide sequence of the active site is complementary to the nucleotide sequence to be cleaved in a mRNA (see e.g., Cech et al., U.S. Pat. No. 4,987,071; and Cech et al., U.S. Pat. No. 5,116,742). Also, target mRNA sequences can be used to select a catalytic RNA having a specific ribonuclease activity from a pool of RNA molecules (Bartel and Szostak, 1993).

Colorectal cancer directed molecules include in certain embodiments nucleic acids that can form triple helix structures with a target nucleotide sequence, especially one that includes a regulatory region that controls expression of a polypeptide. Gene expression can be inhibited by targeting nucleotide sequences complementary to the regulatory region of a target nucleotide sequence (e.g., promoter and/or enhancers) to form triple helical structures that prevent transcription of a gene in target cells (Helene, 1991; Helene *et al.,* 1992; Maher, III, 1992). Potential sequences that can be targeted for triple helix formation can be increased by creating a switchback nucleic acid molecule. Switchback molecules are synthesized in an alternating 5'-3',3'-5' manner, such that they base pair with first one strand of a duplex and then the other, eliminating the necessity for a sizeable stretch of either purines or pyrimidines to be present on one strand of a duplex.

Colorectal cancer directed molecules include RNAi and siRNA nucleic acids. Gene expression may be inhibited by the introduction of double-stranded RNA (dsRNA), which induces potent and specific gene silencing, a phenomenon called RNA interference or RNAi. See, e.g., Fire et al., U.S. Pat. No. 6,506,559; Tuschl et al., PCT International Publication No. WO 01/75164; Kay *et al.,* PCT International Publication No. WO 03/010180A1; or Bosher J M, Labouesse (Bosher and Labouesse, 2000). This process has been improved by decreasing the size of the double-stranded RNA to 20-24 base pairs (to create small-interfering RNAs or siRNAs) that switched off genes in mammalian cells without initiating an acute phase response, i.e., a host defense mechanism that often results in cell death (Caplen *et al.,* 2001 a; Elbashir *et al.,* 2002). There is increasing evidence of post-transcriptional gene silencing by RNA interference (RNAi) for inhibiting targeted expression in mammalian cells at the mRNA level, in human cells. There is additional evidence of effective methods for inhibiting the proliferation and migration of tumor cells in human patients, and for inhibiting metastatic cancer development (see, e.g., U.S. patent application No. US2001000993183; Caplen *et al.* (Caplen *et al.,* 2001b), Abderrahman *et al.* (Abderrahmani *et al.,* 2001).

An siRNA or RNAi is a nucleic acid that forms a double stranded RNA and has the ability to reduce or inhibit expression of a gene or target gene when the siRNA is delivered to or expressed in the same cell as the gene or target gene. siRNA is short double-stranded RNA formed by the complementary strands. Complementary portions of the siRNA that hybridize to form the double stranded molecule often have substantial or complete identity to the target molecule sequence. In one embodiment, an siRNA is a nucleic acid that has substantial or complete identity to a target gene and forms a double stranded siRNA.

When designing the siRNA molecules, the targeted region often is selected from a given DNA sequence beginning 50 to 100 nucleotides downstream of the start codon. See, e.g., Elbashir *et al.* (Elbashir *et al.,* 2002). Initially, 5' or 3'UTRs and regions nearby the start codon were avoided assuming that UTR-binding proteins and/or translation initiation complexes may interfere with binding of the siRNP or RISC endonuclease complex. Sometimes regions of the target 23 nucleotides in length conforming to the sequence motif AA (N19)TT (N, an nucleotide), and regions with approximately 30% to 70% G/C-content (often about 50% G/C-content) often are selected. If no suitable sequences are found, the search often is extended using the motif NA (N2 1). The sequence of the sense siRNA sometimes corresponds to (N19) TT or N21 (position 3 to 23 of the 23-nt motif), respectively. In the latter case, the 3' end of the sense siRNA often is converted to TT. The rationale for this sequence conversion is to generate a symmetric duplex with respect to the sequence composition of the sense and antisense 3' overhangs. The antisense siRNA is synthesized as the complement to position 1 to 21 of the 23-nt motif. Because position 1 of the 23-nt motif is not recognized sequence-specifically by the antisense siRNA, the 3'-most nucleotide residue of the antisense siRNA can be chosen deliberately. However, the penultimate nucleotide of the antisense siRNA (complementary to position 2 of the 23-nt motif) often is complementary to the targeted sequence. For simplifying chemical synthesis, TT often is utilized. siRNAs corresponding to the target motif NAR (N17)YNN, where R is purine (A,G) and Y is pyrimidine (C,U), often are selected. Respective 21 nucleotide sense and antisense siRNAs often begin with a purine nucleotide and can also be expressed from pol III expression vectors without a change in targeting site. Expression of RNAs from pol III promoters can be more efficient when the first transcribed nucleotide is a purine.

The sequence of the siRNA can correspond to the full length target gene, or a subsequence thereof. Often, the siRNA is about 15 to about 50 nucleotides in length (e.g., each complementary sequence of the double stranded siRNA is 15 to 50 nucleotides in length, and the double stranded siRNA is about 15 to 50 base pairs in length, sometimes about 20 to 30 nucleotides in length or about 20 to 25 nucleotides in length, e.g., 20, 21, 22, 23, 24, 25, 26, 27, 28, 29, or 30 nucleotides in length. The siRNA sometimes is about 21 nucleotides in length. Methods of using siRNA are known in the art, and specific siRNA molecules may be purchased from a number of companies including Dharmacon Research, Inc.

Antisense, ribozyme, RNAi and siRNA nucleic acids can be altered to form modified nucleic acid molecules. The nucleic acids can be altered at base moieties, sugar moieties or phosphate backbone moieties to improve stability, hybridization, or solubility of the molecule. For example, the deoxyribose phosphate backbone of nucleic acid molecules can be modified to generate peptide nucleic acids (see Hyrup et al., Bioorganic & Medicinal Chemistry 4 (1): 5-23 (1996)). A peptide nucleic acid, or PNA, refers to a nucleic acid mimic such as a DNA mimic, in which the deoxyribose phosphate backbone is replaced by a pseudopeptide backbone and only the four natural nucleobases are retained. The neutral backbone of a PNA can allow for specific hybridization to DNA and RNA under conditions of low ionic strength. Synthesis of PNA oligomers can be performed using standard solid phase peptide synthesis protocols as described, for example, in Hyrup *et al.* (Hyrup and Nielsen, 1996), and Perry-O'Keefe *et al.* (Abderrahmani *et al.,* 2001).

PNA nucleic acids can be used in prognostic, diagnostic, and therapeutic applications. For example, PNAs can be used as anti-sense or anti-gene agents for sequence-specific modulation of gene expression by, for example, inducing transcription or translation arrest or inhibiting replication. PNA nucleic acid molecules can also be used in the analysis of SNPs in a gene, (e.g., by PNA-directed PCR clamping); as artificial restriction enzymes when used in combination with other enzymes, (e.g., S1 nucleases (Hyrup and Nielsen, 1996) or as probes or primers for DNA sequencing or hybridization (Hyrup and Nielsen, 1996; Perry-O'Keefe *et al.,* 1996).

In other embodiments, oligonucleotides may include other appended groups such as peptides (e.g., for targeting host cell receptors *in vivo),* or agents facilitating transport across cell membranes (see e.g., Letsinger *et al.* (Letsinger *et al.,* 1989); Lemaitre *et al.* (Lemaitre *et al.,* 1987) and PCT Publication No. W088/09810) or the blood-brain barrier (see, e.g., PCT Publication No. W089/10134). In addition, oligonucleotides can be modified with hybridization-triggered cleavage agents (van der Krol *et al.,* 1988) or intercalating agents (Zon, 1988). To this end, the oligonucleotide may be conjugated to another molecule, (e.g., a peptide, hybridization triggered cross-linking agent, transport agent, or hybridization-triggered cleavage agent).

Also included as part of this invention are molecular beacon oligonucleotide primer and probe molecules having one or more regions complementary to a target nucleotide sequence, two complementary regions one having a fluorophore and one a quencher such that the molecular beacon is useful for quantifying the presence of the nucleic acid in a sample. Molecular beacon nucleic acids are described, for example, in Lizardi et al., U.S. Pat. No. 5,854,033; Nazarenko et al., U.S. Pat. No. 5,866,336, and Livak et al., U.S. Pat. No. 5,876,930.

### Antibodies

An immunogen typically is used to prepare antibodies by immunizing a suitable subject, (e.g., rabbit, goat, mouse or other mammal). An appropriate immunogenic preparation can contain, for example, recombinantly expressed chemically synthesized polypeptide. The preparation can further include an adjuvant, such as Freund's complete or incomplete adjuvant, or a similar immunostimulatory agent. Amino acid polymorphisms can be detected using antibodies specific for the altered epitope by western analysis after the electrophoresis of denatured proteins. Protein polymorphism can also be detected using fluorescently identified antibodies which bind to specific polymorphic epitopes and detected in whole cells using fluorescence activated cell sorting techniques (FACS). Polymorphic protein sequence may also be determined by NMR spectroscopy or by x-ray diffraction studies. Further, determination of polymorphic sites in proteins may be accomplished by observing differential cleavage by specific or non specific proteases.

An antibody is an immunoglobulin molecule or immunologically active portion thereof, i.e., an antigen-binding portion. Examples of immunologically active portions of immunoglobulin molecules include F(ab) and F(ab')₂ fragments which can be generated by treating the antibody with an enzyme such as pepsin. An antibody can be polyclonal, monoclonal, or recombinant (e.g., a chimeric or humanized), fully human, non-human (e.g., murine), or a single chain antibody. An antibody may have effector function and can fix complement, and is sometimes coupled to a toxin or imaging agent.

A full-length polypeptide or antigenic peptide fragment encoded by a target nucleotide sequence can be used as an immunogen or can be used to identify antibodies made with other immunogens, e.g., cells, membrane preparations, and the like. An antigenic peptide often includes at least 8 amino acid residues of the amino acid sequences encoded by a nucleotide sequence of SEQ ID NO:19, and encompasses an epitope. Antigenic peptides sometimes include 10 or more amino acids, 15 or more amino acids, 20 or more amino acids, or 30 or more amino acids. Hydrophilic and hydrophobic fragments of polypeptides sometimes are used as immunogens.

Epitopes encompassed by the antigenic peptide are regions located on the surface of the polypeptide (e.g., hydrophilic regions) as well as regions with high antigenicity. For example, an Emini surface probability analysis of the human polypeptide sequence can be used to indicate the regions that have a particularly high probability of being localized to the surface of the polypeptide and are thus likely to constitute surface residues useful for targeting antibody production. The antibody may bind an epitope on any domain or region on polypeptides for use in the invention.

Also, chimeric, humanized, and completely human antibodies are useful for applications which include repeated administration to subjects. Chimeric and humanized monoclonal antibodies, comprising both human and non-human portions, can be made using standard recombinant DNA techniques. Such chimeric and humanized monoclonal antibodies can be produced by recombinant DNA techniques, for example using methods described in Robinson et al., PCT International Publication No. WO 87/02671; Akira, *et al.,* European Patent Application 184,187; Taniguchi, M., European Patent Application 171,496; Morrison *et al.,* European Patent Application 173,494; Neuberger et al., PCT International Publication No. WO 86/01533; Cabilly et al., U.S. Pat. No. 4,816,567; Cabilly *et al.,* European Patent Application 125,023; (Better *et al.,* 1988; Liu *et al.,* 1987a; Liu *et al.,* 1987b; Sun *et al.,* 1987; Nishimura *et al.,* 1987; Wood *et al.,* 1985; Shaw *et al.,* 1988; Morrison, 1985; Verhoeyen *et al.,* 1988; Beidler *et al.,* 1988) and Winter, U.S. Pat. No. 5,225,539.

Completely human antibodies can be particularly desirable for therapeutic treatment of human patients. Such antibodies can be produced using transgenic mice that are incapable of expressing endogenous immunoglobulin heavy and light chains genes, but which can express human heavy and light chain genes. See, for example, Lonberg and Huszar (Lonberg and Huszar, 1995) and U.S. Pat. Nos. 5,625,126; 5,633,425; 5,569,825; 5,661,016; and 5,545,806. In addition, companies such as Abgenix, Inc. (Fremont, Calif.) and Medarex, Inc. (Princeton, N.J.), can be engaged to provide human antibodies directed against a selected antigen. Completely human antibodies that recognize a selected epitope also can be generated using guided selection. In this approach a selected non-human monoclonal antibody (e.g., a murine antibody) is used to guide the selection of a completely human antibody recognizing the same epitope. This technology is described for example by Jespers *et al.* (Jespers *et al.,* 1994).

An antibody can be a single chain antibody. A single chain antibody (scFV) can be engineered (see, e.g., Colcher *et al.* (Colcher *et al.,* 1999) and Reiter (Reiter and Pastan, 1996). Single chain antibodies can be dimerized or multimerized to generate multivalent antibodies having specificities for different epitopes of the same target polypeptide.

Antibodies also may be selected or modified so that they exhibit reduced or no ability to bind an Fc receptor. For example, an antibody may be an isotype or subtype, fragment or other mutant, which does not support binding to an Fc receptor (e.g., it has a mutagenized or deleted Fc receptor binding region).

Also, an antibody (or fragment thereof) may be conjugated to a therapeutic moiety such as a cytotoxin, a therapeutic agent or a radioactive metal ion. A cytotoxin or cytotoxic agent includes any agent that is detrimental to cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1 dehydrotestosterone, glucocorticoids, procaine, tetracaine, lidocaine, propranolol, and puromycin and analogs or homologs thereof. Therapeutic agents include antimetabolites (e.g., methotrexate, 6-mercaptopurine, 6-thioguanine, cytarabine, 5-fluorouracil decarbazine), alkylating agents (e.g., mechlorethamine, thiotepa chlorambucil, melphalan, carmustine (BCNU) and lomustine (CCNU), cyclophosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine).

Antibody conjugates can be used for modifying a given biological response. For example, the drug moiety may be a protein or polypeptide possessing a desired biological activity. Such proteins may include, for example, a toxin such as abrin, ricin A, pseudomonas exotoxin, or diphtheria toxin; a polypeptide such as tumor necrosis factor, γ-interferon, α-interferon, nerve growth factor, platelet derived growth factor, tissue plasminogen activator; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other growth factors. Also, an antibody can be conjugated to a second antibody to form an antibody heteroconjugate as described by Segal in U.S. Pat. No. 4,676,980, for example.

An antibody (e.g., monoclonal antibody) can be used to isolate target polypeptides by standard techniques, such as affinity chromatography or immunoprecipitation. Moreover, an antibody can be used to detect a target polypeptide (e.g., in a cellular lysate or cell supernatant) in order to evaluate the abundance and pattern of expression of the polypeptide. Antibodies can be used diagnostically to monitor polypeptide levels in tissue as part of a clinical testing procedure, e.g., to determine the efficacy of a given treatment regimen. Detection can be facilitated by coupling (i.e., physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, .¹³¹I, ³⁵S or ³H. Also, an antibody can be utilized as a test molecule for determining whether it can treat colorectal cancer, and as a therapeutic for administration to a subject for treating colorectal cancer.

An antibody can be made by immunizing with a purified antigen, or a fragment thereof, a membrane associated antigen, tissues, e.g., crude tissue preparations, whole cells, preferably living cells, lysed cells, or cell fractions.

Included as part of this invention are antibodies which bind only a native polypeptide, only denatured or otherwise non-native polypeptide, or which bind both, as well as those having linear or conformational epitopes. Conformational epitopes sometimes can be identified by selecting antibodies that bind to native but not denatured polypeptide. Also featured are antibodies that specifically bind to a polypeptide variant associated with colorectal cancer.

### Screening Assays

The invention includes methods for identifying a candidate therapeutic for treating colorectal cancer. The methods include contacting a test molecule with a target molecule in a system. A target molecule is a nucleic acid molecule having a sequence of any of SEQ ID NOs:1 to 19, or a fragment thereof, or a polypeptide encoded by the nucleic acid molecule of SEQ ID NO:19. The method also includes determining the presence or absence of an interaction between the test molecule and the target molecule, where the presence of an interaction between the test molecule and the nucleic acid or polypeptide identifies the test molecule as a candidate colorectal cancer therapeutic. The interaction between the test molecule and the target molecule may be quantified.

Test molecules and candidate therapeutics include compounds, antisense nucleic acids, siRNA molecules, ribozymes, polypeptides or proteins encoded by target nucleic acids, and immunotherapeutics (e.g., antibodies and HLA-presented polypeptide fragments). A test molecule or candidate therapeutic may act as a modulator of target molecule concentration or target molecule function in a system. A modulator may agonize (i.e., up-regulates) or antagonize (i.e., down-regulates) a target molecule concentration partially or completely in a system by affecting such cellular functions as DNA replication and/or DNA processing (e.g., DNA methylation or DNA repair), RNA transcription and/or RNA processing (e.g., removal of intronic sequences and/or translocation of spliced mRNA from the nucleus), polypeptide production (e.g., translation of the polypeptide from mRNA), and/or polypeptide post-translational modification (e.g., glycosylation, phosphorylation, and proteolysis of pro-polypeptides). A modulator may also agonize or antagonize a biological function of a target molecule partially or completely, where the function may include adopting a certain structural conformation, interacting with one or more binding partners, ligand binding, catalysis (e.g., phosphorylation, dephosphorylation, hydrolysis, methylation, and isomerization), and an effect upon a cellular event (e.g., effecting progression of colorectal cancer).

According to an aspect of this invention a system, i.e., a cell free *in vitro* environment and a cell-based environment such as a collection of cells, a tissue, an organ, or an organism, is contacted with a test molecule in a variety of manners, including adding molecules in solution and allowing them to interact with one another by diffusion, cell injection, and any administration routes in an animal. An interaction refers to an effect of a test molecule on test molecule, where the effect sometimes is binding between the test molecule and the target molecule, and sometimes is an observable change in cells, tissue, or organism.

There are known methods for detecting the presence or absence of interaction between a test molecule and a target molecule. For example, titrametric, acidimetric, radiometric, NMR, monolayer, polarographic, spectrophotometric, fluorescent, and ESR assays probative of a target molecule interaction may be utilized.

Test molecule/target molecule interactions can be detected and/or quantified using known assays. For example, an interaction can be determined by labeling the test molecule and/or the target molecule, where the label is covalently or non-covalently attached to the test molecule or target molecule. The label is sometimes a radioactive molecule such as ¹²⁵I, ¹³¹I, ³⁵S or ³H, which can be detected by direct counting of radio-emission or by scintillation counting. Also, enzymatic labels such as horseradish peroxidase, alkaline phosphatase, or luciferase may be utilized where the enzymatic label can be detected by determining conversion of an appropriate substrate to product. In addition, presence or absence of an interaction can be determined without labeling. For example, a microphysiometer (e.g., Cytosensor) is an analytical instrument that measures the rate at which a cell acidifies its environment using a light-addressable potentiometric sensor (LAPS). Changes in this acidification rate can be used as an indication of an interaction between a test molecule and target molecule (McConnell *et al.,* 1992).

In cell-based systems, cells typically include a nucleic acid from SEQ ID NOs:1 to 19 or a polypeptide encoded by the nucleic acid molecule of SEQ ID NO:19, and are often of mammalian origin, although the cell can be of any origin. Whole cells, cell homogenates, and cell fractions (e.g., cell membrane fractions) can be subjected to analysis. Where interactions between a test molecule with a target polypeptide are monitored, soluble and/or membrane bound forms of the polypeptide may be utilized. Where membrane-bound forms of the polypeptide are used, it may be desirable to utilize a solubilizing agent. Examples of such solubilizing agents include non-ionic detergents such as n-octylglucoside, n-dodecylglucoside, n-dodecylmaltoside, octanoyl-N-methylglucamide, decanoyl-N-methylglucamide, Triton™X-100, Triton™ X-114, etc.

An interaction between a test molecule and target molecule also can be detected by monitoring fluorescence energy transfer (FET) (see, e.g., Lakowicz et al., U.S. Pat. No. 5,631,169; Stavrianopoulos et al., U.S. Pat. No. 4,868,103). A fluorophore label on a first, donor molecule is selected such that its emitted fluorescent energy will be absorbed by a fluorescent label on a second, acceptor molecule, which in turn is able to fluoresce due to the absorbed energy. Alternately, the donor polypeptide molecule may simply utilize the natural fluorescent energy of tryptophan residues. Labels are chosen that emit different wavelengths of light, such that the acceptor molecule label may be differentiated from that of the donor. Since the efficiency of energy transfer between the labels is related to the distance separating the molecules, the spatial relationship between the molecules can be assessed. In a situation in which binding occurs between the molecules, the fluorescent emission of the acceptor molecule label in the assay should be maximal. An FET binding event can be conveniently measured through standard fluorometric detection means well known in the art (e.g., using a fluorimeter).

In another embodiment, determining the presence or absence of an interaction between a test molecule and a target molecule can be effected by monitoring surface plasmon resonance (Sjolander and Urbaniczky, 1991; Szabo *et al.,* 1995). Surface plasmon resonance (SPR) or biomolecular interaction analysis (BIA) can be utilized to detect biospecific interactions in real time, without labeling any of the interactants (e.g., BIAcore). Changes in the mass at the binding surface (indicative of a binding event) result in alterations of the refractive index of light near the surface (the optical phenomenon of surface plasmon resonance, resulting in a detectable signal which can be used as an indication of real-time reactions between biological molecules.

In another embodiment, the target molecule or test molecules are anchored to a solid phase, facilitating the detection of target molecule/test molecule complexes and separation of the complexes from free, uncomplexed molecules. The target molecule or test molecule is immobilized to the solid support. In one embodiment, the target molecule is anchored to a solid surface, and the test molecule, which is not anchored, can be labeled, either directly or indirectly, with detectable labels.

It may be desirable to immobilize a target molecule, an anti-target molecule antibody, and/or test molecules to facilitate separation of target molecule/test molecule complexes from uncomplexed forms, as well as to accommodate automation of the assay. The attachment between a test molecule and/or target molecule and the solid support may be covalent or non-covalent (see, e.g., U.S. Pat. No. 6,022,688 for non-covalent attachments). The solid support may be one or more surfaces of the system, such as one or more surfaces in each well of a microtiter plate, a surface of a silicon wafer, a surface of a bead (Lam *et al.,* 1991) that is optionally linked to another solid support, or a channel in a microfluidic device, for example. Types of solid supports, linker molecules for covalent and non-covalent attachments to solid supports, and methods for immobilizing nucleic acids and other molecules to solid supports are known (see, e.g., U.S. Pat. Nos. 6,261,776; 5,900,481; 6,133,436; and 6,022,688; and WIPO publication WO 01/18234).

In one embodiment, a target molecule may be immobilized to surfaces via biotin and streptavidin. For example, a biotinylated polypeptide can be prepared from biotin-NHS (N-hydroxysuccinimide, e.g., biotinylation kit, Pierce Chemicals, Rockford, Ill.), and immobilized in the wells of streptavidin-coated 96 well plates (Pierce Chemical). In another embodiment, a target polypeptide can be prepared as a fusion polypeptide. For example, glutathione-S-transferase/-polypeptide fusion can be adsorbed onto glutathione sepharose beads (Sigma Chemical, St. Louis, Mo.) or glutathione derivatized microtiter plates, which are then combined with a test molecule under conditions conducive to complex formation (e.g., at physiological conditions for salt and pH). Following incubation, the beads or microtiter plate wells are washed to remove any unbound components, or the matrix is immobilized in the case of beads, and complex formation is determined directly or indirectly as described above. Alternatively, the complexes can be dissociated from the matrix, and the level of target molecule binding or activity is determined using standard techniques.

In one embodiment, the non-immobilized component is added to the coated surface containing the anchored component. After the reaction is complete, unreacted components are removed (e.g., by washing) under conditions such that a significant percentage of complexes formed will remain immobilized to the solid surface. The detection of complexes anchored on the solid surface can be accomplished in a number of manners. Where the previously non-immobilized component is pre-labeled, the detection of label immobilized on the surface indicates that complexes were formed. Where the previously non-immobilized component is not pre-labeled, an indirect label can be used to detect complexes anchored on the surface, e.g., by adding a labeled antibody specific for the immobilized component, where the antibody, in turn, can be directly labeled or indirectly labeled with, e.g., a labeled anti-Ig antibody.

In another embodiment, an assay is performed utilizing antibodies that specifically bind a target molecule or test molecule but do not interfere with binding of the target molecule to the test molecule. Such antibodies can be linked to a solid support, and unbound target molecule may be immobilized by antibody conjugation. Methods for detecting such complexes, in addition to those described above for the GST-immobilized complexes, include immunodetection of complexes using antibodies reactive with the target molecule, as well as enzyme-linked assays which rely on detecting an enzymatic activity associated with the target molecule.

Cell free assays also can be conducted in a liquid phase. In such an assay, reaction products are separated from unreacted components, by known techniques, including: differential centrifugation (Rivas and Minton, 1993); electrophoresis (1999) and immunoprecipitation (1999). Media and chromatographic techniques are known (Heegaard, 1998; Hage and Tweed, 1997). Further, fluorescence energy transfer may also be conveniently utilized to detect binding without further purification of the complex from solution.

In another embodiment, modulators of target molecule expression are identified. For example, a cell or cell free mixture is contacted with a candidate compound and the expression of target mRNA or polypeptide is evaluated relative to the level of expression of target mRNA or polypeptide in the absence of the candidate compound. When expression of target mRNA or polypeptide is greater in the presence of the candidate compound than in its absence, the candidate compound is identified as an agonist of target mRNA or polypeptide expression. Alternatively, when expression of target mRNA or polypeptide is less (e.g., less with statistical significance) in the presence of the candidate compound than in its absence, the candidate compound is identified as an antagonist or inhibitor of target mRNA or polypeptide expression. The level of target mRNA or polypeptide expression can be determined by methods described herein.

In another embodiment, binding partners that interact with a target molecule are detected. The target molecules can interact with one or more cellular or extra-cellular macromolecules, such as polypeptides *in vivo,* and these interacting molecules or binding partners. Binding partners can agonize or antagonize target molecule biological activity. Also, test molecules that agonize or antagonize interactions between target molecules and binding partners can be useful as therapeutic molecules as they can up-regulate or down-regulated target molecule activity *in vivo* and thereby treat colorectal cancer.

Binding partners of target molecules can be identified by known methods. For example, binding partners may be identified by lysing cells and analyzing cell lysates by electrophoretic techniques. Alternatively, a two-hybrid assay or three-hybrid assay can be utilized (Zervos *et al.,* 1993; Madura *et al.,* 1993; Bartel *et al.,* 1993; Iwabuchi *et al.,* 1993): see also, e.g., U.S. Pat. No. 5,283,317 and Brent WO94/10300. A two-hybrid system is based on the modular nature of most transcription factors, which consist of separable DNA-binding and activation domains. The assay often utilizes two different DNA constructs. In one construct, a nucleic acid of SEQ ID NO:19, sometimes referred to as the bait, is fused to a gene encoding the DNA binding domain of a known transcription factor (e.g., GAL-4). In another construct, a DNA sequence from a library of DNA sequences that encodes a potential binding partner, sometimes referred to as the prey, is fused to a gene that encodes an activation domain of the known transcription factor. Sometimes, a target nucleic acid can be fused to the activation domain. If the bait and the prey molecules interact *in vivo,* the DNA-binding and activation domains of the transcription factor are brought into close proximity. This proximity allows transcription of a reporter gene (e.g., *lacZ*) which is operably linked to a transcriptional regulatory site responsive to the transcription factor. Expression of the reporter gene can be detected and cell colonies containing the functional transcription factor can be isolated and used to identify the potential binding partner.

In an embodiment for identifying test molecules that antagonize or agonize complex formation between target molecules and binding partners, a reaction mixture containing the target molecule and the binding partner is prepared, under conditions and for a time sufficient to allow complex formation. The reaction mixture often is provided in the presence or absence of the test molecule. The test molecule can be included initially in the reaction mixture, or can be added at a time subsequent to the addition of the target molecule and its binding partner. Control reaction mixtures are incubated without the test molecule or with a placebo. Formation of any complexes between the target molecule and the binding partner then is detected. Decreased formation of a complex in the reaction mixture containing test molecule as compared to in a control reaction mixture indicates that the molecule antagonizes target molecule/binding partner complex formation. Alternatively, increased formation of a complex in the reaction mixture containing test molecule as compared to in a control reaction mixture, indicates that the molecule agonizes target molecule/binding partner complex formation. In another embodiment, complex formation of target molecule/binding partner can be compared to complex formation of mutant target molecule/binding partner (e.g., amino acid modifications in a target polypeptide). Such a comparison can be important in those cases where it is desirable to identify test molecules that modulate interactions of mutant but not non-mutated target gene products.

The assays can be conducted in a heterogeneous or homogeneous format. In heterogeneous assays, a target molecule and/or the binding partner are immobilized to a solid phase, and complexes are detected on the solid phase at the end of the reaction. In homogeneous assays, the entire reaction is carried out in a liquid phase. In either approach, the order of addition of reactants can be varied to obtain different information about the molecules being tested. For example, test compounds that agonize target molecule/binding partner interactions can be identified by conducting the reaction in the presence of the test molecule in a competition format. Alternatively, test molecules that agonize preformed complexes, e.g., molecules with higher binding constants that displace one of the components from the complex, can be tested by adding the test compound to the reaction mixture after complexes have been formed.

In a heterogeneous assay, the target molecule or the binding partner is anchored onto a solid surface (e.g., a microtiter plate), while the non-anchored species is labeled, either directly or indirectly. The anchored molecule can be immobilized by non-covalent or covalent attachments. Alternatively, an immobilized antibody specific for the molecule to be anchored can be used to anchor the molecule to the solid surface. The partner of the immobilized species is exposed to the coated surface with or without the test molecule. After the reaction is complete, unreacted components are removed (e.g., by washing) such that a significant portion of any complexes formed will remain immobilized on the solid surface. Where the non-immobilized species is pre-labeled, the detection of label immobilized on the surface is indicative of complex. Where the non-immobilized species is not pre-labeled, an indirect label can be used to detect complexes anchored to the surface; e.g., by using a labeled antibody specific for the initially non-immobilized species. Depending upon the order of addition of reaction components, test compounds that inhibit complex formation or that disrupt preformed complexes can be detected.

The reaction can be conducted in a liquid phase in the presence or absence of test molecule, where the reaction products are separated from unreacted components, and the complexes are detected (e.g., using an immobilized antibody specific for one of the binding components to anchor any complexes formed in solution, and a labeled antibody specific for the other partner to detect anchored complexes). Again, depending upon the order of addition of reactants to the liquid phase, test compounds that inhibit complex or that disrupt preformed complexes can be identified.

In an alternate embodiment, a homogeneous assay can be utilized. For example, a preformed complex of the target gene product and the interactive cellular or extra-cellular binding partner-product is prepared. One or both of the target molecule or binding partner is labeled, and the signal generated by the label(s) is quenched upon complex formation (e.g., U.S. Pat. No. 4,109,496 that-utilizes this approach for immunoassays). Addition of a test molecule that competes with and displaces one of the species from the preformed complex will result in the generation of a signal above background. In this way, test substances that disrupt target molecule/binding partner complexes can be identified.

### Identification of Candidate Therapeutics

Candidate therapeutics for treating colorectal cancer are identified from a group of test molecules that interact with a target molecule. Test molecules are normally ranked according to the degree with which they modulate (e.g., agonize or antagonize) a function associated with the target molecule (e.g., DNA replication and/or processing, RNA transcription and/or processing, polypeptide production and/or processing, and/or biological function/activity), and then top ranking modulators are selected. Also, pharmacogenomic information can determine the rank of a modulator. The top 10% of ranked test molecules often are selected for further testing as candidate therapeutics, and sometimes the top 15%, 20%, or 25% of ranked test molecules are selected for further testing as candidate therapeutics. Candidate therapeutics typically are formulated for administration to a subject.

### Therapeutic Formulations

Formulations and pharmaceutical compositions typically include in combination with a pharmaceutically acceptable carrier one or more target molecule modulators. The modulator often is a test molecule identified as having an interaction with a target molecule by a screening method. The modulator may be a compound, an antisense nucleic acid, a ribozyme, an antibody, or a binding partner. Also, formulations may include a polypeptide combination with a pharmaceutically acceptable carrier.

A pharmaceutically acceptable carrier includes solvents, dispersion media, coatings, antibacterial and antifungal agents, isotonic and absorption delaying agents, and the like, compatible with pharmaceutical administration. See for example, Remington's Pharmaceutical Sciences (2005). Supplementary active compounds can also be incorporated into the compositions. Pharmaceutical compositions can be included in a container, pack, or dispenser together with instructions for administration.

A pharmaceutical composition typically is formulated to be compatible with its intended route of administration. Examples of routes of administration include parenteral, e.g., intravenous, intradermal, subcutaneous, oral (e.g., inhalation), transdermal (topical), transmucosal, and rectal administrations Solutions or suspensions used for parenteral, intradermal, or subcutaneous application can include the following components: a sterile diluent such as water for injection, saline solution, fixed oils, polyethylene glycols, glycerin, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Oral compositions generally include an inert diluent or an edible carrier. For the purpose of oral therapeutic administration, the active compound can be incorporated with excipients and used in the form of tablets, troches, or capsules, e.g., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). The composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol or sorbitol, and/or sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the methods of preparation often utilized are vacuum drying and freeze-drying which yields a powder of the active ingredient plus any additional desired ingredient from a previously sterile-filtered solution thereof.

Systemic administration might be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art. Molecules can also be prepared in the form of suppositories (e.g., with conventional suppository bases such as cocoa butter and other glycerides) or retention enemas for rectal delivery.

In one embodiment, active molecules are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. Materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No. 4,522,811.

It is advantageous to formulate oral or parenteral compositions in dosage unit form for ease of administration and uniformity of dosage. Each unit containing a predetermined quantity of active compound is calculated to produce the desired therapeutic effect in association with the required pharmaceutical carrier.

Toxicity and therapeutic efficacy of such compounds can be determined by standard pharmaceutical procedures in cell cultures or experimental animals, e.g., for determining the LD₅₀ (the dose lethal to 50% of the population) and the ED.sub.50 (the dose therapeutically effective in 50% of the population). The dose ratio between toxic and therapeutic effects is the therapeutic index and it can be expressed as the ratio LD₅₀/ED₅₀. Molecules which exhibit high therapeutic indices often are utilized. While molecules that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such molecules typically lies within a range of circulating concentrations that include the ED₅₀ with little or no toxicity. The dosage may vary within this range depending upon the dosage form employed and the route of administration utilized. For any molecules used in methods described herein, the therapeutically effective dose can be estimated initially from cell culture assays. A dose may be formulated in animal models to achieve a circulating plasma concentration range that includes the IC.sub.50 (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

As defined herein, a therapeutically effective amount of protein or polypeptide (i.e., an effective dosage) ranges from about 0.001 to 30 mg/kg body weight, sometimes about 0.01 to 25 mg/kg body weight, often about 0.1 to 20 mg/kg body weight, and more often about 1 to 10 mg/kg, 2 to 9 mg/kg, 3 to 8 mg/kg, 4 to 7 mg/kg, or 5 to 6 mg/kg body weight. The protein or polypeptide can be administered one time per week for between about 1 to 10 weeks, sometimes between 2 to 8 weeks, often between about 3 to 7 weeks, and more often for about 4, 5, or 6 weeks. The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of a protein, polypeptide, or antibody can include a single treatment or, can include a series of treatments.

For antibodies, a dosage of 0.1 mg/kg of body weight (generally 10 mg/kg to 20 mg/kg) is often utilized. If the antibody is to act in the brain, a dosage of 50 mg/kg to 100 mg/kg is often appropriate. Generally, partially human antibodies and fully human antibodies have a longer half-life within the human body than other antibodies. Accordingly, lower dosage and less frequent administration is often possible. Modifications such as lipidation can be used to stabilize antibodies and to enhance uptake and tissue penetration (e.g., into the brain). A method for lipidation of antibodies is described by Cruikshank *et al.* (Cruikshank *et al.,* 1997).

Antibody conjugates can be used for modifying a given biological response, the drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a polypeptide such as tumor necrosis factor, alpha-interferon, beta-interferon, nerve growth factor, platelet derived growth factor, tissue plasminogen activator; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other growth factors. Alternatively, an antibody can be conjugated to a second antibody to form an antibody heteroconjugate as described by Segal in U.S. Pat. No. 4,676,980.

For compounds, exemplary doses include milligram or microgram amounts of the compound per kilogram of subject or sample weight, for example, about 1 microgram per kilogram to about 500 milligrams per kilogram, about 100 micrograms per kilogram to about 5 milligrams per kilogram, or about 1 microgram per kilogram to about 50 micrograms per kilogram. It is understood that appropriate doses of a small molecule depend upon the potency of the small molecule with respect to the expression or activity to be modulated. When one or more of these small molecules is to be administered to an animal (e.g., a human) in order to modulate expression or activity of a polypeptide or nucleic acid described herein, a physician, veterinarian, or researcher may, for example, prescribe a relatively low dose at first, subsequently increasing the dose until an appropriate response is obtained. In addition, it is understood that the specific dose level for any particular animal subject will depend upon a variety of factors including the activity of the specific compound employed, the age, body weight, general health, gender, and diet of the subject, the time of administration, the route of administration, the rate of excretion, any drug combination, and the degree of expression or activity to be modulated.

With regard to nucleic acid formulations, gene therapy vectors can be delivered to a subject by, for example, intravenous injection, local administration (see, e.g., U.S. Pat. No. 5,328,470) or by stereotactic injection (Chen *et al.,* 1994). Pharmaceutical preparations of gene therapy vectors can include a gene therapy vector in an acceptable diluent, or can comprise a slow release matrix in which the gene delivery vehicle is imbedded. Alternatively, where the complete gene delivery vector can be produced intact from recombinant cells (e.g., retroviral vectors) the pharmaceutical preparation can include one or more cells which produce the gene delivery system. Examples of gene delivery vectors are described herein.

### Therapeutic Methods

A therapeutic formulation described above can be administered to a subject in need of a therapeutic for treating colorectal cancer. Therapeutic formulations can be administered by any of the paths described herein. With regard to both prophylactic and therapeutic methods of treatment, such treatments may be specifically tailored or modified, based on knowledge obtained from pharmacogenomic analyses described herein.

A treatment is the application or administration of a therapeutic formulation to a subject, or application or administration of a therapeutic agent to an isolated tissue or cell line from a subject with the purpose to cure, heal, alleviate, relieve, alter, remedy, ameliorate, improve or affect colorectal cancer, symptoms of colorectal cancer or a predisposition towards colorectal cancer. A therapeutic formulation includes small molecules, peptides, antibodies, ribozymes and antisense oligonucleotides. Administration of a therapeutic formulation can occur prior to the manifestation of symptoms characteristic of colorectal cancer, such that the cancer is prevented or delayed in its progression. The appropriate therapeutic composition can be determined based on screening assays described herein.

As discussed, successful treatment of colorectal cancer can be brought about by techniques that serve to agonize target molecule expression or function, or alternatively, antagonize target molecule expression or function. These techniques include administration of modulators that include, but are not limited to, small organic or inorganic molecules; antibodies (including, for example, polyclonal, monoclonal, humanized, anti-idiotypic, chimeric or single chain antibodies, and FAb, F(ab')₂ and FAb expression library fragments, scFV molecules, and epitope-binding fragments thereof); and peptides, phosphopeptides, or polypeptides.

Further, antisense and ribozyme molecules that inhibit expression of the target gene can also be used to reduce the level of target gene expression, thus effectively reducing the level of target gene activity. Still further, triple helix molecules can be utilized in reducing the level of target gene activity. Antisense, ribozyme and triple helix molecules are discussed above. It is possible that the use of antisense, ribozyme, and/or triple helix molecules to reduce or inhibit mutant gene expression can also reduce or inhibit the transcription (triple helix) and/or translation (antisense, ribozyme) of mRNA produced by normal target gene alleles, such that the concentration of normal target gene product present can be lower than is necessary for a normal phenotype. In such cases, nucleic acid molecules that encode and express target gene polypeptides exhibiting normal target gene activity can be introduced into cells via gene therapy method. Alternatively, in instances in that the target gene encodes an extra-cellular polypeptide, it can be preferable to co-administer normal target gene polypeptide into the cell or tissue in order to maintain the requisite level of cellular or tissue target gene activity.

Another method by which nucleic acid molecules may be utilized in treating or preventing colorectal cancer is use of aptamer molecules specific for target molecules. Aptamers are nucleic acid molecules having a tertiary structure which permits them to specifically bind to ligands (Osborne *et al.,* 1997; Patel, 1997).

Yet another method of utilizing nucleic acid molecules for colorectal cancer treatment is gene therapy, which can also be referred to as allele therapy. The invention thus includes a gene therapy method for treating colorectal cancer in a subject, which includes contacting one or more cells in the subject or from the subject with a nucleic acid having a first nucleotide sequence. Genomic DNA in the subject includes a second nucleotide sequence having one or more SNPs associated with colorectal cancer. The first and second nucleotide sequences typically are substantially identical to one another, and the first nucleotide sequence comprises fewer SNPs associated with colorectal cancer than the second nucleotide sequence. The first nucleotide sequence may comprise a gene sequence that encodes a full-length polypeptide or a fragment thereof. The subject is often a human. Allele therapy methods often are utilized in conjunction with a method of first determining whether a subject has genomic DNA that includes SNPs associated with colorectal cancer.

Another allele therapy is a method which comprises contacting one or more cells in the subject or from the subject with a polypeptide encoded by a nucleic acid having a first nucleotide sequence. Genomic DNA in the subject includes a second nucleotide sequence having one or more SNPs associated with colorectal cancer. The first and second nucleotide sequences typically are substantially identical to one another, and the first nucleotide sequence includes fewer SNPs associated with colorectal cancer than the second nucleotide sequence. The first nucleotide sequence may include a gene sequence that encodes a full-length polypeptide or a fragment thereof. The subject is usually a human.

For antibody-based therapies, antibodies can be generated that are both specific for target molecules and that reduce target molecule activity. Such antibodies may be administered in instances where antagonizing a target molecule function is appropriate for the treatment of colorectal cancer.

In circumstances where stimulating antibody production in an animal or a human subject by injection with a target molecule is harmful to the subject, it is possible to generate an immune response against the target molecule by use of anti-idiotypic antibodies (Herlyn and Birebent, 1999; Bhattacharya-Chatterjee and Foon, 1998). Introducing an anti-idiotypic antibody to a mammal or human subject often stimulates production of anti-anti-idiotypic antibodies, which typically are specific to the target molecule. Vaccines directed to colorectal cancer also may be generated in this fashion.

In instances where the target molecule is intracellular and whole antibodies are used, internalizing antibodies often are utilized. Lipofectin or liposomes can be used to deliver the antibody or a fragment of the Fab region that binds to the target antigen into cells. Where fragments of the antibody are used, the smallest inhibitory fragment that binds to the target antigen often is utilized. For example, peptides having an amino acid sequence corresponding to the Fv region of the antibody can be used. Alternatively, single chain neutralizing antibodies that bind to intracellular target antigens can also be administered. Such single chain antibodies can be administered, for example, by expressing nucleotide sequences encoding single-chain antibodies within the target cell population (Marasco *et al.,* 1993).

Modulators can be administered to a patient at therapeutically effective doses to treat colorectal cancer. A therapeutically effective dose refers to an amount of the modulator sufficient to result in amelioration of symptoms of colorectal cancer. Toxicity and therapeutic efficacy of modulators can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Modulators that exhibit large therapeutic indices often are utilized. While modulators that exhibit toxic side effects can be used, care should be taken to design a delivery system that targets such molecules to the site of affected tissue in order to minimize potential damage to uninfected cells, thereby reducing side effects.

Data obtained from cell culture assays and animal studies can be used in formulating a range of dosages for use in humans. The dosage of such compounds typically lies within a range of circulating concentrations that include the ED₅₀ with little or no toxicity. The dosage can vary within this range depending upon the dosage form employed and the route of administration utilized. The therapeutically effective dose can be estimated initially from cell culture assays. A dose can be formulated in animal models to achieve a circulating plasma concentration range that includes the IC₅₀ (i.e., the concentration of the test compound that achieves a half-maximal inhibition of symptoms) as determined in cell culture. Such information can be used to more accurately determine useful doses in humans. Levels in plasma can be measured, for example, by high performance liquid chromatography.

Another example of effective dose determination for an individual is the ability to directly assay levels of "free" and "bound" compound in the serum of the test subject. Such assays may utilize antibody mimics and/or "biosensors" that have been created through molecular imprinting techniques. Molecules that modulate target molecule activity are used as a template, or "imprinting molecule", to spatially organize polymerizable monomers prior to their polymerization with catalytic reagents. The subsequent removal of the imprinted molecule leaves a polymer matrix which contains a repeated "negative image" of the compound and is able to selectively rebind the molecule under biological assay conditions. A detailed review of this technique can be seen in Ansell *et al.* (Ansell *et al.,* 1996). Such "imprinted" affinity matrixes are amenable to ligand-binding assays, whereby the immobilized monoclonal antibody component is replaced by an appropriately imprinted matrix. An example of the use of such matrixes in this way can be seen in Vlatakis, *et al.* (Vlatakis *et al.,* 1993). Through the use of isotope-labeling, the "free" concentration of compound which modulates target molecule expression or activity readily can be monitored and used in calculations of IC₅₀. Such "imprinted" affinity matrixes can also be designed to include fluorescent groups whose photon-emitting properties measurably change upon local and selective binding of target compound. These changes readily can be assayed in real time using appropriate fiberoptic devices, in turn allowing the dose in a test subject to be quickly optimized based on its individual IC₅₀.

The examples set forth below are intended to illustrate but not limit the invention.

Genomic DNA samples from patients aged 25-74 and patients with both familial and sporadic CRC with family and unrelated ethnically matched controls were studied. We identified CRC-associated alleles by measuring 385,562 single nucleotide polymorphisms in peripheral blood DNA from 2,128 subjects (1,059 cases with colorectal cancer and 1,069 age matched individuals undiseased at the time of testing), and validating the identified CRC-associated alleles by using peripheral blood DNA from a second and third, different, group of 2,194 subjects (687 and 452 cases, respectively, with colorectal cancer and 688 and 367 age matched individuals undiseased, respectively, at the time of testing). Patients with clinically documented well characterized inherited colorectal cancer syndromes such as Familial Adenomatous Polyposis (FAP) or Hereditary Non Polyposis Colorectal Cancer were excluded from our analysis. Single nucleotide polymorphisms were selected to maximize measurement of genomic variability by choosing these markers that were in the greatest degree of linkage disequilibrium with neighboring SNPs. This was determined by calculating correlation coefficients (r²) with successive neighboring SNPs at each site of polymorphism until an arbitrary cut off of 0.8 was observed. Marker SNPs selected for measurement were in linkage disequilibrium with a maximal number of adjacent SNPs, thus providing an economical method for measuring diversity over a large portion of the genome.

Single Nucleotide Polymorphisms selected for study were derived from the International Haplotype Mapping Project (http://www.hapmap.org) August 2004 release, information about which is available from the National Institutes of Health, National Institutes of Health (NIH; http://www.nih.gov/), 9000 Rockville Pike, Bethesda, Maryland 20892. The SNPs were analyzed on DNA from our control and study population using the Affymetrix GeneChip® Human Mapping 500K Array Set platform (http://www.affymetrix.com, Affymetrix, Inc., 3380 Central Expressway, Santa Clara, CA 95051). The SNPs for the Affymetrix GeneChip® Human Mapping 500K Array Set platforms were selected as to cover the entire genome, but the SNPs were preferentially selected in genic regions present on *Nsp*I and *Sty*I restriction fragments varying in length from about 200 base pairs to about 1100 base pairs. Data was stored and organized using the Nanuq informatics environment of the McGill University and Genome Quebec Innovation Centre (http://www.genomequebec.mcgill.ca/; McGill University and Genome Québec Innovation Centre, 740, Docteur Penfield Avenue, Montreal, Québec H3A 1A4). Allele frequencies found within DNA from patients with colorectal cancer and those without this disease were compared using the univariate Mantel-Haenszel Chi-Square statistic.

The inventors of the present invention have discovered single base pair polymorphisms that are present in a highly significant percentage of the genetic DNA of individuals effected with colorectal cancer while only present in a smaller percentage of individuals who are not known to be affected by the disease.

### Example 1

For individuals with colorectal cancer, the distribution of polymorphic alleles at position 110676919 of chromosome 11, found within the LOC120376 gene, was different from those without colorectal cancer (Table 1). The trend test for risk associated with carrying the C allele had an empirical p-value of 0.000600 based on permutation analysis, and the corresponding Mantel-Haenszel odds ratio for trend is 1.237 (Table 1). These data further suggest that this marker, located within the LOC120376 gene, is associated with colorectal cancer risk and that the C allele at position 110676919 of chromosome 11 is associated with an increased risk of developing colorectal cancer.

| **Table 1** | | | | | | | | |
|---|---|---|---|---|---|---|---|---|
| rs no. | | | | 3802842 | | | | |
| Chromosome; Position | | | | 11; 110676919 | | | | |
| Gene Name | | | | LOC120376 | | | | |
| SEQ ID NO; Position | | | | 19; 2145 | | | | |
| Genotype; Phenotype | | | | n=C; increased risk | | | | |
| Hardy-Weinberg | | | | 0.88774 | | | | |
| Case Flag | Allele B | AA | AB | | BB | Model | p-Value | Odds Ratio |
| 0 | C | 566 | 482 | | 100 | Trend | 0.000600 | 1.237 |
| 1 | C | 486 | 513 | | 131 | | | |

Table 1A indicates SNPs found to be in strong linkage disequilibrium with rs3802842. To generate this list, correlation coefficients (r²) were calculated between the index SNP and all neighboring SNPs cited in the June 2006 HapMap data set release. An r² cut off of 0.50 was selected for inclusion as evidence for strong genetic linkage, i.e., a "strong linkage disequilibrium.

| **Table 1A** | **Linked SNPs** | | |
|---|---|---|---|
| **SNP** | **r²** | **Position on chr11** | **SEQ ID NO** |
| rs11213801 | 0.858 | 110624904 | 1 |
| rs10891239 | 0.676 | 110625908 | 2 |
| rs2001754 | 0.87 | 110630722 | 3 |
| rs7116087 | 0.831 | 110638215 | 4 |
| rs11213809 | 0.874 | 110640955 | 5 |
| rs4548657 | 0.956 | 110645253 | 6 |
| rs1987128 | 0.956 | 110657706 | 7 |
| rs7130173 | 0.951 | 110659282 | 8 |
| rs3087967 | 0.955 | 110662046 | 9 |
| rs4477469 | 1.0 | 110665963 | 10 |
| rs10789822 | 1.0 | 110667901 | 11 |
| rs6589218 | 0.84 | 110672767 | 12 |
| rs3802840 | 1.0 | 110676856 | 13 |
| rs3802842 | - | 110676919 | 14 |
| rs4608113 | 1.0 | 110677389 | 15 |
| rs6589220 | 0.952 | 110678500 | 16 |
| rs11213825 | 0.545 | 110685600 | 17 |
| rs7933961 | 0.513 | 110685841 | 18 |

Another aspect of the invention is a method of diagnosing colorectal cancer in an individual, or determining whether the individual is at altered risk for colorectal cancer, by detecting polymorphism in a subject by treating a tissue sample from the subject with an antibody to a polymorphic genetic variant of the present invention and detecting binding of said antibody. A person of skill in the art would know how to produce such an antibody (see, for instance, Harlow, E. and Lane, eds., 1988, "Antibodies: A Laboratory Manual", Cold Spring Harbor Press, Cold Spring Harbor). Such antibodies may include, but are not limited to polyclonal antibodies, monoclonal antibodies (mAbs), humanized or chimeric antibodies, single chain antibodies, Fab fragments, F(ab')₂ fragments, fragments produced by a Fab expression library, anti-idiotypic (anti-Id) antibodies, and epitope-binding fragments of any of the above. The present invention also provides an animal model to study colorectal cancer and susceptibility to colorectal cancer. Such studies can be performed using transgenic animals. For example, one can produce transgenic mice, which contain a specific allelic variant of a containing any of the SNPs disclosed herein. These mice can be created, e.g., by replacing their wild-type gene with an allele containing a SNP disclosed herein, or of the corresponding human gene containing such a SNP.

In a preferred embodiment, the present invention provides a transgenic mammalian animal, said animal having cells incorporating a recombinant expression system adapted to express a gene containing a SNP disclosed herein (preferably the human gene containing a SNP disclosed herein). Generally, the recombinant expression system will be stably integrated into the genome of the transgenic animal and will thus be heritable so that the offspring of such a transgenic animal may themselves contain the transgene. Transgenic animals can be engineered by introducing the a nucleic acid molecule containing only the coding portion of the gene into the genome of animals of interest, using standard techniques for producing transgenic animals. Animals that can serve as a target for transgenic manipulation include, without limitation, mice, rats, rabbits, guinea pigs, sheep, goats, pigs, and non-human primates, e.g. baboons, chimpanzees and monkeys. Techniques known in the art to introduce a transgene into such animals include pronucleic microinjection (U.S. Pat. No. 4,873,191); retrovirus-mediated gene transfer into germ lines (e.g. Van der Putten et al. 1985, Proc. Natl. Acad. Sci. USA 82: 6148-6152); gene targeting in embryonic stem cells (Thompson et al., Cell 56 (1989), 313-321); electroporation of embryos and sperm-mediated gene transfer (for a review, see for example, U.S. Pat. No. 4,736,866). For the purpose of the present invention, transgenic animals include those that carry the recombinant molecule only in part of their cells ("mosaic animals"). The molecule can be integrated either as a single transgene, or in concatamers. Selective introduction of a nucleic acid molecule into a particular cell type is also possible by following, for example, the technique of Lasko et al., Proc. Natl. Acad. Sci. USA 89 (1992): 6232-6236. Particular cells could also be targeted for molecular incorporation with tissue-specific enhancers. The expression of the integrated molecule can be monitored by standard techniques such as *in situ* hybridization, Northern Blot analysis, PCR or immunocytochemistry. Transgenic animals that include a copy of such a nucleic acid molecule introduced into the germ line of the animal at an embryonic stage can be used to examine the effect of increased expression of DNA encoding the corresponding protein. In accordance with this facet of the invention, an animal is treated with the reagent and a reduced incidence of the pathological condition, compared to untreated animals bearing the transgene, would indicate a potential therapeutic intervention for the pathological condition.

The present invention has been described in detail by way of illustration and example in order to acquaint others skilled in the art with the invention, its principles and its practical application. Particular formulations and processes of the present invention are not limited to the descriptions of the specific embodiments presented, but rather the descriptions and examples should be viewed in terms of the claims that follow and their equivalents. While some of the examples and descriptions above include some conclusions about the way the invention may function, the inventors do not intend to be bound by those conclusions and functions, but put them forth only as possible explanations.

It is to be further understood that the specific embodiments of the present invention as set forth are not intended as being exhaustive or limiting of the invention, and that many alternatives, modifications and variations will be apparent to those of ordinary skill in the art in light of the foregoing examples and detailed description. Accordingly, this invention is intended to embrace all such alternatives, modifications and variations that fall within the spirit and scope of the following claims.

### Reference List

All publications mentioned in the specification are herein incorporated by reference to the same extent as if each individual publication was specifically and individually indicated to be incorporated by reference herein.
(1999) Current Protocols in Molecular Biology. Wiley, New York.
(2005) Remington's Pharmaceutical Sciences. Mack.
Abderrahmani A, Steinmann M, Plaisance V, Niederhauser G, Haefliger J A, Mooser V, Bonny C, Nicod P and Waeber G (2001) The Transcriptional Repressor REST Determines the Cell-Specific Expression of the Human MAPK8IP1 Gene Encoding IB1 (JIP-1). Mol Cell Biol 21: pp 7256-7267.
Abecasis GR, Noguchi E, Heinzmann A, Traherne J A, Bhattacharyya S, Leaves N I, Anderson G G, Zhang Y, Lench N J, Carey A, Cardon L R, Moffatt M F and Cookson W O (2001) Extent and Distribution of Linkage Disequilibrium in Three Genomic Regions. Am J Hum Genet 68: pp 191-197.
Agresti A (2001) Exact Inference for Categorical Data: Recent Advances and Continuing Controversies. Stat Med 20: pp 2709-2722.
Ansell RJ, Kriz D and Mosbach K (1996) Molecularly Imprinted Polymers for Bioanalysis: Chromatography, Binding Assays and Biomimetic Sensors. Curr Opin Biotechnol 7: pp 89-94.
Banerji J, Olson L and Schaffner W (1983) A Lymphocyte-Specific Cellular Enhancer Is Located Downstream of the Joining Region in Immunoglobulin Heavy Chain Genes. Cell 33: pp 729-740.
Bartel DP and Szostak J W (1993) Isolation of New Ribozymes From a Large Pool of Random Sequences. Science 261: pp 1411-1418.
Bartel P, Chien C T, Sternglanz R and Fields S (1993) Elimination of False Positives That Arise in Using the Two-Hybrid System. Biotechniques 14: pp 920-924.
Beidler CB, Ludwig J R, Cardenas J, Phelps J, Papworth C G, Melcher E, Sierzega M, Myers L J, Unger B W, Fisher M and. (1988) Cloning and High Level Expression of a Chimeric Antibody With Specificity for Human Carcinoembryonic Antigen. J Immunol 141: pp 4053-4060.
Better M, Chang C P, Robinson R R and Horwitz A H (1988) Escherichia Coli Secretion of an Active Chimeric Antibody Fragment. Science 240: pp 1041-1043.
Bhattacharya-Chatterjee M and Foon K A (1998) Anti-Idiotype Antibody Vaccine Therapies of Cancer. Cancer Treat Res 94:51-68.: pp 51-68.
Bosher JM and Labouesse M (2000) RNA Interference: Genetic Wand and Genetic Watchdog. Nat Cell Biol 2: pp E31-E36.
Byrne GW and Ruddle F H (1989) Multiplex Gene Regulation: a Two-Tiered Approach to Transgene Regulation in Transgenic Mice. Proc Natl Acad Sci U S A 86: pp 5473-5477.
Calame K and Eaton S (1988) Transcriptional Controlling Elements in the Immunoglobulin and T Cell Receptor Loci. Adv Immunol 43:235-75.: pp 235-275.
Camper SA and Tilghman S M (1989) Postnatal Repression of the Alpha-Fetoprotein Gene Is Enhancer Independent. Genes Dev 3: pp 537-546.
Caplen NJ, Parrish S, Imani F, Fire A and Morgan R A (2001a) Specific Inhibition of Gene Expression by Small Double-Stranded RNAs in Invertebrate and Vertebrate Systems. Proc Natl Acad Sci U S A 98: pp 9742-9747.
Caplen NJ, Parrish S, Imani F, Fire A and Morgan R A (2001b) Specific Inhibition of Gene Expression by Small Double-Stranded RNAs in Invertebrate and Vertebrate Systems. Proc Natl Acad Sci U S A 98: pp 9742-9747.
Carstensen B, Soll-Johanning H, Villadsen E, Sondergaard J O and Lynge E (1996) Familial Aggregation of Colorectal Cancer in the General Population. Int J Cancer 68: pp 428-435.
Chen SH, Shine H D, Goodman J C, Grossman R G and Woo S L (1994) Gene Therapy for Brain Tumors: Regression of Experimental Gliomas by Adenovirus-Mediated Gene Transfer in Vivo. Proc Natl Acad Sci U S A 91: pp 3054-3057.
Cho CY, Moran E J, Cherry S R, Stephans J C, Fodor S P, Adams C L, Sundaram A, Jacobs J W and Schultz P G (1993) An Unnatural Biopolymer. Science 261: pp 1303-1305.
Clark AG, Weiss K M, Nickerson D A, Taylor S L, Buchanan A, Stengard J, Salomaa V, Vartiainen E, Perola M, Boerwinkle E and Sing C F (1998) Haplotype Structure and Population Genetic Inferences From Nucleotide-Sequence Variation in Human Lipoprotein Lipase. Am J Hum Genet 63: pp 595-612.
Colcher D, Pavlinkova G, Beresford G, Booth B J and Batra S K (1999) Single-Chain Antibodies in Pancreatic Cancer. Ann N Y Acad Sci 880:263-80.: pp 263-280.
Collins FS, Brooks L D and Chakravarti A (1998) A DNA Polymorphism Discovery Resource for Research on Human Genetic Variation. Genome Res 8: pp 1229-1231.
Cotton RG, Rodrigues N R and Campbell R D (1988) Reactivity of Cytosine and Thymine in Single-Base-Pair Mismatches With Hydroxylamine and Osmium Tetroxide and Its Application to the Study of Mutations. Proc Natl Acad Sci U S A 85: pp 4397-4401.
Cronin MT, Fucini R V, Kim S M, Masino R S, Wespi R M and Miyada C G (1996) Cystic Fibrosis Mutation Detection by Hybridization to Light-Generated DNA Probe Arrays. Hum Mutat 7: pp 244-255.
Crow JF (1995) Spontaneous Mutation As a Risk Factor. Exp Clin Immunogenet 12: pp 121-128.
Cruikshank WW, Doctrow S R, Falvo M S, Huffman K, Maciaszek J, Viglianti G, Raina J, Kornfeld H and Malfroy B (1997) A Lipidated Anti-Tat Antibody Enters Living Cells and Blocks HIV-1 Viral Replication. J Acquir Immune Defic Syndr Hum Retrovirol 14: pp 193-203.
Cull MG, Miller J F and Schatz P J (1992) Screening for Receptor Ligands Using Large Libraries of Peptides Linked to the C Terminus of the Lac Repressor. Proc Natl Acad Sci U S A 89: pp 1865-1869.
Cwirla SE, Peters E A, Barrett R W and Dower W J (1990) Peptides on Phage: a Vast Library of Peptides for Identifying Ligands. Proc Natl Acad Sci U S A 87: pp 6378-6382.
Daly MJ, Rioux J D, Schaffner S F, Hudson T J and Lander E S (2001) High-Resolution Haplotype Structure in the Human Genome. Nat Genet 29: pp 229-232.
Dawson E, Abecasis G R, Bumpstead S, Chen Y, Hunt S, Beare D M, Pabial J, Dibling T, Tinsley E, Kirby S, Carter D, Papaspyridonos M, Livingstone S, Ganske R, Lohmussaar E, Zernant J, Tonisson N, Remm M, Magi R, Puurand T, Vilo J, Kurg A, Rice K, Deloukas P, Mott R, Metspalu A, Bentley D R, Cardon L R and Dunham I (2002) A First-Generation Linkage Disequilibrium Map of Human Chromosome 22. Nature 418: pp 544-548.
de Leon MP, Pedroni M, Benatti P, Percesepe A, Di Gregorio C, Foroni M, Rossi G, Genuardi M, Neri G, Leonardi F, Viel A, Capozzi E, Boiocchi M and Roncucci L (1999) Hereditary Colorectal Cancer in the General Population: From Cancer Registration to Molecular Diagnosis. Gut 45: pp 32-38.
Devlin JJ, Panganiban L C and Devlin P E (1990) Random Peptide Libraries: a Source of Specific Protein Binding Molecules. Science 249: pp 404-406.
DeWitt SH, Kiely J S, Stankovic C J, Schroeder M C, Cody D M and Pavia M R (1993) "Diversomers": an Approach to Nonpeptide, Nonoligomeric Chemical Diversity. Proc Natl Acad Sci U S A 90: pp 6909-6913.
Dunning AM, Durocher F, Healey C S, Teare M D, McBride S E, Carlomagno F, Xu C F, Dawson E, Rhodes S, Ueda S, Lai E, Luben R N, Van Rensburg E J, Mannermaa A, Kataja V, Rennart G, Dunham I, Purvis I, Easton D and Ponder B A (2000) The Extent of Linkage Disequilibrium in Four Populations With Distinct Demographic Histories. Am J Hum Genet 67: pp 1544-1554.
Edlund T, Walker M D, Barr P J and Rutter W J (1985) Cell-Specific Expression of the Rat Insulin Gene: Evidence for Role of Two Distinct 5' Flanking Elements. Science 230: pp 912-916.
Elbashir SM, Harborth J, Weber K and Tuschl T (2002) Analysis of Gene Function in Somatic Mammalian Cells Using Small Interfering RNAs. Methods 26: pp 199-213.
Erb E, Janda K D and Brenner S (1994) Recursive Deconvolution of Combinatorial Chemical Libraries. Proc Natl Acad Sci U S A 91: pp 11422-11426.
Felici F, Castagnoli L, Musacchio A, Jappelli R and Cesareni G (1991) Selection of Antibody Ligands From a Large Library of Oligopeptides Expressed on a Multivalent Exposition Vector. J Mol Biol 222: pp 301-310.
Fodor SP, Rava R P, Huang X C, Pease A C, Holmes C P and Adams C L (1993) Multiplexed Biochemical Assays With Biological Chips. Nature 364: pp 555-556.
Gabriel SB, Schaffner S F, Nguyen H, Moore J M, Roy J, Blumenstiel B, Higgins J, DeFelice M, Lochner A, Faggart M, Liu-Cordero S N, Rotimi C, Adeyemo A, Cooper R, Ward R, Lander E S, Daly M J and Altshuler D (2002) The Structure of Haplotype Blocks in the Human Genome. Science 296: pp 2225-2229.
Gallop MA, Barrett R W, Dower W J, Fodor S P and Gordon E M (1994) Applications of Combinatorial Technologies to Drug Discovery. 1. Background and Peptide Combinatorial Libraries. J Med Chem 37: pp 1233-1251.
Gautier C, Morvan F, Rayner B, Huynh-Dinh T, Igolen J, Imbach J L, Paoletti C and Paoletti J (1987) Alpha-DNA. IV: Alpha-Anomeric and Beta-Anomeric Tetrathymidylates Covalently Linked to Intercalating Oxazolopyridocarbazole. Synthesis, Physicochemical Properties and Poly (RA) Binding. Nucleic Acids Res 15: pp 6625-6641.
Goeddel DV (1990) Systems for Heterologous Gene Expression. Methods Enzymol 185:3-7.: pp 3-7.
Gottesman S (1990) Minimizing Proteolysis in Escherichia Coli: Genetic Solutions. Methods Enzymol 185:119-29.: pp 119-129.
Hage DS and Tweed S A (1997) Recent Advances in Chromatographic and Electrophoretic Methods for the Study of Drug-Protein Interactions. J Chromatogr B Biomed Sci Appl 699: pp 499-525.
Haseloff J and Gerlach W L (1988) Simple RNA Enzymes With New and Highly Specific Endoribonuclease Activities. Nature 334: pp 585-591.
Heegaard NH (1998) Capillary Electrophoresis for the Study of Affinity Interactions. J Mol Recognit 11: pp 141-148.
Helene C (1991) The Anti-Gene Strategy: Control of Gene Expression by Triplex-Forming-Oligonucleotides. Anticancer Drug Des 6: pp 569-584.
Helene C, Thuong N T and Harel-Bellan A (1992) Control of Gene Expression by Triple Helix-Forming Oligonucleotides. The Antigene Strategy. Ann N YAcad Sci 660:27-36.: pp 27-36.
Herlyn D and Birebent B (1999) Advances in Cancer Vaccine Development. Ann Med 31: pp 66-78.
Herzog TJ (2003) New approaches for the management of cervical cancer. Gynecol Oncol. (3 Pt 2):S22-7.
Houghten RA, Appel J R, Blondelle S E, Cuervo J H, Dooley C T and Pinilla C (1992) The Use of Synthetic Peptide Combinatorial Libraries for the Identification of Bioactive Peptides. Biotechniques 13: pp 412-421.
Hyrup B and Nielsen P E (1996) Peptide Nucleic Acids (PNA): Synthesis, Properties and Potential Applications. Bioorg Med Chem 4: pp 5-23.
Inoue H, Hayase Y, Imura A, Iwai S, Miura K and Ohtsuka E (1987a) Synthesis and Hybridization Studies on Two Complementary Nona (2'-O-Methyl)Ribonucleotides. Nucleic Acids Res 15: pp 6131-6148.
Inoue H, Hayase Y, Iwai S and Ohtsuka E (1987b) Sequence-Dependent Hydrolysis of RNA Using Modified Oligonucleotide Splints and RNase H. FEBS Lett 215: pp 327-330.
Iwabuchi K, Li B, Bartel P and Fields S (1993) Use of the Two-Hybrid System to Identify the Domain of P53 Involved in Oligomerization. Oncogene 8: pp 1693-1696.
Jespers LS, Roberts A, Mahler S M, Winter G and Hoogenboom H R (1994) Guiding the Selection of Human Antibodies From Phage Display Repertoires to a Single Epitope of an Antigen. Biotechnology (N Y) 12: pp 899-903.
Kessel M and Gruss P (1990) Murine Developmental Control Genes. Science 249: pp 374-379.
Kikuchi T, Daigo Y, Katagiri T, Tsunoda T, Okada K, Kakiuchi S, Zembutsu H, Furukawa Y, Kawamura M, Kobayashi K, Imai K and Nakamura Y (2003) Expression Profiles of Non-Small Cell Lung Cancers on CDNA Microarrays: Identification of Genes for Prediction of Lymph-Node Metastasis and Sensitivity to Anti-Cancer Drugs. Oncogene 22: pp 2192-2205.
Kondrashov AS (1995) Contamination of the Genome by Very Slightly Deleterious Mutations: Why Have We Not Died 100 Times Over? J Theor Biol 175: pp 583-594.
Kozal MJ, Shah N, Shen N, Yang R, Fucini R, Merigan T C, Richman D D, Morris D, Hubbell E, Chee M and Gingeras T R (1996) Extensive Polymorphisms Observed in HIV-1 Clade B Protease Gene Using High-Density Oligonucleotide Arrays. Nat Med 2: pp 753-759.
Lam KS (1997) Application of Combinatorial Library Methods in Cancer Research and Drug Discovery. Anticancer Drug Des 12: pp 145-167.
Lam KS, Salmon S E, Hersh E M, Hruby V J, Kazmierski W M and Knapp R J (1991) A New Type of Synthetic Peptide Library for Identifying Ligand-Binding Activity. Nature 354: pp 82-84.
Lemaitre M, Bayard B and Lebleu B (1987) Specific Antiviral Activity of a Poly (L-Lysine)-Conjugated Oligodeoxyribonucleotide Sequence Complementary to Vesicular Stomatitis Virus N Protein MRNA Initiation Site. Proc Natl Acad Sci U S A 84: pp 648-652.
Letsinger RL, Zhang G R, Sun D K, Ikeuchi T and Sarin P S (1989) Cholesteryl-Conjugated Oligonucleotides: Synthesis, Properties, and Activity As Inhibitors of Replication of Human Immunodeficiency Virus in Cell Culture. Proc Natl Acad Sci U S A 86: pp 6553-6556.
Liu AY, Robinson R R, Hellstrom K E, Murray E D, Jr., Chang C P and Hellstrom I (1987a) Chimeric Mouse-Human IgG1 Antibody That Can Mediate Lysis of Cancer Cells. Proc Natl Acad Sci U S A 84: pp 3439-3443.
Liu AY, Robinson R R, Murray E D, Jr., Ledbetter J A, Hellstrom I and Hellstrom K E (1987b) Production of a Mouse-Human Chimeric Monoclonal Antibody to CD20 With Potent Fc-Dependent Biologic Activity. J Immunol 139: pp 3521-3526.
Liu N, Sawyer S L, Mukherjee N, Pakstis A J, Kidd J R, Kidd K K, Brookes A J and Zhao H (2004) Haplotype Block Structures Show Significant Variation Among Populations. Genet Epidemiol 27: pp 385-400.
Lonberg N and Huszar D (1995) Human Antibodies From Transgenic Mice. Int Rev Immunol 13: pp 65-93.
Lynch HT and de La C A (2003) Hereditary Colorectal Cancer. N Engl J Med 348: pp 919-932.
Madura K, Dohmen R J and Varshavsky A (1993) N-Recognin/Ubc2 Interactions in the N-End Rule Pathway. J Biol Chem 268: pp 12046-12054.
Maher LJ, III (1992) DNA Triple-Helix Formation: an Approach to Artificial Gene Repressors? Bioessays 14: pp 807-815.
Malik F, Delgado C, Knusli C, Irvine A E, Fisher D and Francis G E (1992) Polyethylene Glycol (PEG)-Modified Granulocyte-Macrophage Colony-Stimulating Factor (GM-CSF) With Conserved Biological Activity. Exp Hematol 20: pp 1028-1035.
Marasco WA, Haseltine W A and Chen S Y (1993) Design, Intracellular Expression, and Activity of a Human Anti-Human Immunodeficiency Virus Type 1 Gp120 Single-Chain Antibody. Proc Natl Acad Sci U S A 90: pp 7889-7893.
McConnell HM, Owicki J C, Parce J W, Miller D L, Baxter G T, Wada H G and Pitchford S (1992) The Cytosensor Microphysiometer: Biological Applications of Silicon Technology. Science 257: pp 1906-1912.
Morrison SL (1985) Transfectomas Provide Novel Chimeric Antibodies. Science 229: pp 1202-1207.
Myers RM, Larin Z and Maniatis T (1985) Detection of Single Base Substitutions by Ribonuclease Cleavage at Mismatches in RNA:DNA Duplexes. Science 230: pp 1242-1246.
Nishimura Y, Yokoyama M, Araki K, Ueda R, Kudo A and Watanabe T (1987) Recombinant Human-Mouse Chimeric Monoclonal Antibody Specific for Common Acute Lymphocytic Leukemia Antigen. Cancer Res 47: pp 999-1005.
Osborne SE, Matsumura I and Ellington A D (1997) Aptamers As Therapeutic and Diagnostic Reagents: Problems and Prospects. Curr Opin Chem Biol 1: pp 5-9.
Patel DJ (1997) Structural Analysis of Nucleic Acid Aptamers. Curr Opin Chem Biol 1: pp 32-46.
Perry-O'Keefe H, Yao X W, Coull J M, Fuchs M and Egholm M (1996) Peptide Nucleic Acid Pre-Gel Hybridization: an Alternative to Southern Hybridization. Proc Natl Acad Sci U S A 93: pp 14670-14675.
Pinkert CA, Ornitz D M, Brinster R L and Palmiter R D (1987) An Albumin Enhancer Located 10 Kb Upstream Functions Along With Its Promoter to Direct Efficient, Liver-Specific Expression in Transgenic Mice. Genes Dev 1: pp 268-276.
Queen C and Baltimore D (1983) Immunoglobulin Gene Transcription Is Activated by Downstream Sequence Elements. Cell 33: pp 741-748.
Reich DE, Cargill M, Bolk S, Ireland J, Sabeti P C, Richter D J, Lavery T, Kouyoumjian R, Farhadian S F, Ward R and Lander E S (2001) Linkage Disequilibrium in the Human Genome. Nature 411: pp 199-204.
Reiter Y and Pastan I (1996) Antibody Engineering of Recombinant Fv Immunotoxins for Improved Targeting of Cancer: Disulfide-Stabilized Fv Immunotoxins. Clin Cancer Res 2: pp 245-252.
Rioux JD, Daly M J, Silverberg M S, Lindblad K, Steinhart H, Cohen Z, Delmonte T, Kocher K, Miller K, Guschwan S, Kulbokas E J, O'Leary S, Winchester E, Dewar K, Green T, Stone V, Chow C, Cohen A, Langelier D, Lapointe G, Gaudet D, Faith J, Branco N, Bull S B, McLeod R S, Griffiths A M, Bitton A, Greenberg G R, Lander E S, Siminovitch K A and Hudson T J (2001) Genetic Variation in the 5q31 Cytokine Gene Cluster Confers Susceptibility to Crohn Disease. Nat Genet 29: pp 223-228.
Rivas G and Minton A P (1993) New Developments in the Study of Biomolecular Associations Via Sedimentation Equilibrium. Trends Biochem Sci 18: pp 284-287.
Sachidanandam R, Weissman D, Schmidt S C, Kakol J M, Stein L D, Marth G, Sherry S, Mullikin J C, Mortimore B J, Willey D L, Hunt S E, Cole C G, Coggill P C, Rice C M, Ning Z, Rogers J, Bentley D R, Kwok P Y, Mardis E R, Yeh R T, Schultz B, Cook L, Davenport R, Dante M, Fulton L, Hillier L, Waterston R H, McPherson J D, Gilman B, Schaffner S, Van Etten W J, Reich D, Higgins J, Daly M J, Blumenstiel B, Baldwin J, Stange-Thomann N, Zody M C, Linton L, Lander E S and Altshuler D (2001) A Map of Human Genome Sequence Variation Containing 1.42 Million Single Nucleotide Polymorphisms. Nature 409: pp 928-933.
Saiki RK, Bugawan T L, Horn G T, Mullis K B and Erlich H A (1986) Analysis of Enzymatically Amplified Beta-Globin and HLA-DQ Alpha DNA With Allele-Specific Oligonucleotide Probes. Nature 324: pp 163-166.
Sasieni D P (1997) From Genotypes to Genes: Doubling the Sample Size. Biometrics V.53,(4) 1253-1261.
Scott JK and Smith G P (1990) Searching for Peptide Ligands With an Epitope Library. Science 249: pp 386-390.
Shaw DR, Khazaeli M B and LoBuglio A F (1988) Mouse/Human Chimeric Antibodies to a Tumor-Associated Antigen: Biologic Activity of the Four Human IgG Subclasses. J Natl Cancer Inst 80: pp 1553-1559.
Sjolander S and Urbaniczky C (1991) Integrated Fluid Handling System for Biomolecular Interaction Analysis. Anal Chem 63: pp 2338-2345.
Smith & Johnson (1988) Single-step purification of polypeptides expressed in Escherichia coli as fusions with glutathione S-transferase. Gene 67: 31-40.
Sun LK, Curtis P, Rakowicz-Szulczynska E, Ghrayeb J, Chang N, Morrison S L and Koprowski H (1987) Chimeric Antibody With Human Constant Regions and Mouse Variable Regions Directed Against Carcinoma-Associated Antigen 17-1A. Proc Natl Acad Sci USA 84: pp 214-218.
Szabo A, Stolz L and Granzow R (1995) Surface Plasmon Resonance and Its Use in Biomolecular Interaction Analysis (BIA). Curr Opin Struct Biol 5: pp 699-705.
Taillon-Miller P, Piernot E E and Kwok P Y (1999) Efficient Approach to Unique Single-Nucleotide Polymorphism Discovery. Genome Res 9: pp 499-505.
van der Krol AR, Mol J N and Stuitje A R (1988) Modulation of Eukaryotic Gene Expression by Complementary RNA or DNA Sequences. Biotechniques 6: pp 958-976.
Verhoeyen M, Milstein C and Winter G (1988) Reshaping Human Antibodies: Grafting an Antilysozyme Activity. Science 239: pp 1534-1536.
Vlatakis G, Andersson L I, Muller R and Mosbach K (1993) Drug Assay Using Antibody Mimics Made by Molecular Imprinting. Nature 361: pp 645-647.
Wada K, Wada Y, Ishibashi F, Gojobori T and Ikemura T (1992) Codon Usage Tabulated From the GenBank Genetic Sequence Data. Nucleic Acids Res 20 Suppl:2111-8.: pp 2111-2118.
Wang DG, Fan J B, Siao C J, Berno A, Young P, Sapolsky R, Ghandour G, Perkins N, Winchester E, Spencer J, Kruglyak L, Stein L, Hsie L, Topaloglou T, Hubbell E, Robinson E, Mittmann M, Morris M S, Shen N, Kilburn D, Rioux J, Nusbaum C, Rozen S, Hudson T J, Lipshutz R, Chee M and Lander E S (1998) Large-Scale Identification, Mapping, and Genotyping of Single-Nucleotide Polymorphisms in the Human Genome. Science 280: pp 1077-1082.
Winoto A and Baltimore D (1989) A Novel, Inducible and T Cell-Specific Enhancer Located at the 3' End of the T Cell Receptor Alpha Locus. EMBO J 8: pp 729-733.
Wood CR, Boss M A, Kenten J H, Calvert J E, Roberts N A and Emtage J S (1985) The Synthesis and in Vivo Assembly of Functional Antibodies in Yeast. Nature 314: pp 446-449.
Zervos AS, Gyuris J and Brent R (1993) Mxi1, a Protein That Specifically Interacts With Max to Bind Myc-Max Recognition Sites. Cell 72: pp 223-232.
Zon G (1988) Oligonucleotide Analogues As Potential Chemotherapeutic Agents. Pharm Res 5: pp 539-549.
Zuckermann RN, Martin E J, Spellmeyer D C, Stauber G B, Shoemaker K R, Kerr J M, Figliozzi G M, Goff D A, Siani M A, Simon R J and. (1994) Discovery of Nanomolar Ligands for 7-Transmembrane G-Protein-Coupled Receptors From a Diverse N-(Substituted)Glycine Peptoid Library. J Med Chem 37: pp 2678-2685.

## Claims

1. A method for identifying an individual who has an altered risk for developing colorectal cancer, comprising detecting a single nucleotide polymorphism (SNP) at a base located at position 110676919 on human chromosome 11;
or one or more bases in linkage disequilibrium with a r² value greater than 0.5 with said base located at position 110676919 on human chromosome 11.

2. The method of claim 1, wherein said one or more bases in linkage disequilibrium with a r² value greater than 0.5 with said base located at position 110676919 on human chromosome 11 are located at positions 110624904, 110625908, 110630722, 110638215, 110640955, 110645253, 110657706, 110659282, 110662046, 110665963, 110667901, 110672767, 110676856, 110677389, 110678500, 110685600 and 110685841 on human chromosome 11.

3. The method of claim 1 or 2, wherein:
when the base at position 110676919 on human chromosome 11 is C or is in disequilibrium linkage therewith, then said base exerts a increased risk..

4. The method of claim 3, wherein the allelic effect is as specified in Table 1.

5. The method of claim 3, wherein the SNP at position 110676919 on chromosome 11 is rs3802842.

6. The method of claim 2, wherein the SNP at position 110624904 on human chromosome 11 is ras11213801, the SNP at position 110625908 on human chromosome 11 is rs10891239, the SNP at position 110630722 on human chromosome 11 is rs2001754, the SNP at position 110638215 on human chromosome 11 is rs7116087, the SNP at position 110640955 on human chromosome 11 is rs11213809, the SNP at position 110645253 on human chromosome 11 is rs4548657, the SNP at position 110657706 on human chromosome 11 is rs1987128, the SNP at position 110659282 on human chromosome 11 is rs7130173, the SNP at position 110662046 on human chromosome 11 is rs3087967, the SNP at position 110665963 on human chromosome 11 is rs4477469, the SNP at position 110667901 on human chromosome 11 is rs10789822, the SNP at position 110672767 on human chromosome 11 is rs6589218, the SNP at position 110676856 on human chromosome 11 is rs3802840, the SNP at position 110677389 on human chromosome 11 is rs4608113, the SNP at position 110678500 on human chromosome 11 is rs6589220, the SNP at position 110685600 on human chromosome 11 is rs11213825 and the SNP at position 110685841 on human chromosome 11 is rs7933961.

7. A method for identifying an individual who has an altered risk for developing colorectal cancer, comprising:
(a) providing a sample containing genetic material of the individual;
(b) amplifying the genetic material in the presence of a pair of primers wherein a first of the primers comprises at least 10 consecutive nucleotides selected from one of sequences of SEQ ID NOs: 1-18, each located upstream of the base located at position 331 of each sequence and a second primer comprising at least 10 consecutive nucleotides selected from within the same sequence and located downstream of the base located at position 331; and
(c) determining the identity of the base in the genetic material that corresponds to position 331.

8. A method for identifying a correlation between a single nucleotide polymorphism and a susceptibility of an individual to colorectal cancer, comprising:
(a) accessing a database containing nucleotide sequence data on single nucleotide polymorphisms located between position 110624904 and position 110685841 on human chromosome 11;
(b) determining the position of a said single nucleotide polymorphism on the human chromosome;
(c) storing the position determined in step (b);
(d) providing the identity of a nucleotide base at the position of the single nucleotide polymorphism stored in step (c) for each member of a clinical population that has been diagnosed as having colorectal cancer;
(e) providing the identity of a nucleotide base at the position of the single nucleotide polymorphism stored in step (c) for each member of a control population; and
(f) calculating the degree of correlation between the identities of the nucleotide bases provided in steps (d) and (e) and a diagnosis of colorectal cancer in the clinical population.

9. A method for identifying a correlation between a single nucleotide polymorphism and a susceptibility of an individual to colorectal cancer, comprising:
(a) determining the nucleotide sequence for each member of a clinical population that has been diagnosed as having colorectal cancer wherein the sequence is located between position 110624904 and position 110685841 on human chromosome 11;
(b) storing the sequence;
(c) accessing a database containing nucleotide sequence data for said sequence;
(d) determining the position of a single nucleotide polymorphism within the sequence;
(e) providing the identity of a nucleotide base at the position of the single nucleotide polymorphism determined in step (d) for each member of a control population; and
(f) calculating the degree of correlation between the identities of the nucleotide bases provided in steps (c) and (d) and a diagnosis of colorectal cancer in the clinical population.

10. A method for identifying a correlation between a single nucleotide polymorphism and a susceptibility of an individual to colorectal cancer, comprising:
(a) determining the nucleotide sequence for each member of a clinical population that has been diagnosed as having colorectal cancer wherein the sequence is located between position 110624904 and position 110685841 on human chromosome 11;
(b) storing the sequence;
(c) determining the nucleotide sequence, for each member of a control population, corresponding to the sequence stored in step (b);
(d) storing sequence determined in step (c);
(e) determining the position of a single nucleotide polymorphism within the sequence stored in step (b); and
(f) calculating the degree of correlation between the identities of the nucleotide bases at the position determined in step (e) and a diagnosis of colorectal cancer in the clinical population.

11. A method for identifying a correlation between a single nucleotide polymorphism and a susceptibility of an individual to colorectal cancer, comprising:
(a) accessing a database that includes nucleotide sequence data on single nucleotide polymorphisms located between position 110624904 and position 110685841 on human chromosome 11 different from those listed in Table 1A;
(b) determining the position of a said single nucleotide polymorphism on the human chromosome;
(c) storing the position determined in step (b);
(d) providing the identity of a nucleotide base at the position of the single nucleotide polymorphism stored in step (c) for each member of a population;
(e) determining the identity of a nucleotide base at the position of a single nucleotide polymorphism listed in Table 1A for each member of the population; and
(f) determining whether the nucleotide bases determined in steps (b) and (e), respectively, are in strong linkage disequilibrium with each other, wherein, if the nucleotide bases are in strong disequilibrium with each other, then the single nucleotide polymorphism determined in step (b) can be used for predicting the susceptibility of an individual to colorectal cancer in the same way as the single nucleotide polymorphism of step (e).

12. A method for identifying increased susceptibility to colorectal cancer in a subject, comprising: obtaining a sample from a human subject; determining if the sample contains an C allele at position 110676919 of chromosome 11 or a single nucleotide polymorphism in linkage disequilibrium with a r² value greater than 0.5 with said base located at position 110676919 on human chromosome 11.

13. A method for identifying a human subject as having an increased susceptibility for developing colorectal cancer, said method comprising detecting a C nucleotide at a position corresponding to position 331 of SEQ ID. NO: 14.

14. The method according claim 13, wherein a blood sample from the subject is used to detect the presence of the polymorphism.

15. The method according to claim 13 or 14, wherein the step of detecting comprises DNA extraction and a process selected from the group consisting of: allele-specific probe hybridization, allele-specific primer extension, allele-specific amplification, sequencing, 5' nuclease digestion, molecular beacon assay, oligonucleotide ligation assay, size analysis, and single-stranded conformation polymorphism.
